# EUROPEAN PATENT APPLICATION

(11) **EP 3 505 527 A1**
(43) Date of publication of application: **03.07.2019**
(21) Application number: 17306979.0
(22) Date of filing: 29.12.2017
(51) Int. Cl.: C07H 21/00, A61K 31/7084, A61P 31/00, A61P 31/12, A61P 31/18

(54) **CYCLIC DINUCLEOTIDES FOR CYTOKINE INDUCTION**

(71) Applicant: Invivogen, 31400 Toulouse (FR)
(72) Inventor: LIOUX, Thierry, 31130 BALMA (FR); ANDREINI, Manuel, 31650 SAINT-ORENS DE GAMEVILLE (FR); VERNEJOUL, Fabienne, 31100 TOULOUSE (FR); BOULARAN, Cédric, 31500 TOULOUSE (FR); TIRABY, Michèle, 31000 TOULOUSE (FR)
(74) Representative: Cabinet Plasseraud

(57) **Abstract**

The present invention concerns compounds of Formula (I) or Formula (II). 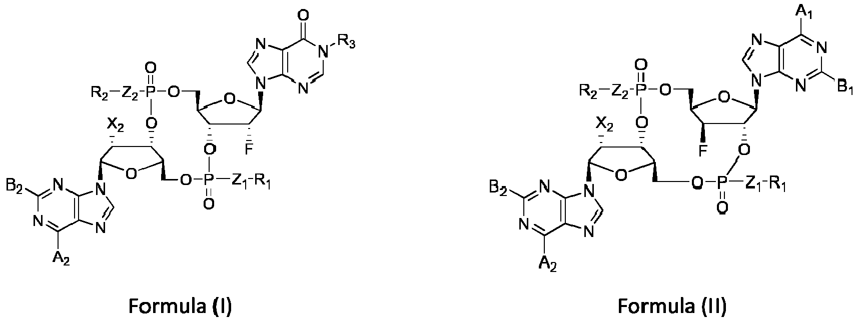 wherein X₂ is H or F; Z₁ and Z₂ are independently O or S; R₁ and R₂ are independently H or an enzyme-labile group which, together with the oxygen atom or sulfur atom to which it is attached, provides OH or SH *in vivo* such as pivaloyloxymethyl or acyloxybenzyl group; A₁ is O-R₃ or NH-R₃; A₂ is O-R_{3'} or NH-R_{3'}; B₁ is H, NH-R₄ or an halogen such as F, Cl, Br, I; B₂ is H, NH-R_{4'} or an halogen such as F, Cl, Br, I; R₃, R_{3'}, R₄ and R_{4'} are independently H or an enzyme-labile group which, together with the oxygen atom or sulfur atom to which it is attached, provides OH or NH₂ *in vivo* such as pivaloyloxymethyl,
or a pharmaceutically acceptable salt, stereoisomer, tautomer or solvate thereof,
except a compound of Formula (I) wherein X₂ = F, Z₁ = Z₂= O, R₁ = R₂= R₃= H, A₂ = OH and B₂ = H.

The invention also concerns pharmaceutical compositions comprising said cyclic dinucleotide, as well as their use in the treatment of a bacterial infection, a viral infection or a cancer.

## Description

### FIELD OF THE INVENTION

The present invention is in the field of immunotherapy. It concerns cyclic dinucleotides (CDNs) of Formulae (I) or (II). In particular, it concerns fluorinated CDNs which are able to induce production of Type I interferons in human and animal cells. The cytokine-induction activity of these cyclic dinucleotides requires the presence of the eukaryotic cellular receptor *stimulator of interferon genes* (STING) as demonstrated *in vitro.*

### BACKGROUND OF THE INVENTION

### Cytokine induction immunotherapy

Immunotherapy is a rapidly expanding area of medical treatment in which a patient's immune system is deliberately activated, suppressed or otherwise modulated for therapeutic benefit. Immunotherapy agents include cells, antigens (e.g. fragments of bacteria or viruses), antibodies, nucleic acids, peptides, proteins, naturally occurring ligands and synthetic molecules. Cytokines are small glycoprotein messengers known chiefly for their role in orchestrating immune response through complex signaling networks, although they also perform non-immune functions. They have been extensively explored as immunotherapy agents. However, direct administration of cytokines as immunotherapy is limited by numerous factors, including the very short half-life of cytokines in blood, which must be compensated for with frequent dosing and high doses. One highly promising immunotherapy approach is cytokine induction, whereby the patient is treated with an immunomodulatory agent that triggers the production of one or more therapeutically beneficial cytokines in their body as needed.

### STING, cytokines and immune response

A major player in physiological production of cytokines is stimulator of interferon genes (STING; also known as ERIS, MITA, MPYS, or TM173), a transmembrane receptor protein that is paramount in innate immunity. Human STING is encoded by the gene TMEM173. Activation of STING leads to production of Type I interferons (e.g. IFN-α and IFN-β), via the IRF3 (interferon regulatory factor 3) pathway; and to production of pro-inflammatory cytokines (IL-1α, IL-1β, IL-2, IL-6, TNF-α, etc.), via the oncogenic transcription factor NF-κB (nuclear factor kappa-light-chain-enhancer of activated B cells) pathway. Moreover, researchers recently reported that in response to viral infection, STING activates STAT6 (signal transducer and activator of transcription 6) to induce (Th2-type), increase (IL-12) or decrease (IL-10) production of various cytokines, including the chemokines CCL2, CCL20, and CCL26 (Chen et al., 2011).

### STING agonists

Human STING is currently known to be activated three ways: via binding of exogenous (3',3) cyclic dinucleotides (c-diGMP, c-diAMP and c-GAMP) that are released by invading bacteria or archaea (see (Gomelsky, 2011) and references therein); via binding of (2',3')cyclic guanosine monophosphate-adenosine monophosphate ((2',3')c-GAMP), a recently discovered endogenous cyclic dinucleotide that is produced by the enzyme cyclic GMP-AMP synthase (cGAS; also known as C6orf150 or MB21D1) in the presence of exogenous double-stranded DNA (e.g. that released by invading bacteria, viruses or protozoa) or of self-DNA in mammals (see, for example: (Ablasser et al., 2013) and (Zhang et al., 2013)); or via binding of synthetic ligands, such as analogs of the aforementioned naturally-occurring cyclic dinucleotides (see, for example: (Dubensky, Kanne, & Leong, 2013) and (Li et al., 2014)).

### Modulation of STING in immunotherapy

Inspired by the interplay among STING, cytokines and immune response, as well as by the ever-growing body of knowledge on the clinical implications of STING and its mutations, researchers have very recently begun to explore STING as a therapeutic target for myriad indications. New STING agonists are being pursued as therapeutic agents for human and animal health in areas such as cancer or infectious diseases. The known cyclic dinucleotide STING agonists are an excellent class of compounds on which to base analogs that might exhibit interesting biological activities or desirable drug-like properties. The present invention comprises novel cyclic dinucleotides for therapeutic use in human and animal health.

We previously reported that CDNs containing one inosine, such as cAIMP and analogs thereof, activate STING signaling in cell lines and in blood samples (Ref. Lioux, 2016, JMC; Ref. WO 2016/096174; and Ref. WO 2016/096577). We found that some of these CDNs induce distinct STING-dependent cytokine profiles to that obtained with reference STING ligands such as 2',3'-cGAMP or DMXAA. We have also shown that, *in vivo,* such compounds can provide immunostimulatory effects in healthy mice (Ref. WO 2016/096174 and Ref. WO 2016/096577) and therapeutic effects in animal models of cancer (Ref. WO 2016/096577). We and other groups have also evaluated the enzymatic degradation of inosine-containing CDNs *in vitro* (Ref. Lioux, 2016, JMC; Ref. WO 2016/096174; and Ref. Lee, 2017, MedChemComm).

### SUMMARY OF THE INVENTION

The present invention concerns a structurally unprecedented subset of CDNs that show biological activity and have never previously been reported as STING agonists. These CDNs form the basis for the present invention and are distinct from previously reported CDN STING agonists.

In one aspect, the present invention provides CDNs that are defined by the following structural criteria:
- First, unlike in naturally occurring CDNs, the bases in each nucleotide of CDNs of the present invention are preferably an hypoxanthine and/or an adenine and/or a guanine;
- Second, the bases can be *N¹*- or *O*-substituted for hypoxanthine, *N¹*- or *O*- and/or *N²*-substituted guanine and *N⁶*-substituted adenine by a carboxyesterase biodegradable group, such as POM.
- Third, the sugar moiety of each nucleoside is a non-natural sugar, indeed the sugar is preferably a 2'-fluoro-2'-deoxy-ribose or a 3'-fluoro-3'-deoxy-xylose;
- Fourth, in the CDNs of the present invention, the internucleosidic link is preferably a phosphodiester, a phosphorothioate diester or a phosphorothioate triester.

The invention is directed to compounds according to Formula (I) or (II): 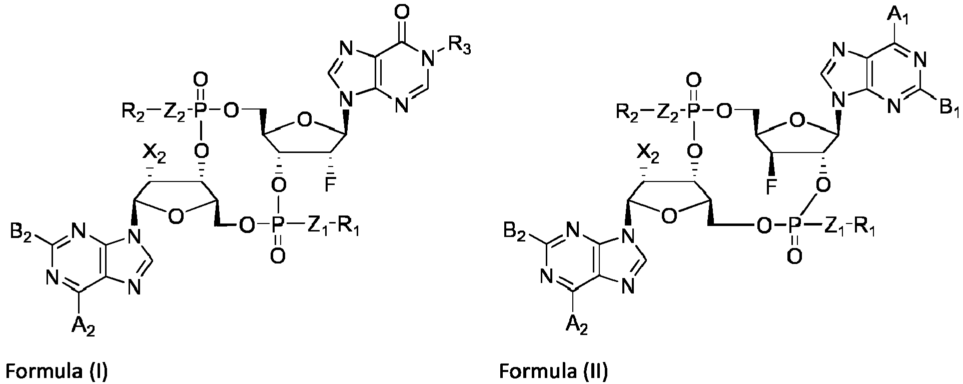 wherein:
- X₂ is H or F;
- Z₁ and Z₂ are independently O or S;
- R₁ and R₂ are independently H or an enzyme-labile group which, together with the oxygen atom or sulfur atom to which it is attached, provides OH or SH *in vivo* such as pivaloyloxymethyl or acyloxybenzyl group;
- A₁ is O-R₃ or NH-R₃;
- A₂ is O-R_{3'} or NH-R_{3'};
- B₁ is H, NH-R₄ or a halogen such as F, Cl, Br, I;
- B₂ is H, NH-R_{4'} or a halogen such as F, Cl, Br, I;
- R₃, R_{3'}, R₄ and R_{4'} are independently H or an enzyme-labile group which, together with the oxygen atom or sulfur atom to which it is attached, provides OH or NH₂ *in vivo* such as pivaloyloxymethyl,
or pharmaceutically acceptable salts, stereoisomers, tautomers or solvates thereof, except a compound of Formula (I) wherein X₂ = F, Z₁ = Z₂ = O, R₁ = R₂ = R₃ = H, A₂ = OH and B₂ = H.

The invention also concerns subclasses of cyclic dinucleotide compounds of formulas (III), (IV), (V), (VI), (VII), (VIII) (IX) and (X) as defined below in the detailed description.

Another object of the invention is a pharmaceutical composition comprising a cyclic dinucleotide compound of the invention and a pharmaceutically acceptable excipient.

Another object of the invention is a cyclic dinucleotide compound of the invention for use in a therapeutic treatment in humans or animals.

Another object of the invention is a cyclic dinucleotide compound of the invention for use in the treatment of a disease that may be alleviated by the induction of an immune response via the STING pathway.

Another object of the invention is a cyclic dinucleotide compound of the invention for use in an immunomodulatory agent.

Another object of the invention is a cyclic dinucleotide compound of the invention for use in the treatment of an inflammation, allergic diseases or autoimmune diseases.

Another object of the invention is a cyclic dinucleotide compound of the invention for use in the treatment of cancer or pre-cancerous syndromes or infectious diseases, such as viral infection, in particular an AIDS infection or an HIV infection.

Another object of the invention is a cyclic dinucleotide compound of the invention for use as immunoadjuvant.

Another object of the invention is a therapeutic combination comprising a cyclic dinucleotide compound of the invention and a therapeutic agent.

Another object of the invention is a kit-of-parts comprising a cyclic dinucleotide compound of the invention and a chemotherapeutic agent for use in the treatment of cancer.

### DETAILED DESCRIPTION

The compounds of the invention are cyclic dinucleotides (CDNs) of Formulae (I) and (II): 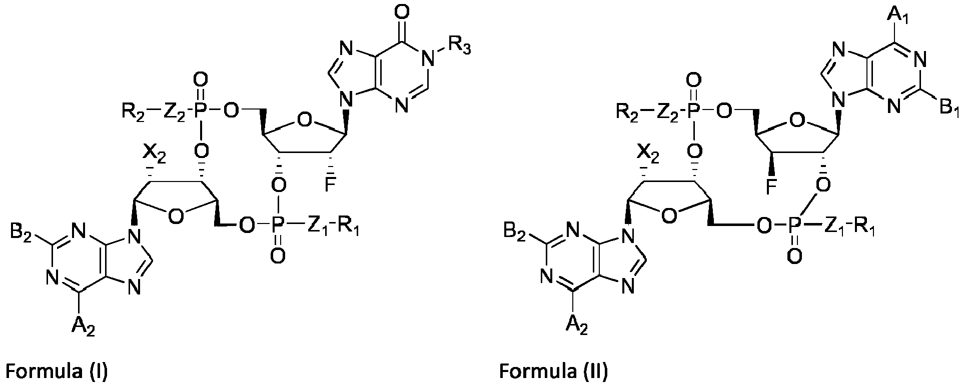 wherein:
- X₂ is H or F;
- Z₁ and Z₂ are independently O or S;
- R₁ and R₂ are independently H or an enzyme-labile group which, together with the oxygen atom or sulfur atom to which it is attached, provides OH or SH *in vivo* such as pivaloyloxymethyl or acyloxybenzyl group;
- A₁ is O-R₃ or NH-R₃;
- A₂ is O-R_{3'} or NH-R_{3'};
- B₁ is H, NH-R₄ or an halogen such as F, Cl, Br, I;
- B₂ is H, NH-R_{4'} or an halogen such as F, Cl, Br, I;
- R₃, R_{3'}, R₄ and R_{4'} are independently H or an enzyme-labile group which, together with the oxygen atom or sulfur atom to which it is attached, provides OH or NH₂ *in vivo* such as pivaloyloxymethyl,
or a pharmaceutically acceptable salt, stereoisomer, tautomer or solvate thereof,

except a compound of Formula (I) wherein X₂ = F, Z₁ = Z₂ = O, R₁ = R₂ = R₃ = H, A₂ = OH and B₂ = H as represented below:

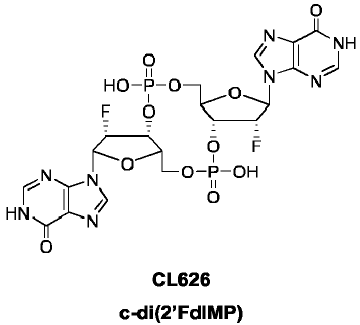

The compounds of the invention are useful as immunomodulatory compounds and as agonists of STING.

In the present invention, the term "cyclic dinucleotide" (abbreviated as "CDN") represents a class of cyclic molecules with:
- two phosphodiester linkages, or
- two phosphorothioate diester linkages, or
- one phosphodiester linkage and one phosphorothioate diester linkage, or
- two phosphorothioate triester linkages, or
- one phosphodiester linkage and one phosphorothioate triester linkage, or
- one phosphorothioate diester linkage and one phosphorothioate triester linkage between two nucleosides.

This includes (3',5')-(3',5') nucleotide linkages (abbreviated as (3',3')); (3',5')-(2',5') nucleotide linkages (abbreviated as (3',2')); (2',5')-(3',5') nucleotide linkages (abbreviated as (2',3')).

Compounds of Formula (I) include (3',3') linkage.

Compounds of Formula (II) include (2',3') linkage.

The term "nucleoside" refers to a glycosylamine comprising a nitrogenous base and a five-carbon sugar, wherein the nitrogenous base is bound to the five-carbon sugar via a beta-glycosidic linkage. Preferred five-carbon sugars are pentofuranosyl sugars selected from the group consisting of D-enantiomers of ribose or xylose and their modified derivatives on position 2' or 3'.

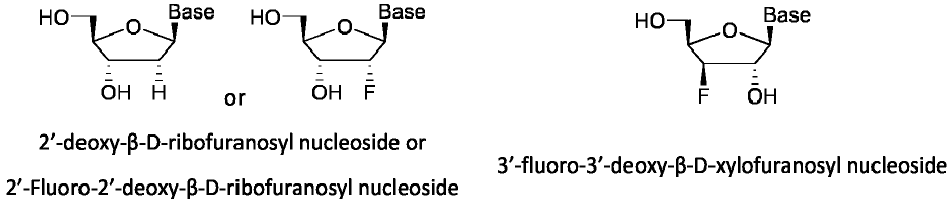

The Base can be:

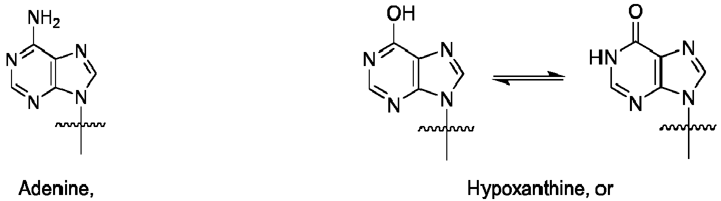

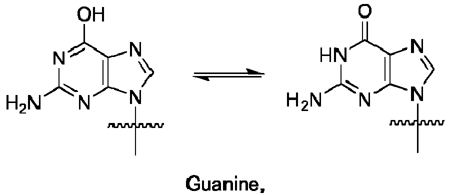

The term "nucleotide" refers to any nucleoside linked to a phosphate group at position 5', 3' or 2' of the sugar moiety.

In the present description, it is considered that the expression "cyclic dinucleotide compound" also includes the pharmaceutically acceptable salts, stereoisomers, tautomers or solvates thereof.

"Pharmaceutically acceptable salts" include those salts derived from pharmaceutically acceptable inorganic or organic bases. Suitable salts include for instance those derived from alkali metals such as potassium and sodium or from alkaline earth metals such as calcium and magnesium or from organic bases such as triethylamine. Due to the negatively charged ionic nature of the phospho or phosphorothioate diester linkage, the counter-ion forming the salt is positively charged.

The expression "enzyme-labile protecting group" denotes a group enabling passive diffusion of a compound across cellular or parasitic membranes via charge-masking, such that, once inside the cell or parasite interior, the compound undergoes an enzymatic transformation (or deprotection) that affords an OH, NH₂ or SH group. Examples of enzyme-labile protecting groups include for instance acyloxyalkyl groups such as pivaloyloxymethyl (POM) or (S)-acyl-2-thioethyl (SATE), or para substituted benzyl groups such as Acetyl-Oxy-Benzyl (AOB) or Para-Amino-Benzyl (PAB).

The compounds of Formula (I) or (II) wherein R₁ and/or R₂ is an enzyme labile group which provides OH or SH *in vivo* are not STING agonists *per se* but are prodrugs of STING agonists, i.e. *in vivo* they release a compound which is a STING agonist.

Indeed, the compounds of the invention bearing a phosphodiester or phosphorothioate diester group (i.e. wherein R₁ and/or R₂ is H), have a negatively charged ionic nature at physiological pH. The therapeutic activity of such compounds is consequently limited, on account of the low diffusion of negatively charged compounds across biological lipid membranes. In particular, negative charged compounds do not diffuse efficiently across cell membranes, or across the cerebral barrier, which are lipidic in nature. One solution to drug delivery and/or bioavailability issues in pharmaceutical development is to convert a diester drug to a triester prodrug. Typically, in a phosphorothioate prodrug, the polar functional group is masked by a pro-moiety, which is labile *in vivo,* i.e. under physiological conditions. Accordingly, prodrugs are usually transported through hydrophobic biological barriers such as membranes and typically possess superior physicochemical properties in comparison to the parent drug. Gong-Xin et al: « Chapter 3.6. Prodrugs of Phosphonates, Phosphinates and Phosphates », Prodrugs Challenges and Reward Part1. Springer New York, US, vol.5.1, 2007, p. 923-964).

"Stereoisomers" include in particular enantiomers. Thus, the compounds of the invention may be enantiomerically pure or provided as a mixture of enantiomers.

Likewise, all tautomeric forms of the compounds of Formulae (I) and (II) are also intended to be included.

For example, the tautomers of the compounds of the invention include compounds deriving from tautomerisation of a hypoxanthine moiety or a guanine moiety as follows:

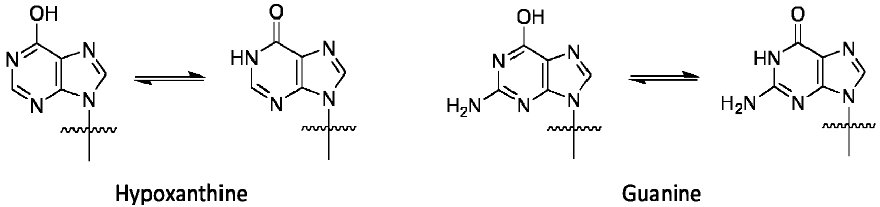

The tautomers of the invention also include for instance oxo derivatives of Formula (II) when A₁ or A₂ is O-R_{3,} as represented below by Formulae (II₁), (II₂), (II₃) and (II₄):

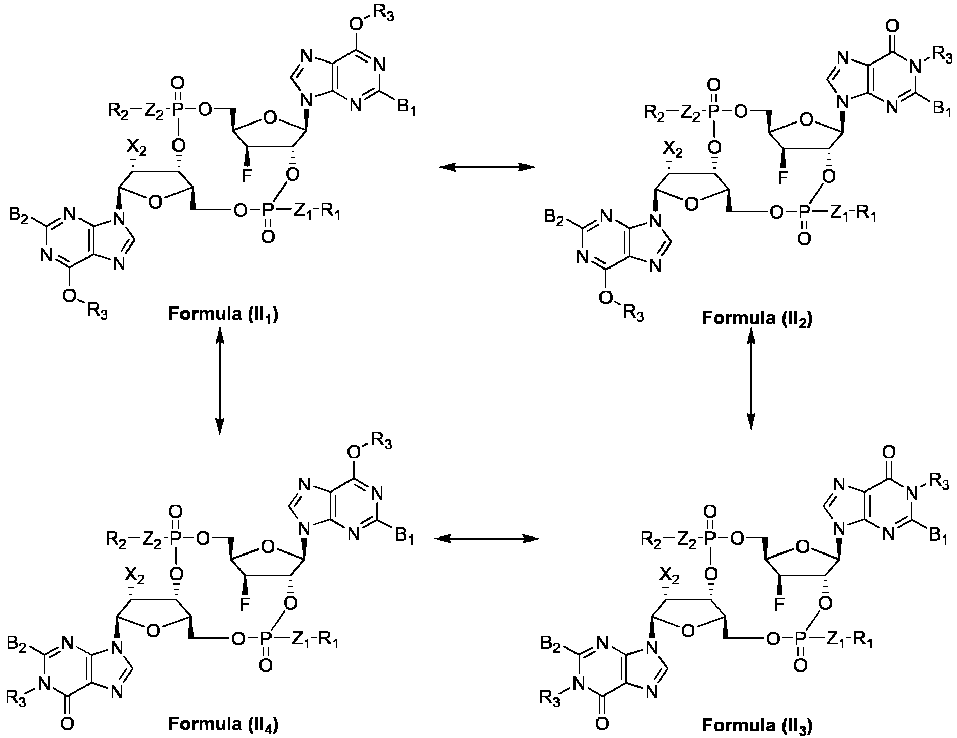

The term "solvate" denotes a compound formed by solvation, for example as a combination of solvent molecules with molecules or ions of a solute. Well known solvent molecules include water, alcohols and other polar organic solvents. Alcohols include methanol, ethanol, n-propanol, isopropanol, n-butanol, isobutanol, and t-butanol. Alcohols also include polymerized alcohols such as polyalkylene glycols (e.g., polyethylene glycol, polypropylene glycol). The best-known and preferred solvent is typically water, and solvate compounds formed by solvation with water are termed hydrates.

A particular class of cyclic dinucleotide compounds of Formula (I) is selected from (3',3') CDNs of Formula (III): 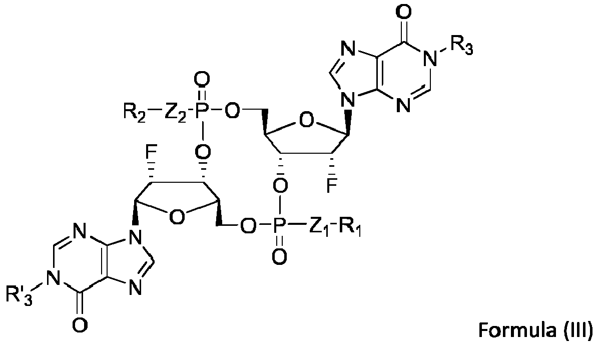 wherein R₁, R₂, Z₁, Z₂, R₃, and R'₃ are as defined above.

A particular class of cyclic dinucleotide compounds of Formula (I) is selected from (3',3') CDNs of Formula (IV): 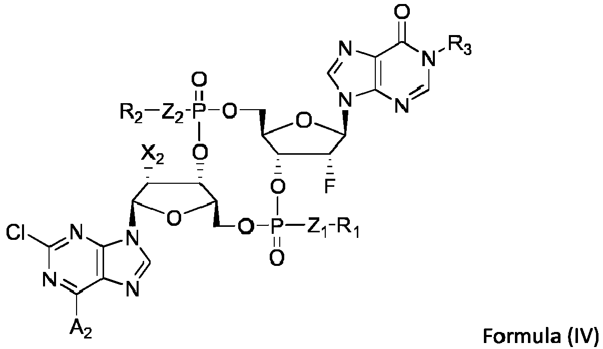 wherein X₂, R₁, R₂, Z₁, Z₂, A₂ and R₃ are as defined above.

A particular class of cyclic dinucleotide compounds of Formula (I) is when A₂ is O-R₃ and B₂ is H, i.e. when the two bases are both hypoxanthine. Compared to cyclic dinucleotides wherein the bases are adenine or guanine, such compounds have the advantage of not being a substrate of endogenous deaminases (ADA: adenosine deaminase or GAH: guanine aminohydrolase). A particular class of cyclic dinucleotide compounds of Formula (II) with the same base is selected from (2',3') CDNs of Formula (V) or (VI): 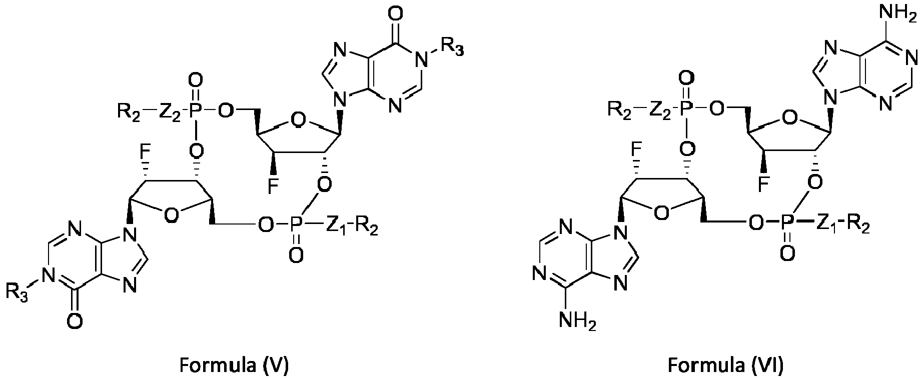 wherein R₁, R₂, Z₁, Z₂ and R₃ are as defined above.

A particular class of cyclic dinucleotide compounds of Formula (II) with two different bases is selected from (2',3') CDNs of Formula (VII) to (X): 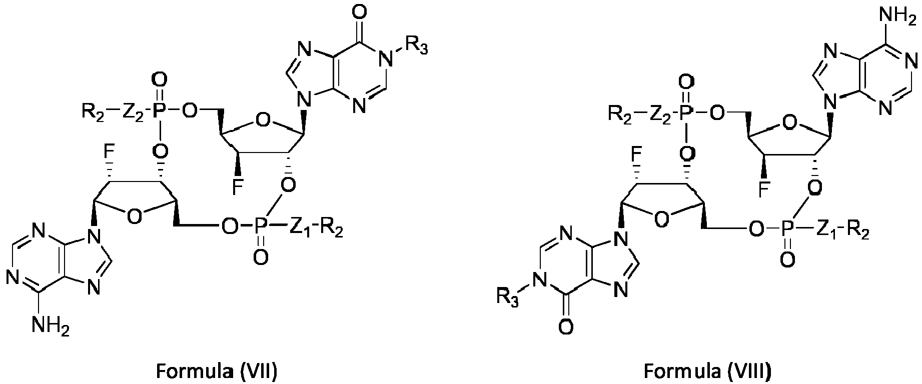 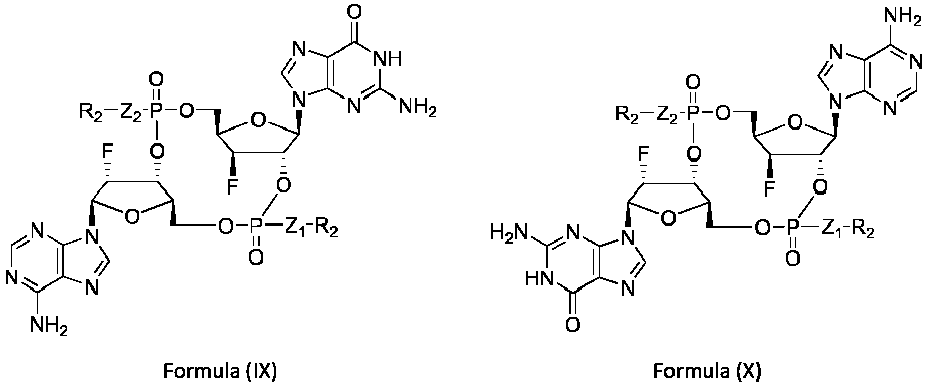 wherein R₁, R₂, Z₁, and Z₂ are as defined above.

A particular class of cyclic dinucleotide compounds of Formulae (I) or (II) is when Z₁ and/or Z₂ are S. Such compounds show a greater activity compared to cyclic dinucleotides of Formulae (I) and (II) when Z₁ and/or Z₂ are O due to an increase resistance to degradation via endogenous nucleases or phosphodiesterase activity. Compounds of Formulae (I) or (II) where Z₁ and/or Z₂ are S might have an enhanced ability to induce type III Interferons compared to compounds of Formulae (I) or (II) where Z₁ and/or Z₂ are O.

A particular class of cyclic dinucleotide compounds of Formulae (I) or (II) is when the two bases are identical, i.e. are both hypoxanthine, guanine or adenine.

A particular class of cyclic dinucleotide compounds of Formulae (I) or (II) is when the two bases are different, e.g. one base is hypoxanthine and the other base is adenine or guanine, or one base is adenine and the other base is hypoxanthine or guanine, or one base is guanine and the other base is adenine or hypoxanthine.

A particular class of cyclic dinucleotide compounds of Formulae (I) or (II) is when X₂ = H.

A particular class of cyclic dinucleotide compounds of Formulae (I) or (II) is when X₂ = F.

A particular class of cyclic dinucleotide compounds of Formulae (I) or (II) is when Z₁ and Z₂ = O.

A particular class of cyclic dinucleotide compounds of Formulae (I) or (II) is when Z₁ and Z₂ = S.

A particular class of cyclic dinucleotide compounds of Formulae (I) or (II) is when Z₁ = O and Z₂ = S.

A particular class of cyclic dinucleotide compounds of Formulae (I) or (II) is when Z₂ = O and Z₁ = S.

A particular class of cyclic dinucleotide compounds of Formulae (I) or (II) is when R₁ and/or R₂ are H.

A particular class of cyclic dinucleotide compounds of Formulae (I) or (II) is when R₁ and/or R₂ are an enzyme-labile group which, together with the oxygen atom or sulfur atom to which it is attached, provides OH or SH *in vivo.*

A particular class of cyclic dinucleotide compounds of Formulae (I) and (II) is selected from the group consisting of:

| **Code** | **Structure / Name** | **Formula** |
|---|---|---|
| **CL735** | 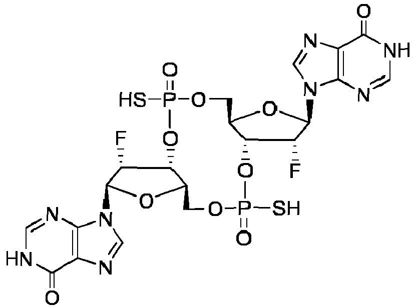 | **Formula (III)** |
| | | Z₁ = Z₂ = S |
| | | R₁ = R₂ = R₃ = R_{3'} = H |
| | **c-di[2'FdIMP(S)]** | |
| **CL744** | 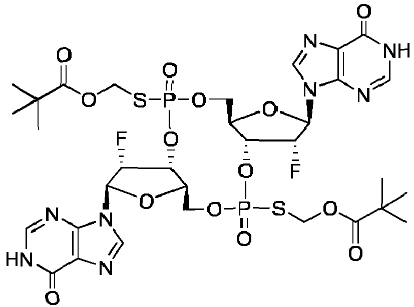 | **Formula (III)** |
| | | Z₁ = Z₂ = S |
| | | R₁ = R₂ = POM |
| | | R₃ = R_{3'} = H |
| | **c-di[2'FdIMP(S-POM)]** | |
| **CL733** | 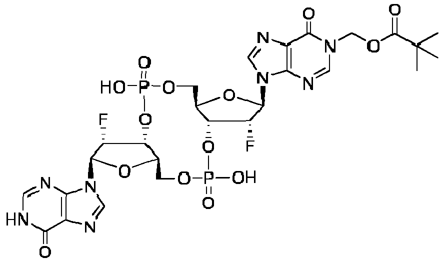 | **Formula (III)** |
| | | Z₁ = Z₂ = O |
| | | R₁ = R₂ = R'₃ = H |
| | | R₃ = POM |
| | **c-[2'FdI(POM)MP-2'FdIMP]** | |
| **CL734** | 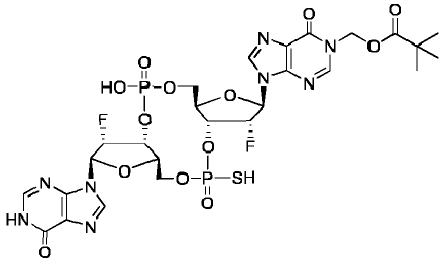 | **Formula (III)** |
| | | Z₁ = O and Z₂ = S |
| | | R₁ = R₃ = R'₃ = H |
| | | R₃ = POM |
| | **c-[2'FdI(POM)MP-2'FdIM(PS)]** | |
| **CL736** | 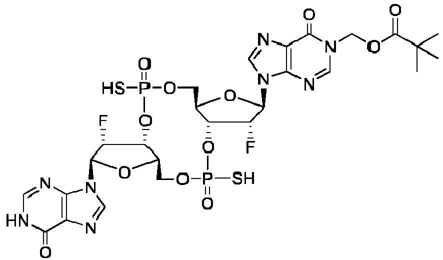 | **Formula (III)** |
| | | Z₁ = Z₂ = S |
| | | R₁ = R₂ = R'₃ = H |
| | | R₃ = POM |
| | **c-[2'FdI(POM)M(PS)-2'FdIM(PS)]** | |
| **CL737** | 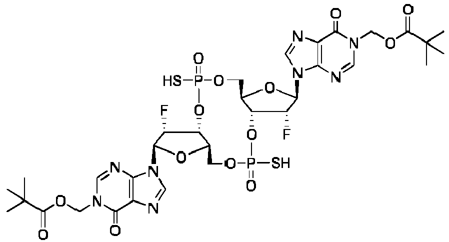 | **Formula (III)** |
| | | Z₁ = Z₂ = S |
| | | R₁ = R₂ = H |
| | | R₃ = R'₃ = POM |
| | **c-di[2'FdI(POM)M(PS)]** | |
| **CL742** | 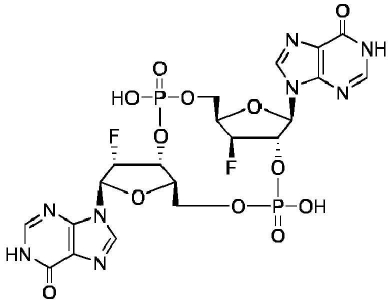 | **Formula (V)** |
| | | Z₁ = Z₂ =O |
| | | R₁ = R₂ = R₃ = R'₃ = H |
| | **(2',3')c-[3'F3'XylodIMP-2'FdIMP]** | |
| **CL752** | 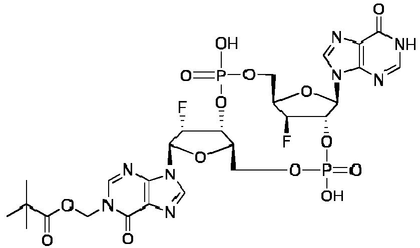 | **Formula (V)** |
| | | Z₁ = Z₂ =O |
| | | R₁ = R₂ = R₃ = H |
| | | R'₃ = POM |
| | **(2',3')c-[3'F3'XylodIMP-2'FdI(POM)MP]** | |
| **CL746** | 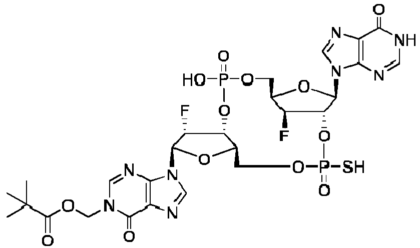 | **Formula (V)** |
| | | Z₁ = S and Z₂ = O |
| | | R₁ = R₂ = R₃ = H |
| | | R'₃ = POM |
| | **(2',3')c-[3'F3'XylodIM(PS)-2'FdI(POM)MP]** | |
| **CL753** | 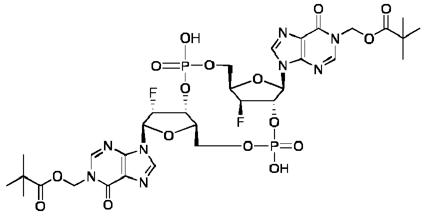 | **Formula (V)** |
| | | Z₁ = Z₂ = O |
| | | R₁ = R₂ = H |
| | | R₃ = R'₃ = POM |
| | **(2',3')c-[3'F3'XylodI(POM)M(PS)-2'FdI(POM)MP]** | |
| **CL747** | 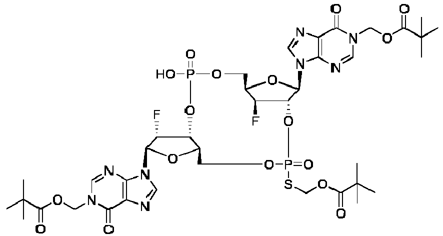 | **Formula (V)** |
| | | Z₁ = S and Z₂ = O |
| | | R₂ = H |
| | | R₁ = R₃ = R'₃ = POM |
| | **(2',3')c-[xylo3'F3'dI(POM)M(PS-POM)-2'FdI(POM)MP]** | |
| **CL785** | 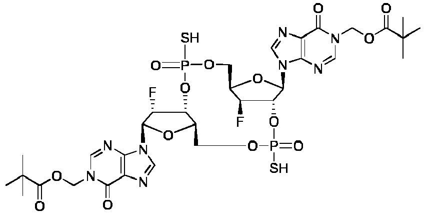 | **Formula (V)** |
| | | Z₁ = Z₂ =S |
| | | R₂ = H |
| | | R₃ = R'₃ = POM |
| | **(2',3')c-[xylo3'F3'dI(POM)M(PS)-2'FdI(POM)M(PS)]** | |
| **CL786** | 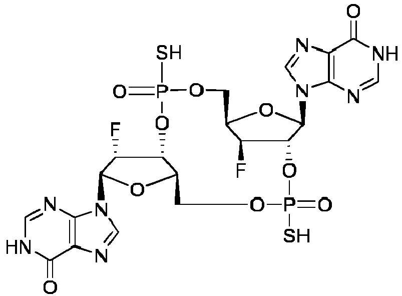 | **Formula (V)** |
| | | Z₁ = Z₂ =S |
| | | R₁ = R₂ = R₃ = R'₃ = H |
| | **(2',3')c-[xylo3'F3'dIM(PS)-2'FdIM(PS)]** | |
| **CL787** | 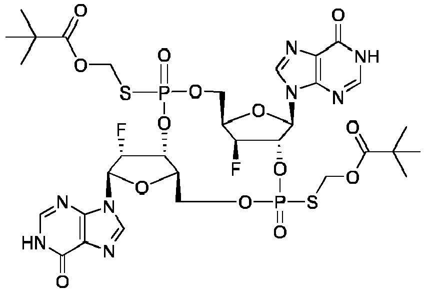 | **Formula (V)** |
| | | Z₁ = Z₂ =S |
| | | R₁ = R₂ = POM |
| | | R₃ = R'₃ = H |
| | **(2',3')c-[xylo3'F3'dIM(PS-POM)-2'FdIM(PS-POM)]** | |
| **CL761** | 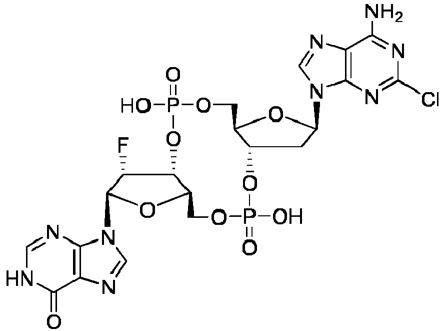 | **Formula (IV)** |
| | | X₂ = H |
| | | Z₁ = Z₂ = O |
| | | R₁ = R₂ = R₃ = H |
| | | A₂ = NH₂ |
| | **(3'3')c-(2CIdAMP-2'FdIMP)** | |
| **CL762** | 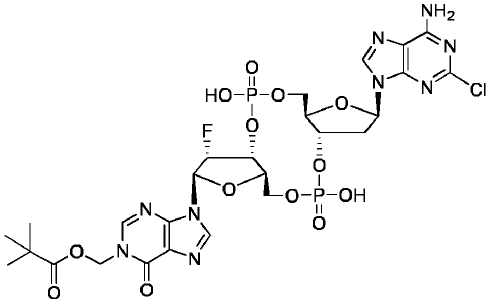 | **Formula (IV)** |
| | | X₂ = H |
| | | Z₁ = Z₂ = O |
| | | R₁ = R₂ = H |
| | | R₃ = POM |
| | | A₂ = NH₂ |
| | **(3'3')c-[2CIdAMP-2'FdI(POM)MP]** | |
| **CL763** | 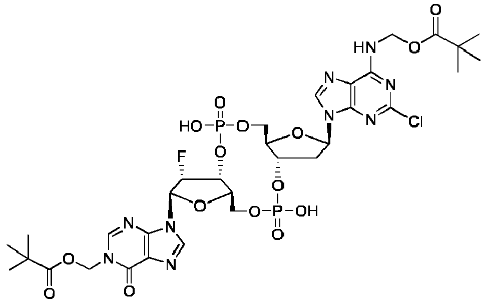 | **Formula (IV)** |
| | | X₂ = H |
| | | Z₁ = Z₂ = O |
| | | R₁ = R₂ = H |
| | | R₃ = POM |
| | | A₂ = NH-POM |
| | **(3'3')c-[2CIdA(POM)MP-2'FdI(POM)MP]** | |
| **CL731** | 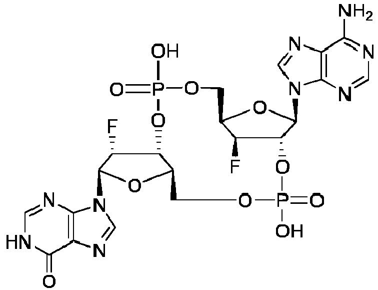 | **Formula (IX)** |
| | | Z₁ = Z₂ = O |
| | | R₁ = R₂ = H |
| | **(2',3')c-(3'F3'XylodAMP-2'FdIMP)** | |
| **CL764** | 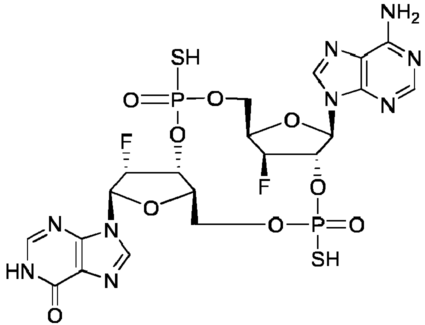 | **Formula (IX)** |
| | | Z₁ = Z₂ = S |
| | | R₁ = R₂ = H |
| | **(2',3')c-[3'F3'XylodAM(PS)-2'FdIM(PS)]** | |
| **CL765** | 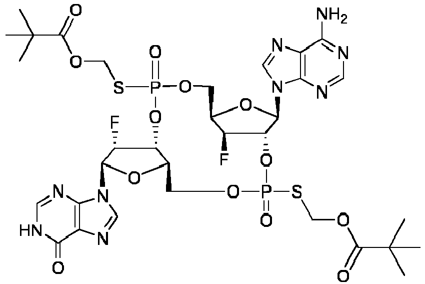 | **Formula (IX)** |
| | | Z₁ = Z₂ = S |
| | | R₁ = R₂ = POM |
| | **(2',3')c-[3'F3'XylodAM(PS-POM)-2'FdIM(PS-POM)]** | |
| **CL777** | 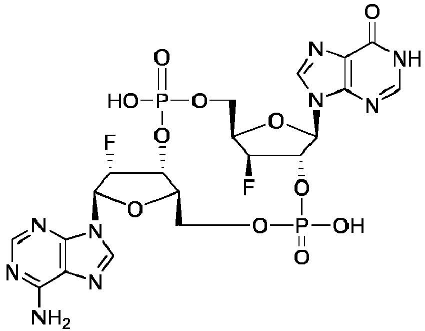 | **Formula (VIII)** |
| | | Z₁ = Z₂ = O |
| | | R₁ = R₂ = R_{3'} = H |
| | **(2',3')c-(3'F3'XylodIMP-2'FdAMP)** | |
| **CL779** | 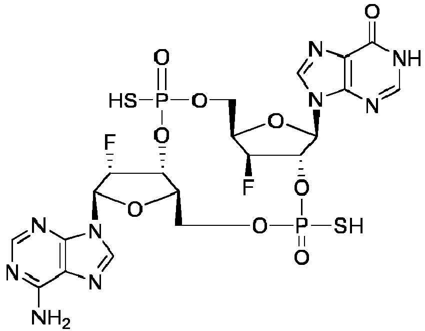 | **Formula (VIII)** |
| | | Z₁ = Z₂ = S |
| | | R₁ = R₂ = R_{3'} = H |
| | **(2',3')c-[3'F3'XylodIM(PS)-2'FdAM(PS)]** | |
| **CL780** | 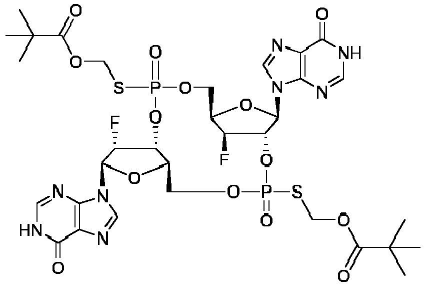 | **Formula (VIII)** |
| | | Z₁ = Z₂ = S |
| | | R₁ = R₂ = POM |
| | | R_{3'} = H |
| | **(2',3')c-[3'F3'XylodIM(PS-POM)-2'FdAM(PS-POM)]** | |
| **CL781** | 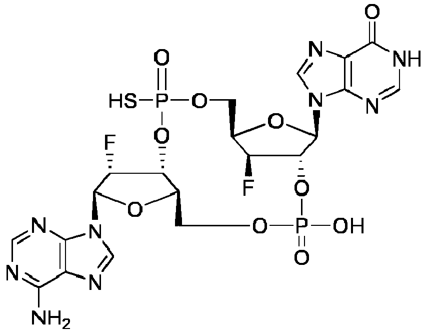 | **Formula (VIII)** |
| | | Z₁ = O, Z₂ = S |
| | | R₁ = R₂ = R_{3'} = H |
| | **(2',3')c-[3'F3'XylodIMP-2'FdAM(PS)]** | |
| **CL784** | 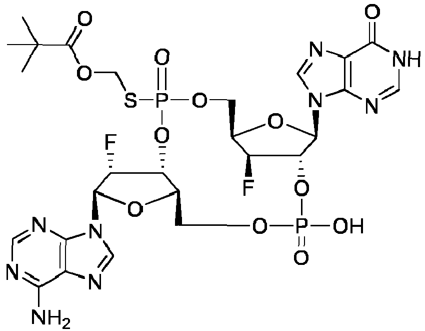 | **Formula (VIII)** |
| | | Z₁ = O, Z₂ = S |
| | | R₁ = R_{3'} = H |
| | | R₂ = POM |
| | **(2',3')c-[3'F3'XylodIMP-2'FdAM(PS-POM)]** | |
| **CL710** | 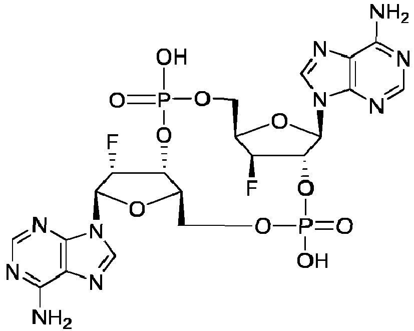 | **Formula (VI)** |
| | | Z₁ = Z₂ = O |
| | | R₁ = R₂ = H |
| | (2',3')c-[xylo3'F3'dAMP-2'FdAMP] | |
| **CL714** | 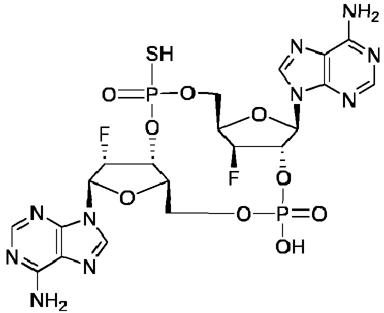 | **Formula (VI)** |
| | | Z₁ = O, Z₂ = S |
| | | R₁ = R₂ = H |
| | **(2',3')c-[xylo3'F3'dAM(PS)-2'FdAMP]** | |
| **CL760** | 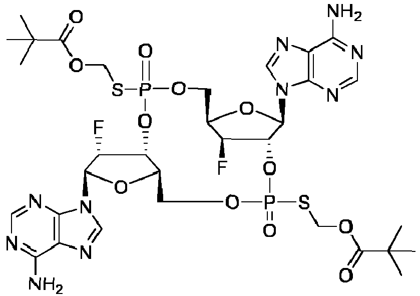 | **Formula (VI)** |
| | | Z₁ = Z₂ = S |
| | | R₁ = R₂ = POM |
| | **(2',3')c-[xylo3'F3'dAM(PS-POM)-2'FdAM(PS-POM)]** | |
| **CL766** | 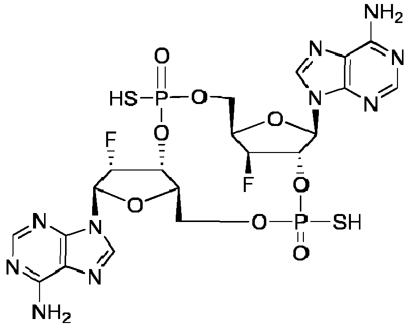 | **Formula (VI)** |
| | | Z₁ = Z₂ = S |
| | | R₁ = R₂ = H |
| | **(2',3')c-[xylo 3'F3'dAM(PS)-2'FdAM(PS)]** | |
| **CL672** | 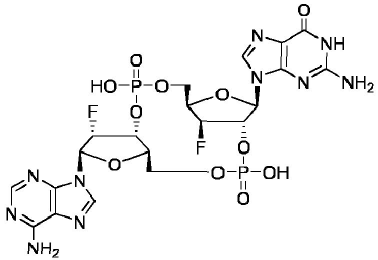 | **Formula (IX)** |
| | | Z₁ = Z₂ = O |
| | | R₁ = R₂ = H |
| | **(2',3')c-[xylo3'F3'dGMP-2'FdAMP]** | |
| **CL739** | 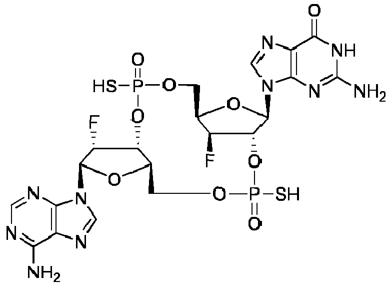 | **Formula (IX)** |
| | | Z₁ = Z₂ = S |
| | | R₁ = R₂ = H |
| | **(2',3')c-[xylo3'F3'dGM(PS)-2'FdAM(PS)]** | |
| **CL751** | 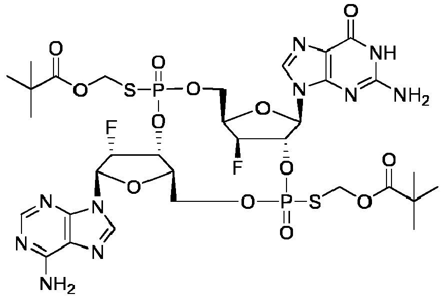 | **Formula (IX)** |
| | | Z₁ = Z₂ = S |
| | | R₁ = R₂ = POM |
| | **(2',3')c-[3'F3'XylodGM(PS-POM)-2'FdAM(PS-POM)]** | |
| **CL715** | 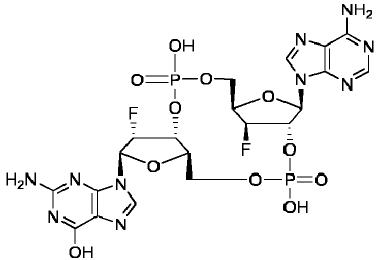 | **Formula (X)** |
| | | Z₁ = Z₂ = O |
| | | R₁ = R₂ = H |
| | **(2',3')c-[3'F3'XylodAMP-2'FdGMP]** | |

The cyclic dinucleotides of the present invention induce Type I interferons and/or pro-inflammatory cytokines *in vitro* in human cells, animal cells and human blood. The cytokine-induction activity of these cyclic dinucleotides requires the presence of STING, as confirmed by *in vitro* experiments in human or animal cells with invalidated gene of STING.

The cyclic dinucleotides of the invention are agonists of the receptor STING.

The term "agonist" refers to any substance that activates a biologic receptor *in vitro* or *in vivo* to provoke a physiological response.

"STING" is an abbreviation of "stimulator of interferon genes", which is also known as "endoplasmic reticulum interferon stimulator (ERIS)", "mediator of IRF3 activation (MITA)", "MPYS" or "transmembrane protein 173 (TM173)". STING is a transmembrane receptor protein that in humans is encoded by the gene *TMEM173.* Activation of STING by cyclic dinucleotides (CDN) leads to activation of the IRF3 and NF-kB pathways and consequently, to induction of Type I interferons and of pro-inflammatory cytokines, respectively. In response to viral infection, STING activates STAT6 (signal transducer and activator of transcription 6) to induce (Th2-type), increase (IL-12) or decrease (IL-10) production of various cytokines, including the chemokines CCL2, CCL20, and CCL26 (Chen et al., 2011).

The term **"STING agonist"** herein refers to a substance that activates the receptor STING *in vitro* or *in vivo.* According to the invention, a compound is deemed to be a STING agonist if:
- it induces Type I interferons *in vitro* in human or animal cells that contain active STING; and
- it does not induce Type I interferons *in vitro* in human or animal cells that do not contain active STING.

A typical test to ascertain whether a ligand is a STING agonist is to incubate the ligand in a wild-type human or animal cell line and in the corresponding cell line in which the STING coding gene has been genetically inactivated by small or long base deletions (e.g. a homozygous STING knockout cell line). An agonist of STING will induce Type I interferons in the wild-type cells but will not induce Type I interferons in the cells in which the STING coding gene has been inactivated.

The cyclic dinucleotides of the invention induce Type I interferons *in vitro* in human or animal cells that contain active STING. However, they do not induce Type I interferons *in vitro* in human or animal cells that do not contain active STING.

The present invention concerns CDNs containing non-natural fluorinated nucleoside in ribose and xylose series with guanine, adenine and hypoxanthine as heterocyclic base.

Specifically, it concerns CDNs containing nucleoside analogs and more specifically, adenosine, guanosine and inosine analogs with:
- 2'-fluoro-2'-deoxy-β,D-ribofuranoyl-*N⁹*-Hypoxanthine derivatives of Formula (XIV): 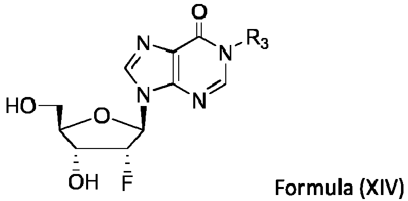 within R₃ is as defined above for CDNs of Formulae (III), (IV), (V), and (VIII);
- 2'-fluoro-2'-deoxy-β,D-ribofuranoyl-*N⁹*-Adenine: 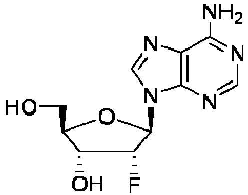 for CDNs of Formulae (VI), and (IX);
- 3'-fluoro-3'-deoxy-β,D-xylofuranoyl-*N⁹*-Adenine: 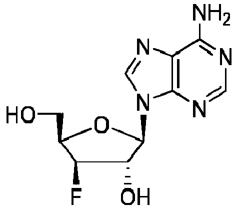 for CDNs of Formulae (VI), (VIII), and (X);
- 3'-fluoro-3'-deoxy-β,D-xylofuranoyl-*N⁹*-Guanine: 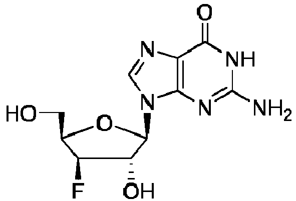 for CDNs of Formula (IX);
- 3'-fluoro-3'-deoxy-β,D-xylofuranoyl-*N⁹*-Hypoxanthine derivatives of Formula (XV): 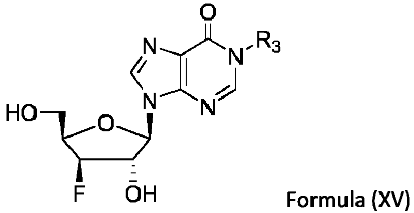 within R₃ is as defined above for CDNs of Formulae (V), and (VII).

The present invention concerns CDNs of Formulas (I) and (II) containing natural and non-natural internucleosidic link:
- Phosphodiester link: 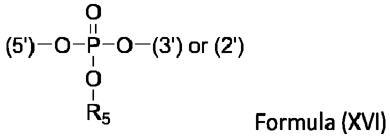 wherein R₅ is H.
- Phosphorothioate diester link: 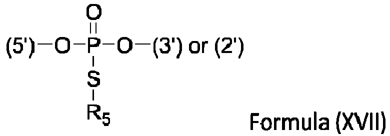 wherein R₅ is H.
- Phosphorothioate triester link: 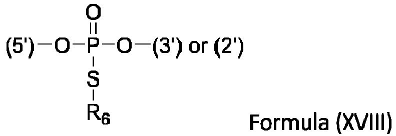 wherein R₆ is:
   o a POM group: 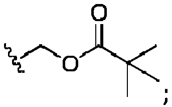
   o a SATE group: 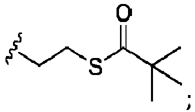
   o a group of formula (IXX): 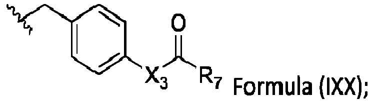 wherein X₃ is O, S, NH and R₇ is an C₁-C₃ alkyl.

It will be appreciated that phosphorothioate diesters or triesters CDNs of Formula (I) may be a mixture of stereoisomers as indicated below:
- Stereoisomers of CDNs of Formula (I) where Z₁ = Z₂ = S are: 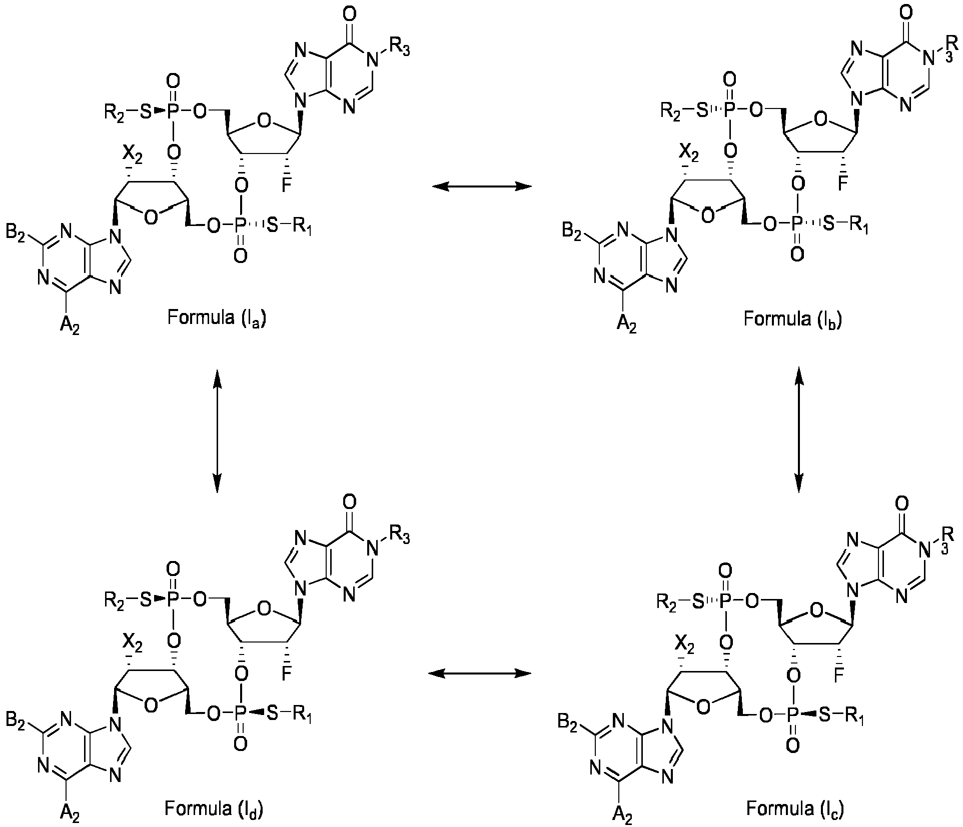 wherein X₂, R₁, R₂, A₂, B₂ and R₃ are as defined above.
- Stereoisomers of CDNs of Formula (I) where Z₁ = O and Z₂ = S are: 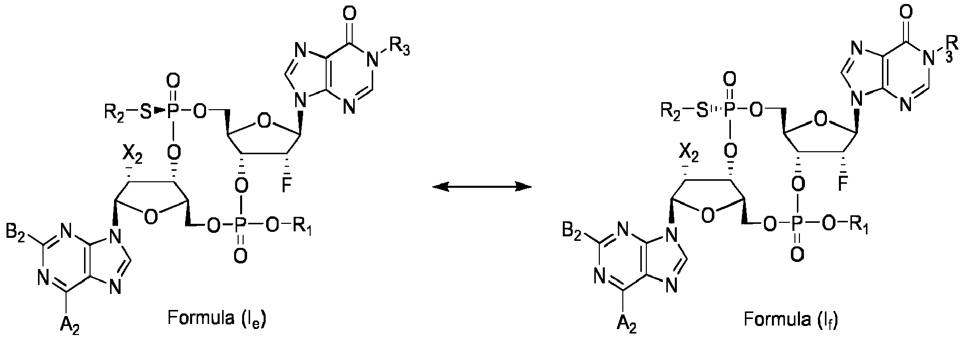 wherein X₂, R₁, R₂, A₂, B₂ and R₃ are as defined above.

It will be appreciated that phosphorothioate diesters or triesters CDNs of Formula (II) may be a mixture of stereoisomers as indicated below:
- Stereoisomers of CDNs of Formula (II) where **Z₁ = Z₂ = S** are: 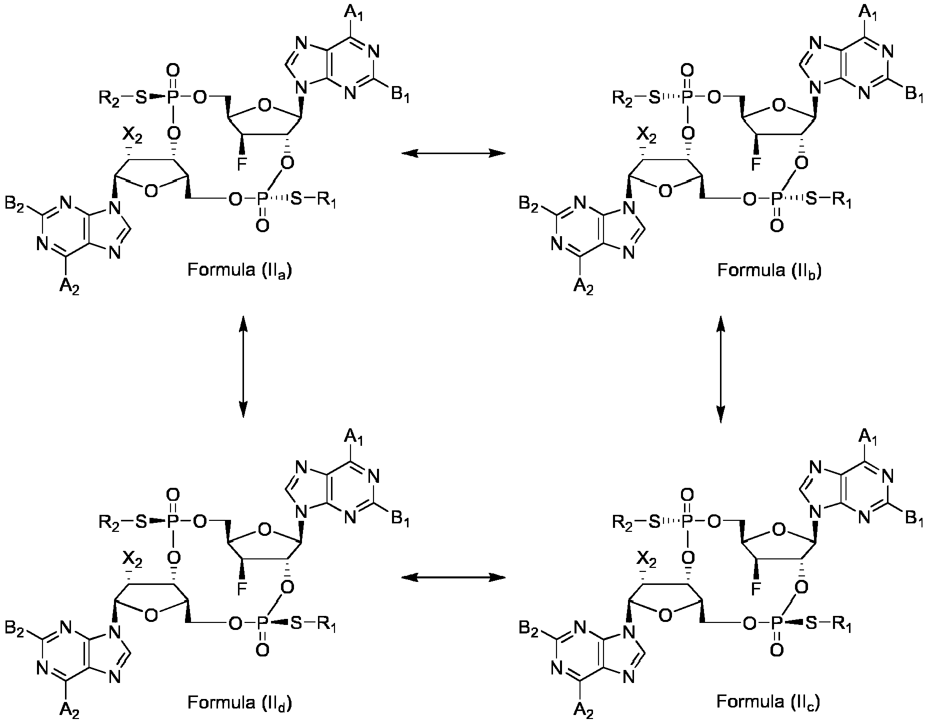 wherein X₂, R₁, R₂, A₁, B₁, A₂ and B₂ are as defined above.
- Stereoisomers of CDNs of Formula (II) where **Z₁ = O and Z₂ = S** are: 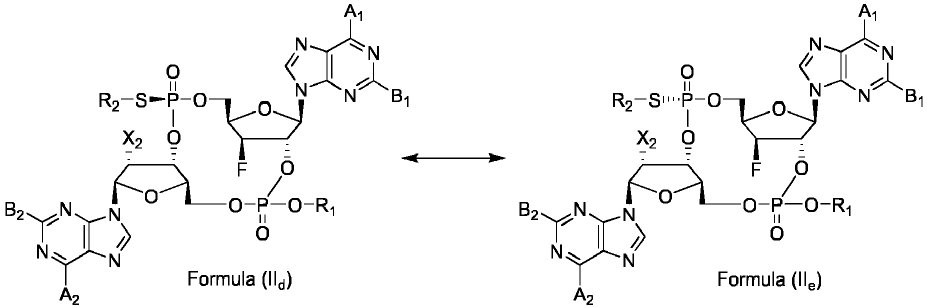 wherein X₂, R₁, R₂, A₁, B₁, A₂ and B₂ are as defined above.
- Stereoisomers of CDNs of Formula (II) where **Z₁ = S and Z₂ = O** are: 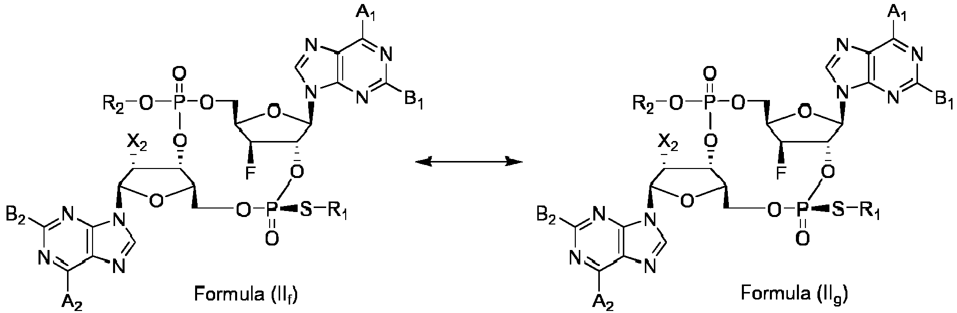 wherein X₂, R₁, R₂, A₁, A₂ and B₂ are as defined above.

### Use of nucleoside analogue for CDNs synthesis

| **Active** | **Biological activity and/or synthetic methods** |
|---|---|
| 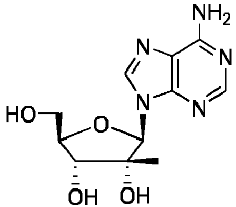 | **2'-C-methyladenosine** and **2'- C-methylguanosine** (aka, INX-08032) have been found to be potent nucleoside inhibitors of HCV RNA replication *in vitro,* see, for example, Eldrup et al., J. Med. Chem. (2004) 47, 5284-5297. |
| **2'-C-methyladenosine** | |
| CAS 15397-12-3 | |
| 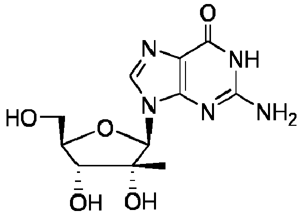 | For example, U.S. Pat. No. 3,480,613, discloses synthesis of 2'-C-methylpurines and pyrimidines. For example, 2'-C-methyladenosine, is prepared in Example 34. See also, U.S. Pat. No. 3,480,613 and Walton et al. J. Am. Chem. Soc. (1966), 88(19), 4524-4525. Eldrup et al., J. Med. Chem. (2004) 47, 2283-2295. See also, WO 2001/090121; WO 2004/058792; and Eldrup et al. J. Med. Chem. (2004), 47(9), 2283-2295. |
| **2'-C-methylguanosine** | |
| CAS 374750-30-8 | |
| 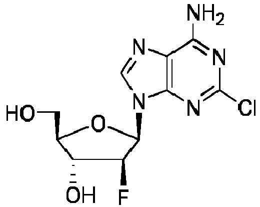 | **Clofarabine,** is an antimetabolite that inhibits DNA synthesis and resists deamination by adenosine deaminase. The preparation of clofarabine is described in: (Watanabe et al EP 219829 and U.S. Pat. No. 4,918,179; Montgomery et al., J. Med. Chem. (1992) 35, 397; and an improved synthesis is described in Bauta et al.Org.Process Res. Dev. (2004) 8,889. |
| **Clofarabine** | |
| CAS 123318-82-1 | |
| 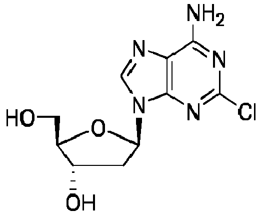 | **Cladribine,** is a substituted purine nucleoside with antileukemic activity. Cladribine is prepared as intermediate in synthesis of 2-deoxynucleosides, see, e.g., Venner, Ber. (1960) 93, 140; Ikehara et al. J. Am. Chem. Soc. (1963) 85, 2344; Ikehara et al. J. Am. Chem. Soc. (1965) 87, 606. |
| **Cladribine** | Christensen et al., J. Med. Chem. (1972) 15, 735 discloses thesynthesis and biological activity, while the following disclose a stereospecific synthesis: Kazimierczuk et al., J. Am. Chem. Soc. (1984) 106, 6379; R. K. Robins, G. R. Revankar, EP 173059; and U.S. Pat. No. 4,760,137. |
| CAS 4291-63-8 | |
| 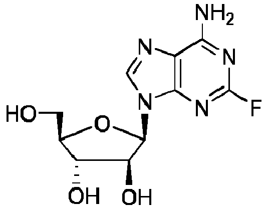 | **Fludarabine** is an adenosine deaminase-resistant purine nucleoside antimetabolite. The preparation of this molecule is described in Montgomery et al. J. Med. Chem. (1969) 12, 498, while improved syntheses are disclosed in Montgomery et al., J. Heterocycl. Chem. (1979) 16, 157 and U.S. Pat. No. 4,210,745. |
| **Fludarabine** | |
| CAS 21679-14-1 | |
| 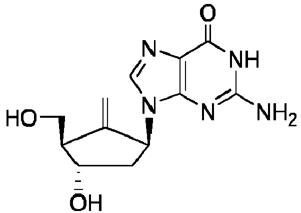 | Entecavir is a deoxyguanine nucleoside analog that inhibits hepatitis B virus (HBV) DNA polymerase. Entecavir can be prepared as disclosed in U.S. Pat. No. 5,206,244 to Zahler et al. Improvements to the synthetic route of Zahler et al. are described in WO 98/09964 to Bisacchi and Sundeen. |
| **Entecavir** | |
| CAS 142217-69-4 | |
| 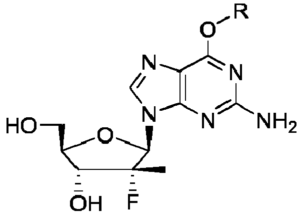 | **Compounds 1a, 1b and 1c** can be prepared, for example, by the synthetic route of Du et al., WO 2009/152095. The compounds have utility, for example, in the preparation of nucleoside cyclic phosphates and can be useful in inhibiting RNA- dependent RNA viral replication and are useful as inhibitors of HCV NS5B polymerase, as inhibitors of HCV replication and for treatment of hepatitis C infection in mammals. |
| **Compounds 1** | |
| **1a R = Me** | |
| **1b R Et** | |
| **1c** R = iPr | |
| 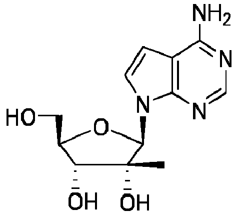 | **7-Deaza-2'-C-methyladenosine** is known to be an inhibitor of HCV RNA replication *in vitro,* and can be prepared as described in, for example, Eldrup et al. J. Med. Chem. (2004) 47, 5284-5297. Eldrup Scheme 1, shows a synthetic route to compound 9 at pages 5293-5294. |
| **7-Deaza-2'-C-methyladenosine** | See also WO 2003/068244; WO 2002/057425; and WO 2002/057287. |
| CAS 443642-29-3 | |
| 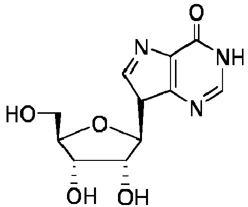 | **9-Deazainosine, 1,5-dihydro-7-β-D-** ribofuranosyl-4H-**Pyrrolo[3,2-d]pyrimidin-4-one,** finds utility in e.g., potential treatment of visceral leishmaniasis. Berman et al. Antimicrob. Agents Chemother. (1987) 31(1), 111-113. It can be prepared by the route disclosed in WO 2007/002191, Chand et al. (disclosed at page 7, and Scheme 3, page 21). |
| **9-Deazainosine** | |
| CAS 89458-19-5 | |
| 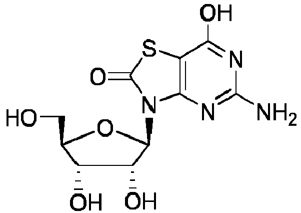 | **Isatoribine** is a selective agonist of TLR7 which possesses antitumor, antiviral and immune system enhancing properties. Isatoribine can be produced by the method disclosed in U.S. Pat. No. 5,041,426, Robins and Cottam(Synthesis is disclosed in Examples 1 and 2, col. 8- 9). See also U.S. Pat. No. 4,880,784. |
| **Isatoribine** | |
| CAS 122970-40-5 | |
| 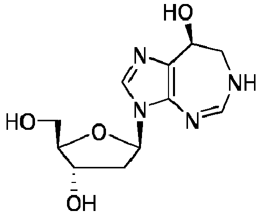 | **Pentostatin** is an adenosine deaminase inhibitor with antineoplastic utility originally isolated from Steptomyces antibioticus. Woo et al., J. Heterocyclic Chem., (1974) 11, 641; Showalter et al. J. Med. Chem. (1982) 47, discloses a multigram synthetic route for pentostatin. See also Baker et al. J. Heterocyclic Chem. (1983) 20(3), 629-634; Showalter, et al. J. Med. Chem. (1983), 26(10), 1478-1482; and Chan et al. J. Org. Chem. (1982), 47(18), 3457-3464. |
| **Pentostatin** | |
| CAS 53910-25-1 | |
| 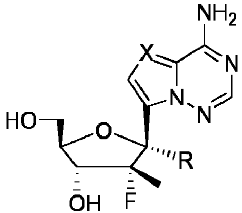 | Compounds 2a-d may be useful for the treatment of HCV. Procedures for preparing compounds 2a-d are disclosed in US 2011/0070194. |
| **Compounds 2** | |
| **2a** R = H, X = CH | |
| **2b** R = CN, X = CH | |
| **2c** R = HX = N | |
| **2d** R = CN, X = N | |
| 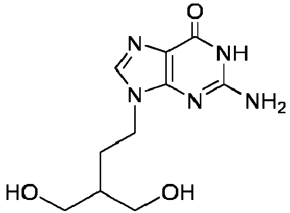 | **Compound 3,** is the carba analog of ganciclovir, which shows activity against several herpes viruses. The preparation of compound 13 is disclosed in Pandit et al., Synth. Commun. (1972) 2, 345; U.S. Pat. No. 5,075,445; Hamden et al., J. Med. Chem. (1987) 30, 1636; and Hannah et al., J. Heterocycl. Chem. (1989) 26, 1261. |
| **Compound 3** | |
| CAS 39809-25-1 | |
| 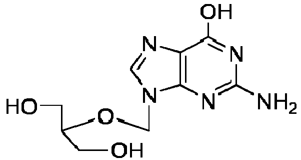 | **Ganciclovir,** is a nucleoside analog structurally related to acyclovir. Its preparation is described in: U.S. Pat. No. 4,355,032; Ogilvie et al., Can. J. Chem. (1982) 60, 3005; Ashton et al., Biochem. Biophys. Res. Commun. (1982) 108, 1716; and Martin et al., J. Med. Chem. (1983) 26, 759. |
| **Ganciclovir** | |
| CAS 82410-32-0 | |
| 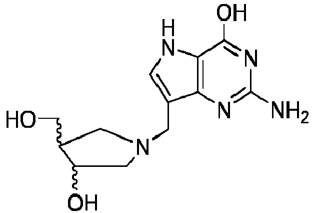 | **Compound 4a** possesses utility as a phosphorylase (PNP), methylthioadenosine phosphorylase (MTAP), 5'-methylthioadenosine nucleosidase (MTAN) and/or a nucleoside hydrolase inhibitor. Compound 16a can also be prepared by the route disclosed in WO 2007/069923, Furneaux et al. |
| **Compound 4** | **Compound 4b** possesses utility as a phosphorylase (PNP) inhibitor and can be prepared by the route disclosed in WO 2004/0698856, Evans et al. |
| **4a** (S,S) CAS 942201-43-6 | |
| **4b** (R,R) CAS 548486-61-9 | |

### Pharmaceutical compositions

Another object of the invention is a pharmaceutical composition comprising a cyclic dinucleotide compound of Formulae (I) or (II) and a pharmaceutically acceptable excipient.

In one embodiment, the pharmaceutical composition is an immunogenic composition or vaccine adjuvant comprising a cyclic dinucleotide compound of Formulae (I) or (II).

In one embodiment, the pharmaceutical composition is a composition comprising a cyclic dinucleotide compound of Formulae (I) or (II) and one or more immunostimulatory agents.

In one embodiment, the pharmaceutical composition is a vaccine or immunogenic composition comprising an antigen or antigen composition and a cyclic dinucleotide compound of Formulae (I) or (II).

The pharmaceutical composition may comprise conventional excipients in conventional proportions, with or without additional active compounds or principles, and the unit dosage forms may contain any suitable effective amount of the active ingredient commensurate with the intended daily dosage range to be employed.

The excipient(s) must be acceptable in the sense of being compatible with the other ingredients of the composition and not deleterious to the recipient thereof.

Generally, the compound of the invention is administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

Pharmaceutical compositions may be presented in unit dose forms containing a predetermined amount of active ingredient per unit dose. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions.

Preferred unit dosage compositions are those containing a daily dose or sub-dose, or an appropriate fraction thereof, of an active ingredient. Such unit doses may therefore be administered once or more than once a day. Such pharmaceutical compositions may be prepared by any of the methods well known in the pharmacy art.

Pharmaceutical compositions may be adapted for administration by any appropriate route, for example by the oral (including buccal or sublingual), rectal, inhaled, intranasal, topical (including buccal, sublingual or transdermal), vaginal or injectable (including subcutaneous, intramuscular, parenteral, intravenous or intradermal) route. Such compositions may be prepared by any method known in the art of pharmacy, for example by bringing into association the active ingredient with the carrier(s) or excipient(s).

### Use of the compounds of the invention

Another object of the present invention is the cyclic dinucleotides of Formulae (I) and (II) for use in a therapeutic treatment in humans or animals.

The cyclic dinucleotide compound of Formulae (I) or (II) may be used as therapeutic agents in a monotherapy or in a combination therapy, such as chemoimmunotherapy.

The term "therapeutic agent" refers to one or more substances that are administered to a human or animal in order to achieve some kind of therapeutic effect in that human or animal, including to prevent, cure, or mitigate the effects of, infection or disease, and/or to otherwise improve the health of that human or animal.

The term "monotherapy" refers to the use of a single substance and/or strategy to treat a human or animal in any clinical or medical context, as opposed to the use of multiple substances and/or strategies to treat a human or animal in the same clinical or medical context, regardless of whether the multiple substances and/or strategies are used sequentially in any order or concurrently.

One object of the invention is the cyclic dinucleotide compound of Formulae (I) or (II) for use in the treatment of a disease that may be alleviated by the induction of an immune response via the STING pathway.

One particular aspect of the invention is a cyclic dinucleotide compound of Formulae (I) or (II) for use as an immunomodulatory agent.

Indeed, the cyclic dinucleotide of Formulae (I) and (II) can be administered as immunotherapy to a human or an animal to induce *in vivo* production of one or more cytokines that are therapeutically beneficial to that human or animal. This type of immunotherapy could be used alone or in combination with other treatment strategies, whether sequentially in any order, or concurrently. It could be used to prevent, cure, and/or mitigate the effects of, infection or disease in that human or animal, and/or to modulate the immune system of that human or animal to achieve some other therapeutic benefit.

The term "immunotherapy" refers to any medical treatment in which one or more components of a human's or animal's immune system is deliberately modulated in order to directly or indirectly achieve some therapeutic benefit, including systemic and/or local effects, and preventative and/or curative effects. Immunotherapy can involve administering one or more immune agents (see definition above), either alone or in any combination, to a human or animal subject by any route (*e.g*. orally, intravenously, dermally, by injection, by inhalation, etc.), whether systemically, locally or both. "Immunotherapy" can involve provoking, increasing, decreasing, halting, preventing, blocking or otherwise modulating the production of cytokines, and/or activating or deactivating cytokines or immune cells, and/or modulating the levels of immune cells, and/or delivering one or more therapeutic or diagnostic substances to a particular location in the body or to a particular type of cell or tissue, and/or destroying particular cells or tissue. Immunotherapy can be used to achieve local effects, systemic effects or a combination of both.

The term "immune system" refers to the ensemble, or to any one or more components, of the molecules, substances (*e.g.* bodily fluids), anatomic structures (*e.g.* cells, tissue and organs) and physiologic processes involved in preventing infection in the body, in protecting the body during infection or during disease, and/or in helping the body to recuperate after infection or disease. A complete definition of "immune system" is beyond the scope of this patent; however, this term should be understood by any ordinary practitioner in the field.

The term "immune agent" refers to any endogenous or exogenous substance that can interact with any one or more components of the immune system. The term "immune agent" includes antibodies, antigens, vaccines and their constituent components, nucleic acids, synthetic drugs, natural or synthetic organic compounds, cytokines, natural or modified cells, synthetic analogs thereof, and/or fragments thereof.

The term "immunosuppressed" describes the state of any human or animal subject whose immune system is functionally diminished, deactivated or otherwise compromised, or in whom one or more immune components is functionally diminished, deactivated or otherwise compromised.

"Immunosuppression" can be the cause, consequence or byproduct of disease, infection, exhaustion, malnutrition, medical treatment or some other physiologic or clinical state.

The terms "immunomodulating substance", "immunomodulatory substance", "immunomodulatory agent" and "immunomodulator", used here synonymously, refer to any substance that, upon administration to a human or animal, directly influences the functioning of the immune system of that human or animal. Examples of common immunomodulators include, but are not limited to, antigens, antibodies and small-molecule drugs.

In one embodiment, the cyclic dinucleotides of the present invention are used for cytokine induction as immunotherapy of immunosuppressed individuals.

The present invention thus discloses a method for inducing cytokine in immunosuppressed individuals, said method comprising administering to a patient in need thereof a cyclic dinucleotide of Formulae (I) and (II).

Another aspect of the invention is a cyclic dinucleotide compound of Formulae (I) or (II) for use as immunoadjuvant.

In particular, the cyclic dinucleotides of the present invention can be used for cytokine induction immunotherapy as vaccine adjuvant therapy.

In this example, a cyclic dinucleotide of Formulae (I) and (II) would be administered to a human or animal subject that has received, is receiving or will receive a vaccination. The benefits provided by the present invention might include enhanced efficacy of the vaccination against the target antigen, reduced toxicity of the vaccination, reduced adverse side effects of the vaccination, or enhanced immune protection of the human or animal subject.

The term "vaccine" refers to a biological preparation administered to a human or animal in order to elicit or enhance a specific immune system response and/or protection against one or more antigens in that human or animal.

The term "vaccination" refers to treatment of a human or animal with a vaccine or to the act of administering a vaccine to a human or animal.

The term "adjuvant" or "immunoadjuvant" refers to a secondary therapeutic substance that is administered together (either sequentially in any order, or concurrently) with a primary therapeutic substance to achieve some kind of complimentary, synergic or otherwise beneficial effect that could not be achieved through use of the primary therapeutic substance alone. An adjuvant can be used together with a vaccine, chemotherapy, or some other therapeutic substance. Adjuvants can enhance the efficacy of the primary therapeutic substance, reduce the toxicity or side effects of the primary therapeutic substance, or provide some kind of protection to the subject that receives the primary therapeutic substance, such as, but not limited to, improved functioning of the immune system.

Another object of the present invention is a cyclic dinucleotide compound of Formula (I) and (II) for use in the treatment of cancer or pre-cancerous syndromes, bacterial infection or infectious diseases, such as viral infection, in particular an AIDS infection or an HIV infection.

As used herein, "cancer" refers to the physiological condition in subjects that is characterized by unregulated or dysregulated cell growth or death. The term "cancer" includes solid tumors and blood-born tumors, whether malignant or benign.

In one embodiment, the cancer is acinar adenocarcinoma, acinar carcinoma, acral-lentiginous melanoma, actinic keratosis, adenocarcinoma, adenocystic carcinoma, adenosquamous carcinoma, adnexal carcinoma, adrenal rest tumor, adrenocortical carcinoma, aldosterone secreting carcinoma, alveolar soft part sarcoma, amelanotic melanoma, ameloblastic thyroid carcinoma, angiosarcoma, apocrine carcinoma, Askin's tumor, astrocytoma, basal cell carcinoma, basaloid carcinoma, basosquamous cell carcinoma, biliary cancer, bone cancer, bone marrow cancer, botryoid sarcoma, brain cancer, breast cancer, bronchioalveolar carcinoma, bronchogenic adenocarcinoma, bronchogenic carcinoma, carcinoma ex pleomorphic adenoma, cervical cancer, chloroma, cholangiocellular carcinoma, chondrosarcoma, choriocarcinoma, choroid plexus carcinoma, clear cell adenocarcinoma, colon cancer, colorectal cancer, comedocarcinoma, cortisol-producing carcinoma, cylindrical cell carcinoma, dedifferentiated liposarcoma, ductal adenocarcinoma of the prostate, ductal carcinoma, ductal carcinoma in situ, duodenal cancer, eccrine carcinoma, embryonal carcinoma, endometrial carcinoma, endometrial stromal carcinoma, epithelioid sarcoma, esophageal cancer, Ewing's sarcoma, exophytic carcinoma, fibroblastic sarcoma, fibrocarcinoma, fibrolamellar carcinoma, fibrosarcoma, follicular thyroid carcinoma, gallbladder cancer, gastric adenocarcinoma, giant cell carcinoma, giant cell sarcoma, giant cell tumor of bone, glioma, glioblastoma multiforme, granulose cell carcinoma, head & neck cancer, hemangioma, hemangiosarcoma, hepatoblastoma, hepatocellular carcinoma, Hürthle cell carcinoma, ileal cancer, infiltrating lobular carcinoma, inflammatory carcinoma of the breast, intraductal carcinoma, intraepidermal carcinoma, jejuna cancer, Kaposi's sarcoma, Krukenberg's tumor, Kulchitsky cell carcinoma, Kupffer cell sarcoma, large cell carcinoma, larynx cancer, lentigo maligna melanoma, liposarcoma, liver cancer, lobular carcinoma, lobular carcinoma in situ, lung cancer, lymphoepithelioma, lymphoepithelioma, lymphosarcoma, malignant melanoma, medullary carcinoma, medullary thyroid carcinoma, medulloblastoma, meningeal carcinoma, Merkel cell carcinoma, micropapillary carcinoma, mixed cell sarcoma, mucinous carcinoma, mucoepidermoid carcinoma, mucosal melanoma, myxoid liposarcoma, myxosarcoma, nasopharyngeal carcinoma, nephroblastoma, neuroblastoma, nodular melanoma, non-clear cell renal cancer, non-small cell lung cancer, oat cell carcinoma, ocular melanoma, oral cancer, osteoid carcinoma, osteosarcoma, ovarian cancer, Paget's carcinoma, pancreatic cancer, pancreatoblastoma, papillary adenocarcinoma, papillary carcinoma, papillary thyroid carcinoma, pelvic cancer, periampullary carcinoma, phyllodes tumor, pituitary cancer, pleomorphic liposarcoma, pleuropulmonary blastoma, primary intraosseous carcinoma, prostate cancer, rectal cancer, renal cell carcinoma, retinoblastoma, rhabdomyosarcoma, round cell liposarcoma, scar cancer, schistosomal bladder cancer, schneiderian carcinoma, sebaceous carcinoma, signet-ring cell carcinoma, skin cancer, small cell lung cancer, small cell osteosarcoma, soft tissue sarcoma, splindle cell carcinoma, spindle cell sarcoma, squamous cell carcinoma, stomach cancer, superficial spreading melanoma, synovial sarcoma, telangiectatic sarcoma, terminal duct carcinoma, testicular cancer, thyroid cancer, transitional cell carcinoma, tubular carcinoma, tumorigenic melanoma, undifferentiated carcinoma, urachal adenocarcinoma, urinary bladder cancer, uterine cancer, uterine corpus carcinoma, uveal melanoma, vaginal cancer, cerrucous carcinoma, villous carcinoma, well-differentiated liposarcoma, Wilm's tumor or yolk sac tumor.

In a particular embodiment, the cancer is from the following group: melanoma, prostate cancer, hepatocarcinoma, colon cancer, bladder cancer, breast cancer, cholangiocellular cancer, leukemia, lung cancer, lymphoma, nasopharyngeal cancer, ovarian cancer, pancreatic cancer and urothelial cancer.

In a particular embodiment, the cancer is from the following group: melanoma, lymphoma and pancreatic cancer.

The present invention thus discloses a method for treating a cancer or pre-cancerous syndromes, a bacterial infection or infectious diseases, such as viral infection, in particular an AIDS infection or an HIV infection, said method comprising administering to a patient in need thereof a cyclic dinucleotide of Formulae (I) or (II).

Another object of the invention is a therapeutic combination comprising a cyclic dinucleotide compound of Formulae (I) or (II) and a therapeutic agent.

Another object of the invention is a kit-of-parts comprising a cyclic dinucleotide compound of Formulae (I) or (II) and a chemotherapeutic agent, for use in the treatment of cancer.

By "kit-of-parts", it is meant a combined preparation wherein the active ingredients are combined together or physically separated for use in a combined therapy by simultaneous administration or sequential administration to the patient.

The cyclic dinucleotides of the present invention can be used for cytokine induction immunotherapy in combination with chemotherapy.

In this example, a cyclic dinucleotide of Formulae (I) or (II) would be administered together with one or more chemotherapeutic agents, sequentially in any order or concomitantly, to a cancer patient to stop the growth of, shrink and/or destroy tumors in that patient. The chemoimmunotherapy resulting from the combination of cytokine induction, provided by the compound(s) of the present invention, and cytotoxicity, provided by the chemotherapeutic agent(s), might be less toxic to the patient, cause fewer side effects in the patient and/or exhibit greater anti-tumor efficacy than would the chemotherapeutic agent(s) when used as monotherapy.

The term "chemotherapeutic agent" herein refers to one or more chemical substances that are administered to a human or animal in order to kill tumors, or slow or stop the growth of tumors, and/or slow or stop the division of cancerous cells and/or prevent or slow metastasis. Chemotherapeutic agents are often administered to treat cancer, but are also indicated for other diseases.

The term "chemotherapy" refers to medical treatment of a human or animal with one or more chemotherapeutic agents (see definition above).

The term "chemoimmunotherapy" refers to the combined use, whether sequentially in any order or concurrently, of chemotherapy substances and/or strategies, and immunotherapy substances and/or strategies. Chemoimmunotherapy is often employed to treat cancer, but can also be employed to treat other diseases.

The present invention thus discloses a method for treating cancer, said method comprising administering to a patient in need thereof:
- a cyclic dinucleotide of Formulae (I) or (II) and
- a chemotherapeutic agent.

When both compounds are administered simultaneously, the kit-of-parts can contain both compounds in a single pharmaceutical composition, such as a tablet, or in separate pharmaceutical compositions. When the compounds are not administered simultaneously, the kit-of-parts will contain each compound in separate pharmaceutical compositions either in a single package or in separate pharmaceutical compositions in separate packages. The kit-of-parts can also be provided by instruction, such as dosage and administration instructions. Such dosage and administration instructions can be of the kind that are provided to a doctor, for example by a drug product label, or they can be of the kind that are provided by a doctor, for example by a drug product label, or they can be of the kind that are provided by a doctor, such as instructions to a patient.

When the combination is administered separately in a sequential manner wherein one is administered first and the other second or vice versa, such sequential administration may be close in time or remote in time. For example, administration of the other agent several minutes to several dozen minutes after the administration of the first agent, and administration of the other agent several hours to several days after the administration of the first agent are included, wherein the lapse of time is not limited, For example, one agent may be administered once a day, and the other agent may be administered 2 or 3 times a day, or one agent may be administered once a week, and the other agent may be administered once a day and the like.

In this example, a cyclic dinucleotide of Formulae (I) and (II) would be administered to an immunosuppressed human or animal subject to induce *in vivo* production of one or more cytokines that directly or indirectly enhance the immune system of that human or animal. Subjects that might benefit from such treatment include those suffering from autoimmune disorders, immune system deficiencies or defects, microbial or viral infections, infectious diseases, or cancer.

### Fluoro-substituted nucleosides for the synthesis of CDNs

Since STING is located in the endoplasmic reticulum and detects cyclic dinucleotides in the cytoplasm, any STING agonist destined for therapeutic use must be able to penetrate into cells. Furthermore, greater cellular uptake of a compound translates to higher bioavailability, which is a desirable property for clinical use.

The fluorinated CDNs of the present invention exhibit greater STING-dependent cytokine induction activity in human cells, animal cells and human blood than do their corresponding non-fluorinated analogs. This greater activity is presumably due to a greater cellular uptake conferred by the one or two fluorine atoms.

### Monofluoro-nucleoside

The 2'-fluoro-2'-deoxy-β,D-ribofuranoyl nucleosides for CDNs of Formulas (III) to (X) refers to a nucleoside with a modification at the 2' position, such that, the hydroxyl group (2'-OH) of adenosine, guanosine or inosine is replaced by a fluorine atom (2'-F) without inversion of configuration at this position.

The 3'-fluoro-3'-deoxy-β,D-xylofuranoyl nucleosides derivatives for CDNs of Formulas (V) to (X) refers to a nucleoside with a modification at the 3' position, such that, the hydroxyl group (3'-OH) of Adenosine or Inosine is replaced by a fluor atom (3'-F) with inversion of configuration at this position.

The 2'-fluoronucleoside derivatives can be prepared by any of the methods known in the art (see, for example: (Herdewijn, 1989; Thomas, 1994) and (Ross, 1997)).

The 3'-fluoronucleoside derivatives can be prepared by any of the methods known in the art (see for example: Aldrich, 2015).

### General Schemes for Preparing Active Compounds

Methods for the easy preparation of cyclic dinucleotides or prodrugs thereof disclosed herein can be prepared as described in detail below, or by other methods known to those skilled in the art. It will be understood by one of ordinary skill in the art that these schemes are in no way limiting and that variations of detail can be made without departing from the spirit and scope of the present invention.

The term "protecting group" as used herein, and unless otherwise defined, refers to a chemical functional group that is attached to an oxygen, nitrogen or phosphorus atom to prevent further reaction of that atom, or for other purposes. A wide variety of protecting groups for oxygen and for nitrogen are known to those skilled in the art of organic synthesis, and are described, for example, in (Wuts, Greene, & Greene, 2014).

Generally, cyclic dinucleotides or prodrugs thereof of the Formulae (III) or (IV) are prepared by first preparing the corresponding nucleoside. After protecting the 5'-hydroxy group, and, if necessary, protecting the heterocyclic base, the appropriately protected nucleosides are converted to the corresponding 3'-phosphoramidite or 2'-phosphoramidite or 3'-H-phosphonate or 2'-H-phosphonate, which constitute the starting material for the preparation of the cyclic dinucleotides described herein.

The reaction schemes shown below apply to the synthesis of CDNs of Formula (I) and can be employed for the synthesis of CDNs of Formula (II):

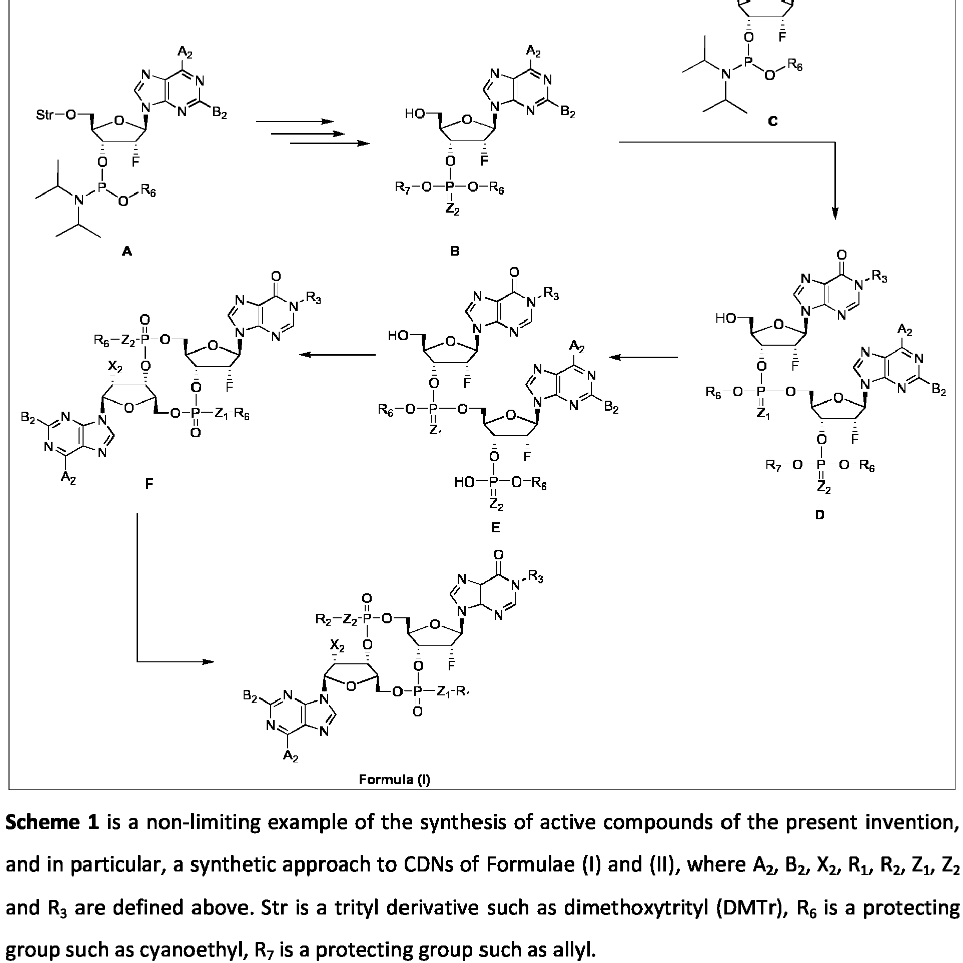

Phosphoramidites of the Formula (A) is converted to phosphotriesters or phosphorothioate triesters of Formula (B) in three steps. Then, dimer of Formula (D) is synthesized by coupling compound of Formula (B) and phosphoramidite of Formula (C). The protection R₆ is cleaved to obtain compound and the dimer of Formula (E) obtained is cyclized in one step to provide compound of Formula (F) which is deprotected to obtain the aim CDN of Formula (I). Eventually, the phosphorothioate diester of Formula (I), Z₁ and/or Z₂ is S and R₁ and/or R₂ are H, can be converted to phosphorothioate triester of Formula (I)..

The reaction schemes shown below apply to the synthesis of CDNs of Formula (II) and can be employed for the synthesis of CDNs of Formula (I):

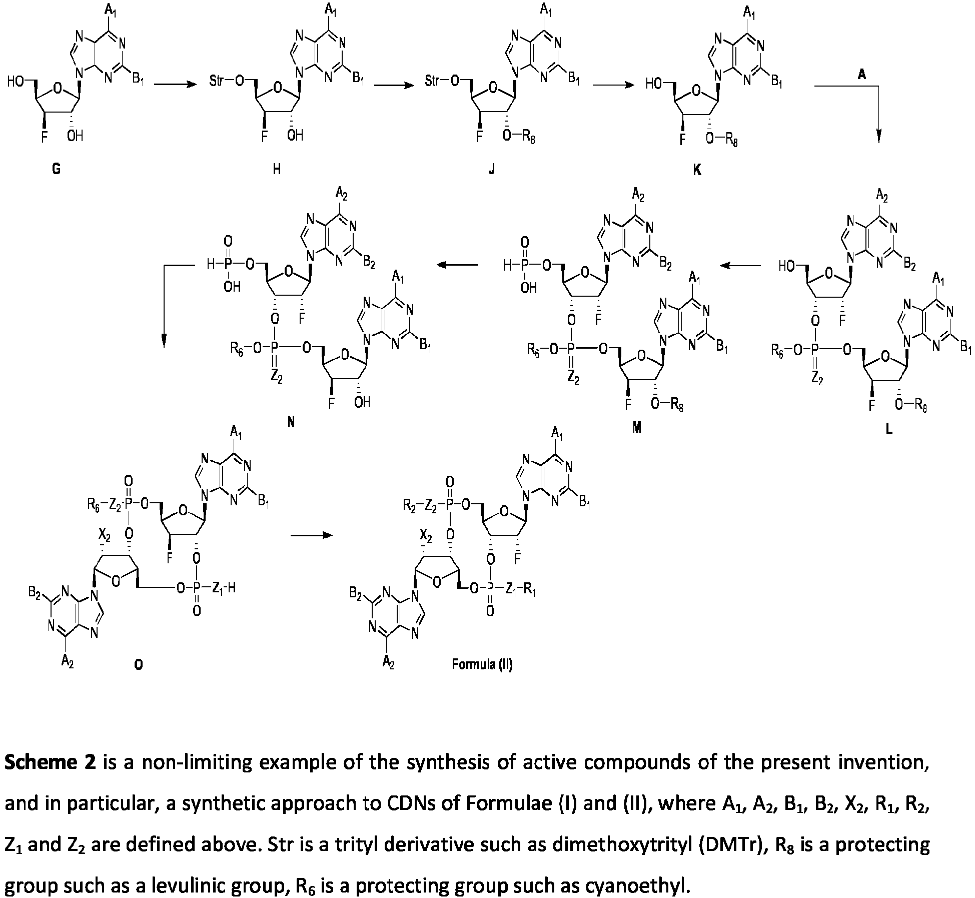

The Inosine analogs, nucleosides of the Formula (G), can be synthesized by one of ordinary skill in the art. Then, the 2', 5'-hydroxyl groups are selectively protected to obtain the fully protected nucleoside of Formula (J). The protecting group at 5'-hydroxyl is cleaved to obtain compound of Formula (K) which is coupled with a phosphoramidite of Formula (A) to provide dimer of Formula (L) in three steps. This dimer is converted to a 5'-H-phosphonate of Formula (M) and the 2'-hydroxyl position is deprotected to provide compound of Formula (N), which is cyclized to obtain compound of Formula (O). This compound is fully deprotected to provide CDN of Formula (II). Eventually, the phosphorothioate diester can be converted to phosphorothioate triester.

### Brief description of the figures

Figure 1 represents Type I IFN induction (ISG54 activation) in THP-1-Dual cells treated with the c-dilMP analogs of the present invention (tested at 3 µg/mL) relative to unstimulated cells.
Figure 2 represents a comparison of ISG54 activity induced by some CDN of the present invention between THP1 WT and THP1 KO STING cells.
Figure 3 is (Top) a histogram representing the fold induction of type I IFN (black) or type III interferon (white) for each compound relative to the reference compound (dash line), 2'3'-cGAMP (tested at the same concentration; ≈ 4 µM) in the whole-blood assay. The data represent the mean fold ± sem from five healthy donors. Bottom: EC50 (µM) values for Type I and Type III interferon induction for each compound in the whole-blood assay.
Figure 4 represents ISG54 activity induced by CDNs described in the present invention on a murine cell line.
Figure 5 represents images of RAW-Difluo™ mLC3 cells treated with the c-dilMP analogs of the present invention (1 µg/mL). Induction of autophagy is indicated by the presence of puncta.
Figure 6 represents PD-L1 expression on THP-1 WT cells that were either untreated (0 µg/mL), or treated with either the c-diIMP analogs of the present invention, or the reference compound 2'3'-cGAMP, at four different concentrations (1 µg/mL, 3 µg/mL, 10 µg/mL or 30 µg/mL).
Figure 7 represents a comparison of ISG54 activity induced (18h) by CDN prodrug analog (solid black) of the present invention between in comparison with non-prodrug molecule (empty black) in THP1 WT cells.
Figure 8 represents the individual tumor volumes for all mice at the end of the experiment.
Figure 9 represents the corresponding tumor weights;
Figure 10 shows the incidence of metastasis in a murine model of Panc02 tumors.
Figure 11 represents in part A-C individual B16-tumor volumes during the course of the experiment for individual mice treated with saline (A), CL742 (B) or CL747 (C) and the mean, sd and p value statistical difference between group are presented for tumor volume measured at day 20 (D).

### EXAMPLES

Specific compounds that are representative of this invention were prepared as per the following examples and are offered by way of illustration to aid in the understanding of the invention. They should not be construed to limit in any way the invention set forth in the claims that follow thereafter. The present compounds can also be used as intermediates in subsequent examples to produce additional compounds of the present invention. No attempt has necessarily been made to optimize the yields obtained in any of the reactions. One skilled in the art would know how to increase such yields through routine variations in reaction times, temperatures, solvents, reagents and chemical synthesis other parameters.

The present invention is further illustrated in Example 1, which shows preparative methods for synthesizing CDNs, and in Examples 2 to 7, which show methods for the biological evaluation of these CDNs. It will be understood by one of ordinary skill in the art that these examples are in no way limiting and that variations of detail can be made without departing from the spirit and scope of the present invention.

The terms used in describing the invention are commonly used and known to those skilled in the art. As used herein, the following abbreviations have the indicated meanings:
°C for degrees Celsius; A for adenosine; ACN for acetonitrile; aq. for aqueous; CDCl₃ for deuterated chloroform; C₁₈ for octadecyl carbon chain bonded silica; d for doublet; dA for deoxyadenosine; dd for doublet of doublets; dI for deoxyinosine; D₂O for deuterium oxide; DCA for dichloroacetic acid; DCM for dichloromethane; DMSO-*d₆* for deuterated dimethylsulfoxide; DMTrCl for 4;4'-dimethoxytrityl chloride; equiv. for equivalent; ES for electrospray ionization; Et₂O for diethyl ether; EtOAc for ethyl acetate; EtOH for ethanol; Et₃N·3HF for triethylamine trihydrofluoride; g for grams; G for guanosine; ¹H for proton; h for hours; Hz for Hertz; HPLC for high-performance liquid chromatography; I for inosine; IFN for interferon; IFN-α for interferon alpha; IFN-β for interferon beta; *i*PrOH for isopropanol; IRF3 for interferon regulatory factor 3; ISG (or ISG54) for interferon-stimulated gene; ISRE for interferon-stimulated response element; i.p. for intraperitoneal; i.t. for intratumoral; i.v. for intravenous; LC for liquid chromatography; m for multiplet; Luc for luciferase; M for molar; m/z for mass-to-charge ratio; MeOH for methanol; mg for milligrams; MgSO₄ for magnesium sulfate; MHz for megahertz; min for Minutes; mL (or ml) for milliliters; mmol for millimoles; mol/L for mole/liter; MS for mass spectrometry; NaHCO₃ for sodium bicarbonate; NaHSO₃ for sodium thiosulfate; NH₄OH for ammonium hydroxide; NF-kB for nuclear factor kappa-light-chain-enhancer of activated B cells; NMR for nuclear magnetic resonance; PADS for phenylacetyl disulfide; ppm for parts per million; PPTS for pyridinium p-toluenesulfonate; rt for room temperature; SEAP for secreted embryonic alkaline phosphatase; s for singlet; sl for large singlet; t for triplet; SKO for homozygous STING knockout; STING for stimulator of interferon genes; THF for tetrahydrofuran; TEAA for triethylammonium acetate; TFA for trifluoroacetic acid; WT for wild type; µg for microgram; µL (or µl) for microliter; µm for micrometer; δ for chemical shift.

Anhydrous solvents and reagents suitable for nucleoside and nucleotide synthesis were purchased and were handled under dry argon or nitrogen using anhydrous technique. Amidite coupling reactions and cyclizations were performed in anhydrous acetonitrile or pyridine under dry argon or nitrogen. The starting materials for all reactions in dry pyridine were dried by concentration (three times) from pyridine. Preparative silica-gel flash chromatography was performed using Fluka 60 Å high-purity grade or Merck Grade 9385 silica using gradients of methanol in dichloromethane. Analytical LC/ES MS was performed on an Agilent 1290 Infinity UHPLC system coupled to a diode array detector (DAD) Agilent 1260 Infinity and an Agilent 6130 Quadrupole mass spectrometer equipped with an electrospray ionization source (ESI) and controlled by Chemstation software. The LC system was equipped with an Aquity CSH C18 50×2.1 mm 1.7 µm column using gradients of 10 mM ammonioum formate and acetonitrile at 300 µl/min flow. The UV detection wavelength was 254 nm. The mass spectrometer was operated in positive and negative ESI modes Preparative HPLC was performed on a Waters preparative 150Q HPLC system monitoring at 254 nm on a SunFire Prep C18 5 µm OBD 30 x 150mm column using gradients of 10 mM ammonium formate and acetonitrile at a flow rate 60 mL/min. The ¹H NMR spectra were acquired on either a Bruker 300 MHz (Fourrier 300) at room temperature and reported in ppm downfield. Molecular sieves (MS) 3Å were employed after drying the commercially supplied product at 250 for 12 h under vacuum. The commercial nucleoside phosphoramidites were supplied from Chemgenes.

The following examples serve to illustrate the present invention. These examples are in no way intended to limit the scope of the invention.

### EXAMPLE 1: Synthesis of the compounds of the invention

### CL735

### Intermediate 1: 5'-O-DMTr-2'-fluoro-2'-deoxy-Inosine

The commercially available 2'-deoxy-2'-fluoro-Inosine (5.0 g, 18.50 mmol) was co-evaporated three times with dry pyridine. The residue was suspended in dry pyridine, and to the resulting solution was added DMAP (2.26 g, 18.50 mmol) and DimethoxyTritryl chloride (DMTrCl; 6.89 g, 20.35 mmol). The resulting mixture was stirred at 70 overnight. Then, the reaction was stopped by addition of methanol, the reaction mixture was diluted with EtOAc and was washed with saturated NaHCO₃, water and brine. The organic layer was dried over MgSO₄, filtered and concentrated *in vacuo,* to provide 6.07 g (57% yield) of Intermediate 1, which was used in the next step without any further purification. LC-MS: Rt = 5.22 min, m/z = 573 [M+H]⁺, m/z = 571 [M-H]⁻.

### Intermediate 2: 5'-O-DMTr-3'-O-Lev-2'-fluoro-2'-deoxy-Inosine

To a solution of **Intermediate 1** (1.0 g, 1.75 mmol) in dry DMF (10 mL) was added EDCI (0.36 g, 1.92 mmol), DMAP (0.21 g, 1.75 mmol) and levulinic acid (0.21 mL, 2.09 mmol). The resulting mixture was stirred at rt overnight. Then, the reaction mixture was diluted with EtOAc and was washed with saturated NaHCO₃, water and brine. The organic layer was dried over MgSO₄, filtered and concentrated *in vacuo.* The crude compound was purified by silica-gel column chromatography, using DCM/MeOH as eluent to provide 1.06 g (90% yield) of **Intermediate 5.** LC-MS: Rt = 5.65 min, m/z = 671 [M+H]⁺, m/z = 669 [M-H]⁻.

### Intermediate 3: N¹-POM-5'-O-DMTr-3'-O-Lev-2'-fluoro-2'-deoxy-Inosine

To a solution of **Intermediate 2** (1.0 g, 1.58 mmol) in dry DMF (10 mL) was added K₂CO₃ (0.65 g, 4.74 mmol), and POM-Cl (0.27 g, 1.89 mmol). The resulting mixture was stirred at rt for 3h00. Then, the reaction mixture was diluted with EtOAc and was washed with saturated NaHCO₃, water and brine. The organic layer was dried over MgSO₄, filtered and concentrated *in vacuo* to provide 1.24 g (99% yield) of **Intermediate 3,** which was used in the next step without any further purification. LC-MS: Rt = 5.85 min, m/z = 785 [M+H]⁺, m/z = 783 [M-H]⁻.

### Intermediate 4: N¹-POM-3'-O-Lev-2'-fluoro-2'-deoxy-Inosine

The **Intermediate 3** (1.24 g, 1.58 mmol) was treated with a solution of DCA/DCM (3%) in the presence of water (10 equiv.) for 15 min. The reaction was quenched by addition of MeOH and pyridine. The solvents were removed *in vacuo* and the residue was purified by silica-gel column chromatography, using DCM/MeOH as eluent to provide 670 mg (88% yield) of **Intermediate 4,** which was used in the next step without any further purification. LC-MS: Rt = 4.37 min, m/z = 483 [M+H]⁺, m/z = 481 [M-H]⁻.

### Intermediate 5: [3'-O-(CE)phosphorothioate-triester-2'-fluoro-2'-deoxy-Inosine]-(3',5')-[N¹-POM-3'-O-Lev-2'-fluoro-2'-deoxy-Inosine]

**Intermediate 4** (1 g, 2.07 mmol) and commercially available phosphoramidite of 2'-fluoro-2'-deoxyInosine (1.60 g, 2.07 mmol) was co-evaporated three times with dry ACN, and the resulting solid was dissolved in a solution of Activator42® (0.1 mol/L, 2 equiv.; 41 mL) in the presence of molecular sieves (3Å). The resulting mixture was stirred for 45 min. Then, a solution of PADS (1.56 g, 2.50 mmol) in pyridine was added to the mixture, which was stirred for 35 min. The solution was filtered and the molecular sieves were washed with DCM. The filtrate was concentrated *in vacuo.* The residue was treated with a solution of DCA/DCM (3%) in the presence of water (10 equiv.) for 15 min. The reaction was quenched with addition of MeOH and pyridine. The solvents were removed *in vacuo* and the residue was purified by silica-gel column chromatography, using DCM/MeOH as eluent to provide 1.26 g (69% yield) of **Intermediate 5.** LC-MS: Rt = 4.64 min, m/z = 884 [M+H]⁺, m/z = 882 [M-H]⁻.

### Intermediate 6: [3'-O-(CE)phosphorothioate-triester-2'-fluoro-2'-deoxy-inosine]-(3',5')-[N¹-POM 3'-O-Lev-2'-fluoro-2'-deoxy-Inosine]

**Intermediate 5** (1.26 g, 1.43 mmol) was treated with a solution of 0.5 M hydrazine (14.25 mL, 7.12 mmol) in a mixture of pyridine/acetic acid (3:2) for 15 min. The reaction was quenched by addition of pentadione (1.45 mL, 14.25 mmol). The solvents were removed *in vacuo* and the residue was triturated with diethyl ether to provide 1.12 mg (99% yield) of **Intermediate 6,** which was used in the next step without any further purification. LC-MS: Rt = 4.22 min, m/z = 786 [M+H]⁺, m/z = 784 [M-H]⁻.

### Intermediate 7: Cyclic-(3',3')-[N¹-POM-3'-O-(CE)phosphorothioate-triester-2'-fluoro-2'-deoxyInosine]-[3'-O-(CE)phosphorothioate-triester-2'-fluoro-2'-deoxy-Inosine]

**Intermediate 6** (1.12 g, 1.42 mmol) was co-evaporated three times with dry ACN, and the resulting solid was dissolved in a solution of Activator42® (0.1 mol/L, 4 equiv.; 57.02 mL) in the presence of molecular sieves (3Å). Then 2-Cyanoethyl *N,N,N',N'*-tetraisopropylphosphoramidite (0.51 g, 1.71 mmol) was added. The resulting mixture was stirred for 90 min. Then, a solution of PADS (1.08 g, 3.56 mmol) in pyridine was added to the mixture, which was stirred for 35 min. The solution was filtered and the molecular sieves were washed with DCM. The filtrate was concentrated *in vacuo* and the residue was purified by silica-gel column chromatography, using DCM/MeOH as eluent to provide 1.16 g (88% yield) of **Intermediate 7.** LC-MS: Rt = 5.25 min, m/z = 917 [M+H]⁺, m/z = 915 [M-H]⁻.

### CL733

### Intermediate 8: [3'-O-(CE)phosphotriester-2'-fluoro-2'-deoxy-Inosine]-(3',5')-[N¹-POM-3'-O-Lev-2'-fluoro-2'-deoxy-Inosine]

**Intermediate 7** (0.67 g, 1.38 mmol) and commercially available phosphoramidite of 2'-fluoro-2'-deoxylnosine (1.07 g, 1.39 mmol) was co-evaporated three times with dry ACN, and the resulting solid was dissolved in a solution of Activator42® (0.1 mol/L, 2 equiv.; 28 mL) in the presence of molecular sieves (3Å). The resulting mixture was stirred for 45 min. Then, tert-butyl hydroperoxide in decane (3.08 mL, 2.77 mmol) was added to the mixture, which was stirred for 30 min. The solution was filtered and the molecular sieves were washed with DCM. The filtrate was concentrated *in vacuo.* The residue was treated with a solution of DCA/DCM (3%) in the presence of water (10 equiv.) for 15 min. The reaction was quenched with addition of MeOH and pyridine. The solvents were removed *in vacuo* and the residue was purified by silica-gel column chromatography, using DCM/MeOH as eluent to provide 445 mg (66% yield) of **Intermediate 8.** LC-MS: Rt = 4.29 min, m/z = 868 [M+H]⁺, m/z = 866 [M-H]⁻.

### Intermediate 9: [3'-O-(CE)phosphotriester-2'-fluoro-2'-deoxy-Inosine]-(3',5')-[N¹-POM-3'-O-Lev-2'-fluoro-2'-deoxy-Inosine]

**Intermediate 8** (0.445 g, 0.51 mmol) was treated with a solution of 0.5 M hydrazine (5.12 mL, 2.56 mmol) in a mixture of pyridine/acetic acid (3:2) for 15 min. The reaction was quenched by addition of pentadione (0.52 mL, 5.12 mmol). The solvents were removed *in vacuo* and the residue was triturated with diethyl ether to provide 315 mg (80% yield) of **Intermediate 9,** which was used in the next step without any further purification. LC-MS: Rt = 3.85 min, m/z = 770 [M+H]⁺, m/z = 768 [M-H]⁻.

### Intermediate 10: Cyclic-(3',3')-[N¹-POM-3'-O-(CE)phosphotriester-2'-fluoro-2'-deoxy-Inosine]-[3'-O-(CE)phosphotriester-2'-fluoro-2'-deoxy-Inosine]

**Intermediate 9** (0.15 g, 0.20 mmol) was co-evaporated three times with dry ACN, and the resulting solid was dissolved in a solution of Activator42® (0.1 mol/L, 4 equiv.; 8.0 mL) in the presence of molecular sieves (3Å). Then 2-Cyanoethyl *N,N,N',N'*-tetraisopropylphosphoramidite (70 mg, 0.24 mmol) was added. The resulting mixture was stirred for 90 min. Then, tert-butyl hydroperoxide in decane (3.08 mL, 2.77 mmol) was added to the mixture, which was stirred for 30 min. The solution was filtered and the molecular sieves were washed with DCM. The filtrate was concentrated *in vacuo* and the residue was purified by silica-gel column chromatography, using DCM/MeOH as eluent to provide 1.16 g (88% yield) of **Intermediate 10.** LC-MS: Rt = 3.46 min, m/z = 829 [M+H]⁺, m/z = 827 [M-H]⁻.

### CL734

### Intermediate 11: Cyclic-(3',3')-[N¹-POM-3'-O-(CE)phosphorothioate-triester-2'-fluoro-2'-deoxyInosine]-[3'-O-(CE)phosphotriester-2'-fluoro-2'-deoxy-Inosine]

**Intermediate 9** (0.16 g, 0.21 mmol) was co-evaporated three times with dry ACN, and the resulting solid was dissolved in a solution of Activator42® (0.1 mol/L, 4 equiv.; 8 mL) in the presence of molecular sieves (3Å). Then 2-Cyanoethyl *N,N,N',N'*-tetraisopropylphosphoramidite (75 mg, 0.25 mmol) was added. The resulting mixture was stirred for 90 min. Then, a solution of PADS (0.16 g, 0.52 mmol) in pyridine was added to the mixture, which was stirred for 35 min. The solution was filtered and the molecular sieves were washed with DCM. The filtrate was concentrated *in vacuo* and the residue was purified by silica-gel column chromatography, using DCM/MeOH as eluent to provide 160 mg (84% yield) of **Intermediate 11.** LC-MS: Rt = 4.73 min, m/z = 901 [M+H]⁺, m/z = 899 [M-H]⁻.

### CL742

### Intermediate 12: 5'-O-DMTr-3'-fluoro-3'-deoxy-xylo-Inosine

**Intermediate 12** (1.06 g, quantitative yield) was prepared from 3'-fluoro-3'-deoxy-xylo-Inosine (500 mg, 1.85 mmol) as starting material using a similar procedure to that described for **Intermediate 1.** LC-MS: Rt = 5.40 min, m/z = 573 [M+H]⁺, m/z = 571 [M-H]⁻.

### Intermediate 13: 5'-O-DMTr-2'-O-Lev-3'-fluoro-3'-deoxy-xylo-Inosine

**Intermediate 13** (870 mg, 70% two steps yield) was prepared from **Intermediate 12** (1.06 g, 1.85 mmol) as starting material using a similar procedure to that described for **Intermediate 2.** LC-MS: Rt = 5.68 min, m/z = 671 [M+H]⁺, m/z = 669 [M-H]⁻.

### Intermediate 14: N¹-POM-5'-O-DMTr-2'-O-Lev-3'-fluoro-3'-deoxy-xylo-Inosine

**Intermediate 14** (4.95 g, 41% yield) was prepared from **Intermediate 13** (10.4 g, 15.5 mmol) as starting material using a similar procedure to that described for **Intermediate 3.** LC-MS: Rt = 7.01 min, m/z = 785 [M+H]⁺, m/z = 783 [M-H]⁻.

### Intermediate 15: N¹-POM-2'-O-Lev-3'-fluoro-3'-deoxy-xylo-Inosine

**Intermediate 15** (2.53 g, 83% yield) was prepared from **Intermediate 14** (4.95 g, 6.3 mmol) as starting material using a similar procedure to that described for **Intermediate 4.** LC-MS: Rt = 4.527 min, m/z = 483 [M+H]⁺, m/z = 481 [M-H]⁻.

### Intermediate 16: [3'-O-(CE)phosphotriester-2'-fluoro-2'-deoxy-Inosine]-(3',5')-[N¹-POM-2'-O-Lev-3'-fluoro-3'-deoxy-xylo-Inosine]

**Intermediate 16** (820 mg, 76% yield) was prepared from **Intermediate 15** (600 mg, 1.24 mmol) as starting material and the commercially available phosphoramidite of 2'-fluoro-2'-deoxy-Inosine (1.0 g, 1.3 mmol) using a similar procedure to that described for **Intermediate 8.** LC-MS: Rt = 4.55 min, m/z = 868 [M+H]⁺, m/z = 866 [M-H]⁻.

### Intermediate 17: [5'-O-H-phosphonate-3'-O-(CE)phosphotriester-2'-fluoro-2'-deoxy-Inosine]-(3',5')-[N¹-POM-2'-O-Lev-3'-fluoro-3'-deoxy-xylo-Inosine]

Diphenyl phosphite (0.36 mL, 1.9 mmol) was added to a solution of Intermediate 16 (820 mg, 0.95 mmol) in dry pyridine (5 mL). The resulting mixture was stirred at room temperature for 2 hours, quenched by an addition of triethylammonium acetate (3.78 mL, 4 equiv.), and then concentrated under reduced pressure. The residue was purified by silica-gel column chromatography, using DCM/MeOH as eluent to provide 830 mg (94% yield) of **Intermediate 17.** LC-MS: Rt = 4.81 min, m/z = 932 [M+H]⁺, m/z = 930 [M-H]⁻.

### Intermediate 18: [5'-O-H-phosphonate-3'-O-(CE)phosphotriester-2'-fluoro-2'-deoxy-Inosine]-(3',5')-[N¹-POM-3'-fluoro-3'-deoxy-xylo-Inosine]

**Intermediate 18** (743 mg, 97% yield) was prepared from **Intermediate 17** (830 mg, 0.91 mmol) as starting material using a similar procedure to that described for **Intermediate 9.** LC-MS: Rt = 3.67 min, m/z = 834 [M+H]⁺, m/z = 832 [M-H].

### Intermediate 19: Cyclic-(2',3')-[N¹-POM-2'-O-phosphorothioate-diester-3'-fluoro-3'-deoxy-xylo-Inosine]-[3'-O-(CE)-phosphotriester-2'-fluoro-2'-deoxy-Inosine]

**Intermediate 18** (380 mg, 0.45 mmol) was co-evaporated three times with dry pyridine, and the resulting solid was dissolved in dry pyridine (59 mL) under inert atmosphere. Pivaloyl chloride (197 µL, 3.5 equiv.) was added and the resulting solution was stirred at r.t. for 40 min. Sulfur (29 mg, 2 equiv.) was added, the resulting solution was let stirred for 1 h at r.t, then was concentrated *in vacuo.* The residue was purified by silica-gel column chromatography, using DCM/MeOH as eluent to provide **Intermediate 19** (386 mg, quantitative yield). LC-MS: Rt = 4.56, 4.71, 5.09 min, m/z = 848 [M+H]⁺.

### CL747

### Intermediate 20: Cyclic-(2',3')-[N¹-POM-2'-O-phosphorothioate-diester-3'-fluoro-3'-deoxy-xylo-Inosine]-[3'-O-phosphodiester-N¹-POM-2'-fluoro-2'-deoxy-Inosine]

**Intermediate 20** (165 mg, 76% two steps yield) was prepared from **Example 1.11** (190 mg, 0.24 mmol) as starting material using a similar procedure to that described for **Intermediate 3.** LC-MS: Rt = 3.38, 3.57, 3.67 min, m/z = 907 [M-H]⁻.

### CL785

### Intermediate 21: N¹-POM-5'-O-DMTr-3'-O-(CE)phosphoramidite-2'-fluoro-2'-deoxy-Inosine

**Intermediate 3** (7.0 g, 8.92 mmol)) was treated with a solution of 0.5 M hydrazine (3.3 mL, 44.6 mmol) in a mixture of pyridine/acetic acid (3 :2) for 15 min. The reaction was quenched by addition of pentadione (9.1 mL, 89 mmol). The solvents were removed in vacuo and the residue was triturated with diethyl ether. The obtained solid was co-evaporated three times with dry acetonitrile. The residue (4.78 g, 6.96 mmol) was dissolved in dry dichloromethane (258 mL), and to the resulting solution was added DIPEA (6.0 mL, 34.8 mmol) follow by a dropwise solution of 2-Cyanoethyl N,N-diisopropylchlorophosphoramidite (1.9 mL, 8.35 mmol) in dry DCM (10 mL). After completion, MeOH was added. The reaction mixture was washed with saturated NaHCO₃ (x3) and brine. The organic layer was dried over MgSO₄, filtered and concentrated in vacuo. The crude compound was purified by silica-gel column chromatography, using DCM/MeOH as eluent to provide 6.00 g (90 % yield) of **Intermediate 21.** LC-MS : Rt = 7.32, 7.42 min, m/z = 887 [M+H]⁺, 885 [M-H]⁻.

### Intermediate 22: [N¹-POM-3'-O-(CE)phosphorothioate-triester-2'-fluoro-2'-deoxy-Inosine]-(3',5')-[N¹-POM-5'-O-DMTr-2'-O-Lev-3'-fluoro-3'-deoxy-xylo-Inosine]

**Intermediate 22** (1.1 g, 53 % yield) was prepared from **Intermediate 14** (1.0 g, 2.1 mmol) and **Intermediate 21** (2.0 g, 2.3 mmol) as starting material using a similar procedure to that describe for **Intermediate 5.** LC-MS : Rt = 5.37 min, m/z = 998 [M+H]⁺, 996 [M-H]⁻.

### Intermediate 23: [N¹-POM-5'-O-H-phosphonate-3'-O-(CE)phosphorothioate-triester-2'-fluoro-2'-deoxy-Inosine]-(3',5')-[N¹-POM-5'-O-DMTr-2'-O-Lev-3'-fluoro-3'-deoxy-xylo-Inosine]

**Intermediate 23 (1.6** g, 65 % yield) was prepared from **Intermediate 22** (2.3 g, 2.3 mmol) as starting material using a similar procedure to that describe for **Intermediate 17.** LC-MS : Rt = 4.45 min, m/z = 1062 [M+H]⁺, 1060 [M-H]⁻.

### Intermediate 24: [N¹-POM-5'-O-H-phosphonate-3'-O-(CE)phosphorothioate-triester-2'-fluoro-2'-deoxy-Inosine]-(3',5')-[N¹-POM-5'-O-DMTr-3'-fluoro-3'-deoxy-xylo-Inosine]

**Intermediate 24** (1.45 g, quant. yield) was prepared from **Intermediate 23** (1.6 g, 1.5 mmol) as starting material using a similar procedure to that describe for **Intermediate 6.** LC-MS : Rt = 4.29 min, m/z = 964 [M+H]⁺, 962 [M-H]⁻.

### Intermediate 25: cyclic-(2'-3')-[N¹-POM-3'-O-(CE)phosphorothioate-triester-2'-fluoro-2'-deoxyInosine]-[N¹-POM-2'-O-phosphorothioate-triester-3'-fluoro-3'-deoxy-xylo-Inosine]

**Intermediate 25** (700 mg, 48% yield) was prepared from **Intermediate 24** (1.45 g, 1.5 mmol) as starting material using a similar procedure to that describe for **Intermediate 19.** LC-MS: Rt = 4.62 min, m/z = 978 [M+H]⁺, 976 [M-H]⁻.

### CL761

### Intermediate 26: 5'-O-DMTr-2-Chloro-2'-deoxy-Adenosine

The 2-chloro-2'-deoxy-Adenosine (1.0 g, 3.50 mmol) was co-evaporated three times with dry pyridine. The residue was suspended in dry pyridine, and to the resulting solution was added DMAP (0.64 g, 5.25 mmol) and DimethoxyTritryl chloride (DMTrCl) (1.78 g, 5.25 mmol). The resulting mixture was stirred at rt for 48h. Then, the reaction was stopped by addition of methanol, the reaction mixture was diluted with EtOAc and was washed with saturated NaHCO₃, water and brine. The organic layer was dried over MgSO₄, filtered and concentrated *in vacuo* and the residue was purified by silica-gel column chromatography, using DCM/Acetone as eluent to provide 1.27 g (63% yield) of **Intermediate 26.** LC-MS: Rt = 5.49 min, m/z = 589 [M+H]⁺, m/z = 587 [M-H]⁻.

### Intermediate 27: 5'-O-DMTr-N⁶-(N,N-dimethylformamide)-2-Chloro-2'-deoxy-Adenosine

**Intermediate 26** (1.26 g, 2.14 mmol) was suspended in dry DMF. To the solution was added DMF-DMA (0.45 mL, 3.21 mmol) and the mixture was stirred at 60°C for 2h. Then, the reaction mixture was diluted with EtOAc and was washed with saturated NaHCO₃, water and brine. The organic layer was dried over MgSO₄, filtered and concentrated *in vacuo* and the residue was purified by silica-gel column chromatography, using DCM/Acetone as eluent to provide 1.30 g (94% yield) of **Intermediate 27.** LC-MS: Rt = 5.63 min, m/z = 644 [M+H]⁺, m/z = 642 [M-H]⁻.

### Intermediate 28: 5'-O-DMTr-3'-O-(2-CE-diisopropylphosphoramidite)-N⁶-(N,N-dimethylformamide)-2-Chloro-2'-deoxy-Adenosine

The **Intermediate 27** (1.30 g, 2.01 mmol) was suspended in dry ACN (20 mL) in presence of molecular sieves (3A). To the solution was added DIEA (2.82 mL, 16.17 mmol) and 2-cyanoethyl *N,N-*diisopropylchlorophosphoramidite (0.49 mL, 2.22mmol) and the mixture was stirred at rt for 1h. Then, the reaction mixture was diluted with EtOAc and was washed with saturated NaHCO₃, water and brine. The organic layer was dried over MgSO₄, filtered and concentrated *in vacuo* and the residue was purified by silica-gel column chromatography, using DCM/Acetone as eluent to provide 1.30 g (94% yield) of **Intermediate 28.** LC-MS: Rt = 7.11 min, m/z = 844 [M+H]⁺, m/z = 842 [M-H]⁻.

### Intermediate 29: [3'-O-(CE)phosphotriester-N⁶-(N,N-dimethylformamide)-2-Chloro-2'-deoxyAdenosine]-(3',5')-[N¹-POM-3'-O-Levulinic-2'-fluoro-2'-deoxy-Inosine]

**Intermediate 28** (1.07 g, 1.27 mmol) was reacted with the **Intermediate 4** (0.60 g, 1.24 mmol) using a similar procedure to that described for preparation of **Intermediate 9,** to give 1.10 g (94%) of **Intermediate 29.** LC-MS: Rt = 4.50 min, m/z = 939 [M+H]⁺, m/z = 937 [M-H]⁻.

### Intermediate 30: [N⁶-(N,N-dimethylformamide)-3'-O-(CE)phosphotriester-2-Chloro-2'-deoxyAdenosine]-(3',5')-[N¹-POM-2'-fluoro-2'-deoxy-Inosine]

**Intermediate 29** (1.10 g, 1.17 mmol) was subjected to a similar procedure to that described for **Intermediate 6,** to give 0.81 g (83%) of **Intermediate 30.** LC-MS: Rt = 5.58 min, m/z = 841 [M+H]⁺, m/z = 839 [M-H]⁻.

### Intermediate 31: Cyclic-(3',3')-[N⁶-(N,N-dimethylformamide)-3'-O-(CE)phosphotriester-2-Chloro-2'-deoxy-Adenosine]-[N¹-POM-3'-O-(CE)phosphotriester-2'-Fluoro-2'-deoxyInosine]

**Intermediate 30** (0.81 g, 0.97 mmol) was subjected to a similar procedure to that described for **Intermediate 7,** to give 0.93 g (96%) of **Intermediate 31.** LC-MS: Rt = 4.85 and 5.01 min, m/z = 956 [M+H]⁺, m/z = 954 [M-H]⁻.

### CL 731

### Intermediate 32: N⁶-Bz-3'-fluoro-3'-deoxy-xylo-Adenosine

At 0°C, to a solution of the known 3'-fluoro-3'-deoxy-xylo-Adenosine (6.90 g, 25.6 mmol) in dry pyridine (320 mL) was added TMSCI (Trimethylsilyl chloride) (16,3 mL, 5 equiv.). The resulting solution was stirred at 0°C for one hour then BzCl (benzoyl chloride) (14,9 mL, 5 equiv.) was added and the reaction mixture was let stirred at r.t. for 12 hours. After completion, the reaction mixture was diluted with water, Ammonia (25% in water) was added. After 15 min, the solution was concentrated in vacuo and the residue was purified by silica-gel column chromatography, using Cyclohexane/EtOAc as eluent to provide 8,5 g of **Intermediate 32.** LC-MS: Rt = 3.29 and 3.52 min, m/z = 374 [M+H]⁺, m/z = 372 [M-H]⁻.

### Intermediate 33: N⁶-Bz-5'-O-DMTr-3'-fluoro-3'-deoxy-xylo-Adenosine

**Intermediate 32** (8.2 g, 21.9 mmol) was subjected to a similar procedure to that described for **Intermediate 1,** to give 10.3 g (70% yield) of **Intermediate 33.** LC-MS: Rt = 6.09 min, m/z = 676 [M+H]⁺.

### Intermediate 34: N⁶-Bz-5'-O-DMTr-3'-fluoro-3'-deoxy-2'-O-(2-(CE)-diisopropylphosphoramidite)-xylo-Adenosine

**Intermediate 33** (10.35 g, 15.3 mmol) was dissolved in dry dichloromethane (567 mL), and to the resulting solution was added DIPEA (13.3 mL, 77 mmol) follow by a dropwise solution of 2-Cyanoethyl *N*,*N*-diisopropylchlorophosphoramidite (4.1 mL, 18.4 mmol) in dry DCM (10 mL). After completion, MeOH was added. The reaction mixture was washed with saturated NaHCO₃ (x3) and brine. The organic layer was dried over MgSO₄, filtered and concentrated in vacuo. The crude compound was purified by silica-gel column chromatography, using DCM/MeOH as eluent to provide 11.4 g (85 % yield) of **Intermediate 34.** LC-MS: Rt = 7.44 and 7.96 min, m/z = 876 [M+H]⁺, m/z = 874 [M-H]⁻.

### Intermediate 35: N⁶-Bz-3'-fluoro-3'-deoxy-2'-O-(Allyl,CE)phosphotriester-xylo-Adenosine

**Intermediate 34** (3.0 g, 3.4 mmol) was co-evaporated three times with dry ACN, and the resulting solid was dissolved in a solution of Activator42® (0.1 mol/L, 2 equiv.) in the presence of molecular sieves (3Â). To the solution was added allyl alcohol (180 µL, 2.64 mmol) and the resulting mixture was stirred for 30 min. Then, tert-butyl hydroperoxide in decane (320 µL, 2.6 mmol) was added to the mixture, which was stirred for 40 min. The solution was filtered and the molecular sieves were washed with DCM. The filtrate was concentrated *in vacuo.* The residue was treated with a solution of DCA/DCM (3%) in the presence of water (10 equiv.) for 15 min. The reaction was quenched with addition of MeOH and pyridine. The solvents were removed *in vacuo* and the residue was purified by silica-gel column chromatography, using DCM/MeOH as eluent to provide 1.35 g (72% yield) of **Intermediate 35.** LC-MS: Rt = 3.92 min, m/z = 547 [M+H]⁺, m/z = 545 [M-H]⁻.

### Intermediate 36: [3'-O-(CE)phosphotriester-2'-fluoro-2'-deoxy-Inosine]-(3',5')-[N⁶-Bz-3'-fluoro-3'-deoxy-2'-O-(Allyl,CE)phosphotriester-xylo-Adenosine]

**Intermediate 35** (440 mg, 0.8 mmol) was co-evaporated three times with dry ACN, and the resulting solid was dissolved in a solution of Activator42® (0.1 mol/L, 2 equiv.; 26 mL) in the presence of molecular sieves (3Â). To the solution was added commercially available of 2'-fluoro-2'-deoxy - Inosine phosphoramidite (1.03g, 1.7 mmol) and the resulting mixture was stirred for 1h00. Then, tert-butyl hydroperoxide in decane (320 µL, 2.56 mmol) was added to the mixture, which was stirred for 30 min. The solution was filtered and the molecular sieves were washed with DCM. The filtrate was concentrated *in vacuo.* The residue was treated with a solution of DCA/DCM (3%) in the presence of water (10 equiv.) for 15 min. The reaction was quenched with addition of MeOH and pyridine. The solvents were removed *in vacuo* and the residue was purified by silica-gel column chromatography, using DCM/MeOH as eluent to give 480 mg (64% yield) of **Intermediate 36.** LC-MS: Rt = 3.24 min, m/z = 892 [M+H]⁺, m/z = 890 [M-H]⁻.

### Intermediate 37: [3'-O-(CE)phosphotriester-2'-fluoro-2'-deoxy-Inosine]-(3',5')-(3'-5')-[N⁶-Bz-3'-fluoro-3'-deoxy-2'-O-(CE)phosphodiester-xylo-Adenosine]

To a solution of dinucleotide **Intermediate 36** (480 mg, 0.8 mmol) in dry THF was added *N*-methyl aniline (0.2 mL, 1.84 mmol) and tetrakis(triphenylphosphine)palladium(0) (0.140 g, 0.12 mmol). The resulting suspension was stirred at rt for 30 min. Then, the solvent was removed *in vacuo* and the residue was triturated with acetone. The resulting colorless precipitate was collected by filtration and washed with chilled acetone to provide 580 mg (90% yield) of **Intermediate 3** which was used for the next step without any purification.

### Intermediate 38: (3',2')Cyclic-[3'-O-(CE)phosphotriester-2'-fluoro-2'-deoxy-Inosine]-[N⁶-Bz-3'-fluoro-3'-deoxy-2'-O-(CE)phosphotriester-xylo-Adenosine]

**Intermediate 37** (330 mg, 0.37 mmol) was co-evaporated three times with dry pyridine. The residue was suspended in dry pyridine, and to the resulting solution was added 1-(Mesitylene-2-sulfonyl)-3-nitro-1,2,4-triazole (MSNT; 1 g, 3.3 mmol). The resulting mixture was stirred at 25 °C for 3 h. Then, the solvent was removed *in vacuo,* to provide 150 mg (46% yield) of **Intermediate 38** which was used in the next step without any further purification. LC-MS: Rt = 3.69, 3.82, 3.92 and 4.04 min, m/z = 874 [M+H]⁺, m/z = 872 [M-H]⁻.

### CL764 et CL765

### Intermediate 39: N¹-POM-5'-O-DMTr-2'-fluoro-2'-deoxy-Inosine

Intermediate 1 (7.0 g, 8.9 mmol) was subjected to a similar procedure to that described for Intermediate 3, to give 5.3 g (86% yield) of **Intermediate 39.** LC-MS: Rt = 6.08 min, m/z = 687 [M+H]⁺, m/z = 685 [M-H]⁻.

### Intermediate 40: N¹-POM-5'-O-DMTr-3'-O-(2-(CE)-diisopropylphosphoramidite)-2'-fluoro-2'-doxy-Inosine

**Intermediate 39** (4.8 g, 7.0 mmol) was subjected to a similar procedure to that described for **Intermediate 35,** to give 6.1 g (quantitative yield) of **Intermediate 40.** LC-MS: Rt = 6.26 and 6.35 min, m/z = 887 [M+H]⁺, m/z = 885 [M-H]⁻.

### Intermediate 41: N⁶-Bz-5'-O-DMTr-3'-fluoro-3'-deoxy-2'-O-Lev-xylo-Adenosine

**Intermediate 32** (4.8 g, 7.1 mmol) was subjected to a similar procedure to that described for **Intermediate 2,** to give **Intermediate 41.** LC-MS: Rt = 6.01 min, m/z = 676 [M+H]⁺, m/z = 674 [M-H]⁻.

### Intermediate 42: N⁶-Bz-2'-O-Lev-3'-fluoro-3'-deoxy-xylo-Adenosine

**Intermediate 41** (5.5 g, 7.1 mmol) was subjected to a similar procedure to that described for **Intermediate 4,** to give 2.7 g (79% yield on 2 steps) of **Intermediate 42.** LC-MS: Rt = 3.62 min, m/z = 472 [M+H]⁺, m/z = 470 [M-H]⁻.

### Intermediate 43: [N¹-POM-3'-O-(CE)phosphorothioate-triester-2'-fluoro-2'-deoxy-Inosine]-(3',5')-[N⁶-Bz-2'-O-Lev-3'-fluoro-3'-deoxy-xylo-Adenosine]

**Intermediate 40** (800 mg, 1.7 mmol) and **Intermediate 42** (1.8 g, 1.2 equiv.) were subjected to a similar procedure to that described for **Intermediate 5,** to give 400 mg (24% yield) of **Intermediate 43.** LC-MS: Rt = 5.19 min, m/z = 987 [M+H]⁺, m/z = 985 [M-H]⁻.

### Intermediate 44: [N¹-POM-5'-O-H-phosphonate-3'-O-(CE)phosphorothioate-triester-2'-fluoro-2'-deoxy-Inosine]-(3',5')-[N⁶-Bz-2'-O-Lev-3'-fluoro-3'-deoxy-xylo-Adenosine]

**Intermediate 43** (400 mg, 0.4 mmol) was subjected to a similar procedure to that described for **Intermediate 17,** to give 370 mg (87% yield) of **Intermediate 43.** LC-MS: Rt = 4.41 min, m/z = 1051 [M+H]⁺, m/z = 1049 [M-H]⁻.

### Intermediate 45: [N¹-POM-5'-O-H-phosphonate-3'-O-(CE)phosphorothioate-triester-2'-fluoro-2'-deoxy-Inosine]-(3',5')-[N⁶-Bz-deoxy-3'-fluoro-3'-xylo-Adenosine]

**Intermediate 44** (370 mg, 0.35 mmol) was subjected to a similar procedure to that described for **Intermediate 6,** to give 250 mg (75% yield) of **Intermediate 45.** LC-MS: Rt = 4.26 min, m/z = 953 [M+H]⁺, m/z = 951 [M-H]⁻.

### Intermediate 46: (3',2')Cyclic-[N¹-POM-3'-O-(CE)phosphorothioate-triester-2'-fluoro-2'-deoxy-Inosine]-[N⁶-Bz-3'-fluoro-3'-deoxy-2'-O-phosphorothioate-diester-xylo-Adenosine]

**Intermediate 45** (250 mg, 0.26 mmol) was subjected to a similar procedure to that described for **Intermediate 19** to give 180 mg (71% yield) of **Intermediate 46.** LC-MS: Rt = 4.86 min, m/z = 967 [M+H]⁺, m/z = 965 [M-H]⁻.

### CL777

### Intermediate 47: [N⁶-Bz-3'-O-(CE)phosphotriester-2'-fluoro-2'-deoxy-Adenosine]-(3',5')-[N¹-POM-2'-O-Lev-3'-fluoro-3'-deoxy-xylo-Inosine]

**Intermediate 47** (0.77 g, 96% yield) was prepared from **Intermediate 15** (400 mg, 0.8 mmol) and commercially available phosphoramidite of 2'-fluoro-2'deoxy-Adenosine (760 mg, 0.9 mmol) as starting material using a similar procedure to that describe for **Intermediate 8.** LC-MS : Rt = 4.76 min, m/z = 971 [M+H]⁺, 969 [M-H]⁻.

### Intermediate 48: [N⁶-Bz-5'-O-H-phosphonate-3'-O-(CE)phosphotriester-2'-fluoro-2'-deoxy-Adenosine]-(3',5')-[N¹-POM-2'-O-Lev-3'-fluoro-3'-deoxy-xylo-Inosine]

**Intermediate 48** (700 mg, 85% yield) was prepared from **Intermediate 47** (770 mg, 0.8 mmol) as starting material using a similar procedure to that describe for **Intermediate 17.** LC-MS: Rt = 3.98 min, m/z = 1035 [M+H]⁺, 1033 [M-H]⁻.

### Intermediate 49: [N⁶-Bz-5'-O-H-phosphonate-3'-O-(CE)phosphotriester-2'-fluoro-2'-deoxy-Adenosine]-(3',5')-[N¹-POM-3'-fluoro-3'-deoxy-xylo-Inosine]

**Intermediate 49** (580 mg, 92% yield) was prepared from **Intermediate 48** (700 mg, 0.7 mmol) as starting material using a similar procedure to that describe for **Intermediate 6.** LC-MS : Rt = 3.81 min, m/z = 937 [M+H]⁺, 935 [M-H]⁻.

### Intermediate 50: Cyclic-(2'-3')-[N⁶-Bz-3'-O-(CE)phosphorotriester-2'-fluoro-2'-deoxy-Adenosine]-[N¹-POM-2'-phosphorodiester-3'-fluoro-3'-deoxy-xylo-Inosine]

**Intermediate 49** (580 mg, 0.6 mmol) was co-evaporated three times with dry pyridine, and the resulting solid was dissolved in dry pyridine (80 mL) under inert atmosphere. DMOCP (400 mg, 2.1 mmol) was added and the resulting solution was stirred at r.t. for 40 min. Water (0.40 mL, 21.7 mmol) was added follow by iodine (204 mg, 0.8 mmol). The resulting solution was let stirred for 1 h at r.t. and then pull into a 0.1 M solution of NaHSO₃ (42 mL). The reaction mixture was diluted in DCM, washed with water (x3), dried on MgSO₄, filtered and concentrated in vacuo. The residue was triturated with diethyl ether, filtrated to provide 420 mg of **Intermediate 50** (72% yield). LC-MS : Rt = 3.80 min, m/z = 935 [M+H]⁺, 933 [M-H]⁻.

### CL779

### Intermediate 51: [N⁶-Bz-3'-O-(CE)phosphorothioate-triester-2'-fluoro-2'-deoxy-Adenosine]-(3',5')-[N¹-POM-2'-Lev-3'-fluoro-3'-deoxy-xylo-Inosine]

**Intermediate 14** (1.6 g, 3.3 mmol) and the commercially available 2'-fluoro-A(Bz) phosphoramidite (3.0 g, 3.96 mmol) were subjected to a similar procedure to that described for preparation of **Intermediate 5,** to give 2.7 g (82% yield) of **Intermediate 51.** LC-MS: Rt = 5.23 min, m/z = 987 [M+H]⁺, m/z = 985 [M-H]⁻.

### Intermediate 52: [N⁶-Bz-5'-O-H-phosphonate-3'-O-(CE)phosphorothioate-triester-2'-fluoro-2'-deoxy-Adenosine]-(3',5')-[N¹-POM-2'-O-Lev-3'-fluoro-3'-deoxy-xylo-Inosine]

**Intermediate 51** (2.7 g, 2.74 mmol) was subjected to a similar procedure to that described for preparation of **Intermediate 17,** to give 1.9 g (66% yield) of **Intermediate 52.** LC-MS: Rt = 4.27 min, m/z = 1051 [M+H]⁺, m/z = 1049 [M-H]⁻.

### Intermediate 53: [N⁶-Bz-5'-O-H-phosphonate-3'-O-(CE)phosphorothioate-triester-2'-fluoro-2'-deoxy-Adenosine]-(3',5')-[N¹-POM-3'-fluoro-3'-deoxy-xylo-Inosine]

**Intermediate 52** (1.9 g, 1.8 mmol) was subjected to a similar procedure to that described for preparation of **Intermediate 6,** to give 1.8 g (quantitative yield) of **Intermediate 53.** LC-MS: Rt = 4.06 min, m/z = 953 [M+H]⁺, m/z = 951 [M-H]⁻.

### Intermediate 54: Cyclic-(3',2')-[N⁶-Bz-3'-O-(CE)phosphorothioate-triester-2'-fluoro-2'-deoxy-Adenosine]-[N¹-POM-2'-O-phosphorothioate-diester-3'-fluoro-3'-deoxy-xylo-Inosine]

**Intermediate 53** (890 mg, 0.93 mmol) was subjected to a similar procedure to that described for preparation of **Intermediate 19,** to give 450 mg of **Intermediate 54.** LC-MS: Rt = 4.70 min, m/z = 967 [M+H]⁺, m/z = 965 [M-H]⁻.

### CL781

### Intermediate 55: Cyclic-(2'-3')-[N⁶-Bz-3'-O-(CE)phosphorothioate-triester-2'-fluoro-2'-deoxy-Adenosine]-[N¹-POM-2'-O-phosphorodiester-3'-fluoro-3'-deoxy-xylo-Inosine]

**Intermediate 55** (800 mg, 90% yield) was prepared from **Intermediate 53** (890 mg, 0.9 mmol) as starting material using a similar procedure to that describe for **Intermediate 50.** LC-MS : Rt = 4.25, 4.32 min, m/z = 951 [M+H]⁺, 949 [M-H]⁻.

### CL710

### Intermediate 56: [N⁶-Bz-3'-O-(CE)phosphotriester-2'-fluoro-2'-deoxy-Adenosine]-(3',5')-[N⁶-Bz-2'-O-(Allyl,CE) phosphotriester-3'-fluoro-3'-deoxy-xylo-Adenosine]

**Intermediate 36** (400 mg, 0.73 mmol) and the commercially available 2'-fluoro-A(Bz) phosphoramidite (770 mg, 1.2 equiv.) were subjected to a similar procedure to that described for **Intermediate 8,** to give 755 mg (99%) of **Intermediate 56.** LC-MS: Rt = 4.56 min, m/z = 1035 [M+H]⁺, m/z = 1033 [M-H]⁻.

### Intermediate 57: [N⁶-Bz-3'-O-(CE)phosphotriester-2'-fluoro-2'-deoxy-Adenosine]-(3',5')-[N⁶-Bz-2'-O-(CE)phosphotriester-3'-fluoro-3'-deoxy-xylo-Adenosine]

**Intermediate 56** (757 mg, 0.73 mmol) was subjected to a similar procedure to that described for **Intermediate 10,** to give 300 mg (41%) of **Intermediate 57.** LC-MS: Rt = 3.76 min, m/z = 995 [M+H]⁺, m/z = 993 [M-H]⁻.

### Intermediate 58: Cyclic-(2',3')-[N⁶-Bz-2'-O-(CE)phosphotriester-3'-Fluoro-3'-deoxy-xylo-Adenosine]-[N⁶-Bz-3'-O-(CE)phosphodiester-2'-fluoro-2'-deoxy-Adenosine]

**Intermediate 57** (300 mg, 0.30 mmol) was subjected to a similar procedure to that described for preparation of **Intermediate 37,** to give 200 mg (67% yield) of **Intermediate 58.** LC-MS: Rt = 4.40 and 5.02 min, m/z = 977 [M+H]⁺, m/z = 975 [M-H]⁻.

### CL714

### Intermediate 59: [N⁶-Bz-3'-O-(CE)phosphorothioate-triester-2'-fluoro-2'-deoxy-Adenosine]-(3',5')-[N⁶-Bz-2'-O-(Allyl,CE) phosphotriester-3'-fluoro-3'-deoxy-xylo-Adenosine]

**Intermediate 36** (1.3 g, 2.3 mmol) and the commercially available 2'-fluoro-A(Bz) phosphoramidite (2.5 g, 1.2 equiv.) were subjected to a similar procedure to that described for **Intermediate 5,** to give 1.97 g (79% yield) of **Intermediate 59.** LC-MS: Rt = 5.09, 5.26 and 5.62 min, m/z = 1051 [M+H]⁺, m/z = 1049 [M-H]⁻.

### Intermediate 60: [N⁶-Bz-3'-O-(CE)phosphorothioate-triester-2'-fluoro-2'deoxy-Adenosine]-(3',5')-[N⁶-Bz-2'-O-(CE)phosphodiester-3'-fluoro-3'-deoxy-xylo-Adenosine]

**Intermediate 59** (1.9 g, 1.8 mmol) was subjected to a similar procedure to that described for **Intermediate** 36, to give 930 mg (49% yield) of **Intermediate 60.** LC-MS: Rt = 4.44 min, m/z = 1011 [M+H]⁺, m/z = 1009 [M-H]⁻.

### Intermediate 61: Cyclic-(2',3')-[N⁶-Bz-2'-O-(CE)phosphotriester-3'-Fluoro-3'-deoxy-xylo-Adenosine]-(3',5')-[N⁶-Bz-3'-O-(CE)-phosphorothioate-triester-2'-fluoro-2'-deoxy-Adenosine]

**Intermediate 60** (930 mg, 0.92 mmol) was subjected to a similar procedure to that described for preparation of **Intermediate 37,** to give 750 mg (82% yield) of **Intermediate 61.** LC-MS: Rt = 4.96 min, m/z = 993 [M+H]⁺, m/z = 992 [M-H]⁻.

### CL760

### Intermediate 62: N⁶-diBOC-3'-fluoro-3'-deoxy-xylo-Adenosine

The known **3'-fluoro-3'-deoxy-xylo-Adenosine** (930 mg, 0.92 mmol) was subjected to a similar procedure to that described for preparation of **Intermediate 39,** to give 550 mg (65% yield) of **Intermediate 62.** LC-MS: Rt = 6.4 min, m/z = 470 [M+H]⁺, m/z = 468 [M-H]⁻.

### Intermediate 63: N⁶-diBOC-5'-O-DMT-3'-fluoro-3'-deoxy-xylo-Adenosine

**Intermediate 62** (550 mg, 1.17 mmol) was subjected to a similar procedure to that described for preparation of **Intermediate 1,** to give 760 mg (84% yield) of **Intermediate 63.** LC-MS: Rt = 7.03 min, m/z = 772 [M+H]⁺, m/z = 770 [M-H]⁻.

### Intermediate 64: N⁶-diBOC-5'-O-DMT-2'-O-Lev-3'-fluoro-3'-deoxy-xylo-Adenosine

**Intermediate 63** (760 mg, 0.98 mmol) was subjected to a similar procedure to that described for preparation of **Intermediate 2,** to give 857 mg (95% yield) **Intermediate 64.** LC-MS: Rt = 7.23 min, m/z = 870 [M+H]⁺, m/z = 868 [M-H]⁻.

### Intermediate 65: N⁶-diBOC-2'-O-Lev-3'-fluoro-3'-deoxy-xylo-Adenosine

**Intermediate 64** (857 mg, 0.98 mmol) was subjected to a similar procedure to that described for preparation of **Intermediate 4,** to give 450 mg (80% yield) of **Intermediate 65.** LC-MS: Rt = 4.59 min, m/z = 568 [M+H]⁺, m/z = 566 [M-H]⁻.

### Intermediate 66: [N⁶-Bz-3'-O-(CE)phosphorothioate-triester-2'-fluoro-2'-deoxy-Adenosine]-(3',5')-[N⁶-diBOC-2'-Lev-3'-fluoro-3'-deoxy-xylo-Adenosine]

**Intermediate 65** (250 mg, 0.44 mmol) and the commercially available 2'-fluoro-A(Bz) phosphoramidite (405 mg, 1.2 equiv.) were subjected to a similar procedure to that described for preparation of **Intermediate 5,** to give 390 mg (83% yield) of **Intermediate 66.** LC-MS: Rt = 5.81 min, m/z = 1072 [M+H]⁺, m/z = 1070 [M-H]⁻.

### Intermediate 67: [N⁶-Bz-5'-O-H phosphonate-3'-O-(CE)phosphorothioate-triester-2'-fluoro-2'-deoxy-Adenosine]-(3',5')-[N⁶-diBOC-2'-O-Lev-3'-fluoro-3'-deoxy-xylo-Adenosine]

**Intermediate 66** (390 mg, 0.36 mmol) was subjected to a similar procedure to that described for preparation of **Intermediate 17,** to give 310 mg (75%) of **Intermediate 67.** LC-MS: Rt = 4.62 min, m/z = 1136 [M+H]⁺, m/z = 1134 [M-H]⁻.

### Intermediate 68: [N⁶-Bz-5'-O-H phosphonate-3'-O-(CE)phosphorothioate-triester-2'-fluoro-2'-deoxy-Adenosine]-(3',5')-[N⁶-diBoc-3'-fluoro-3'-deoxy-xylo-Adenosine]

**Intermediate 67** (310 mg, 0.27 mmol) was subjected to a similar procedure to that described for preparation of **Intermediate 6,** to give 260 mg (92%) of **Intermediate 68.** LC-MS: Rt = 5.08 min, m/z = 1038 [M+H]⁺, m/z = 1036 [M-H]⁻.

### Intermediate 69: Cyclic-(3',2')-[N⁶-Bz-3'-O-(CE)phosphorothioate-triester-2'-fluoro-2'-deoxy-Adenosine]-[N⁶-diBoc-2'-O-phosphorothioate-diester-3'-fluoro-3'-deoxy-xylo-Adenosine]

**Intermediate 68** (260 mg, 0.25 mmol) was subjected to a similar procedure to that described for preparation of **Intermediate 19,** to give **Intermediate 69.** LC-MS: Rt = 5.22 min, m/z = 952 [M+H-Boc]⁺, m/z = 950 [M-H-Boc]⁻.

### CL766

### Intermediate 70: [N⁶-Bz-3'-O-(CE)phosphorothioate-triester-2'-fluoro-2'-deoxy-Adenosine]-(3',5')-[N⁶-Bz-2'-O-Lev-3'-fluoro-3'-deoxy-xylo-Adenosine]

**Intermediate 70** (600 mg, 72% yield) was prepared from **Intermediate 42** (400 mg, 0.85 mmol) and commercially available phosphoramidite of 2'-fluoro-2'deoxyAdenosine (780 mg, 0.9 mmol) as starting material using a similar procedure to that describe for **Intermediate 5.** LC-MS : Rt = 4.99 min, m/z = 976 [M+H]⁺, 974 [M-H]⁻.

### Intermediate 71: [N⁶-Bz-5'-O-H-phosphonate-3'-O-(CE)phosphorothioate-triester-2'-fluoro-2'-deoxy-Adenosine]-(3',5')-[N⁶-Bz-2'-O-Lev-3'-fluoro-3'-deoxy-xylo-Adenosine]

**Intermediate 71** (420 mg, 65% yield) was prepared from **Intermediate 70** (600 mg, 0.6 mmol) as starting material using a similar procedure to that describe for **Intermediate 17.** LC-MS: Rt = 4.08 min, m/z = 1040 [M+H]⁺, 1038 [M-H]⁻.

### Intermediate 72: [N⁶-Bz-5'-O-H-phosphonate-3'-O-(CE)phosphorothioate-triester-2'-fluoro-2'-deoxy-Adenosine]-(3',5')-[N⁶-Bz-3'-fluoro-3'-deoxy-xylo-Adenosine]

**Intermediate 72** (330 mg, 87% yield) was prepared from **Intermediate 69** (420 mg, 0.4 mmol) as starting material using a similar procedure to that describe for **Intermediate 6.** LC-MS : Rt = 4.19 min, m/z = 942 [M+H]⁺, 940 [M-H]⁻.

### Intermediate 73: Cyclic-(2'-3')-[N⁶-Bz-3'-O-(CE)phosphorothioate-triester-2'-fluoro-2'-deoxy-Adenosine]-(3',5')-[N⁶-Bz-2'-O-phosphorothioate-3'-fluoro-3'-deoxy-xylo-Adenosine]

**Intermediate 73** (220 mg, 66% yield) was prepared from **Intermediate 70** (330 mg, 0.35 mmol) as starting material using a similar procedure to that describe for **Intermediate 19.** LC-MS: Rt = 4.80 min, m/z = 956 [M+H]⁺, 954 [M-H]⁻.

### CL672

### Intermediate 74: N²-iBu-3'-fluoro-3'-deoxy-xylo-Guanosine

The **Intermediate 74** was preparated from 3'-fluoro-3'-deoxy-xyloGuanosine in 2 steps. Briefly, to a solution of the nucleoside (0.45 g, 1.57 mmol) was added TMSCI (0.60 mL, 0.51 mmol) and the resulting mixture was stirred for 30 min at RT. Then, (*i*BuCO)₂O (0.32 mL, 0.31 mmol) was added and the new solution was stirred for 2h at rt. The reaction was quenched with water. The solvents were removed in vacuo, and the residue was coevaporated with dry pyridine. The residue was dissolved in a mixture of pyridine/DCM (5/1) (30 mL) and DMTrCl (0.42 g, 1.24 mmol) and the mixture was stirred overnight at rt. The solvents were removed in vacuo, and the residue was applied to silica-gel column chromatography, using DCM/ MeOH with 0.5% pyridine as eluent.to provide 0.65 mg (88% yield) of **Intermediate 74.** LC-MS: Rt = 4.91 min, m/z = 658 [M+H]+, m/z = 656 [M-H]-.

### Intermediate 75: N²-iBu-2'-O-(2-CE-diisopropylphosphoramidite)-3'-fluoro-3'-deoxy-xylo-Guanosine

The **Intermediate 74** (0.28 g, 1.17 mmol) was subjected to a similar procedure to that described for **Intermediate 51,** to give 0.63 g (47%) of **Intermediate 75.** LC-MS: Rt = 5.03, 5.34 min, m/z = 858 [M+H]+, m/z = 856 [M-H]-.

### Intermediate 76: [N²-iBu-2'-O-(CE)phosphotriester-3'-fluoro-3'-deoxy-xylo-Guanosine]-(2',5')-[N⁶-Bz-3'-O-(Allyl,CE)phosphotriester2'-fluoro-2'-deoxy-Adenosine]

**Intermediate 26** (0.64 g, 0.74 mmol) was reacted with **Intermediate 75** (0.37 g, 0.68 mmol) using a similar procedure to that described for preparation of **Intermediate 52,** to give 0.32 g (46% yield) of **Intermediate 79.** LC-MS: Rt = 5.00 and 5.10 min, m/z = 1017 [M+H]⁺, m/z = 1015 [M-H]⁻.

### Intermediate 77: [N²-iBu-2'-O-(CE)phosphotriester-3'-fluoro-3'-deoxy-xylo-Guanosine]-(2',5')-[N⁶-Bz-3'-O-(CE)phosphodiester-2'-deoxy-2'-fluoro-Adenosine]

**Intermediate 76** (0.32 g, 0.31 mmol) was reacted with *N*-methyl aniline (0.1 g, 0.93 mmol) and tetrakis(triphenylphosphine)palladium(0) (0.072 g, 0.062 mmol) using a similar procedure to that described for preparation of **Intermediate 28,** to give 0.196 g (65% yield) of **Intermediate 77.** LC-MS: Rt = 4.40 and 4.69 min, m/z = 977 [M+H]⁺, m/z = 975 [M-H]⁻.

### Intermediate 78: (2',3')Cyclic-[N²-iBu-2'-O-(CE)phosphotriester-3'-fluoro-3'-deoxy-xylo-Guanosine]-[N⁶-Bz-2'-O-(CE)phosphotriester-2'-fluoro-2'-deoxy-Adenosine]

**Intermediate 77** (0.196 g, 0.20 mmol) was reacted with MSNT (0.30 g, 1.0 mmol) and using a similar procedure to that described for preparation of **Intermediate 29,** to give 0.19 g (100% yield) of **Intermediate 78.** LC-MS: Rt = 3.17, m/z = 959 [M+H]⁺, m/z = 957 [M-H]⁻.

### CL739

### Intermediate 79: N²-iBu-5'-O-DMTr-2'-O-Lev-3'-fluoro-3'-deoxy-xylo-Guanosine

The **Intermediate 78** (0.5 g, 0.76 mmol) in dry DMF (10 mL) was reacted with EDCI (0.16 g, 0.83 mmol), DMAP (0.093 g, 0.76 mmol) and levulinic acid (0.106 mL, 0.91 mmol) using a similar procedure to that described for preparation of **Intermediate 2,** to give 0.46 g (80% yield) of **Intermediate 79.** LC-MS: Rt = 6.12 min, m/z = 756 [M+H]⁺, m/z = 754 [M-H]⁻.

### Intermediate 80: N²-iBu-2'-O-Lev-3'-fluoro-3'-deoxy-xylo-Guanosine

The **Intermediate 79** (1.0 g, 1.31 mmol) was subjected to a similar procedure to that described for **Intermediate 4,** to give 0.56 g (94% yield) of **Intermediate 80.** LC-MS: Rt = 3.50 min, m/z = 454 [M+H]⁺, m/z = 452 [M-H]⁻.

### Intermediate 81: [N⁶-Bz-3'-O-(CE)phosphorothioate-triester-2'-fluoro-2'-deoxy-Adenosine]-(3',5')-[N²-iBu-2'-O-Lev-3'-fluoro-3'-deoxy-xylo-Guanosine]

**Intermediate 80** (0.30 g, 0.66 mmol) was reacted with commercially available phosphoramidite of 2'-fluoro-2'-deoxyAdenosine (0.61 g, 0.69 mmol) using a similar procedure to that described for preparation of **Intermediate 5** to provide 0.63 g (99% yield) of **Intermediate 81.** LC-MS: Rt = 4.82 and 4.99 min, m/z = 958 [M+H]⁺, m/z = 956 [M-H]⁻.

### Intermediate 82: [N⁶-Bz-5'-O-(H-phosphonate)-3'-O-(CE)phosphorothioate-triester-2'-fluoro-2'-deoxy-Adenosine]-(3',5')-[N²-iBu-2'-O-Lev-3'-fluoro-3'-deoxy-xyto-Guanosine]

**Intermediate 81** (0.63 g, 0.66 mmol) was subjected to a similar procedure to that described for **Intermediate 17,** to give 0.36 g (53% yield) of **Intermediate 82.** LC-MS: Rt = 4.00 min, m/z = 1022 [M+H]⁺, m/z = 1020 [M-H]⁻.

### Intermediate 83: [N⁶-Bz-5'-O-(H-phosphonate)-3'-O-(CE)phosphorothioate-triester-2'-fluoro-2'-deoxy-Adenosine]-(3',5')-[N²-iBu-3'-fluoro-3'-deoxy-xylo-Guanosine]

**Intermediate 79** (0.36 g, 0.35 mmol) was subjected to a similar procedure to that described for **Intermediate 6,** to give 0.32 g (99% yield) of **Intermediate 83.** LC-MS: Rt = 3.83 min, m/z = 924 [M+H]⁺, m/z = 922 [M-H]⁻.

### Intermediate 84: (2',3')Cyclic-[N²-iBu-2'-O-(CE)phosphorothioate-triester-3'-fluoro-3'-deoxy-xylo-Guanosine]-[N⁶-Bz-2'-O-(CE)phosphorothioate-triester-2'-fluoro-2'-deoxy-Adenosine]

**Intermediate 83** (0.32 g, 0.35 mmol) was subjected to a similar procedure to that described for **Intermediate 19,** to give 0.3 g (92% yield) of **Intermediate 84.** LC-MS: Rt = 4.20 and 4.47 min, m/z = 938 [M+H]⁺, m/z = 936 [M-H]⁻.

### CL715

### Intermediate 85: [N²-iBu-2'-O-(CE)phosphotriester-2'-fluoro-2'-deoxy-Guanosine]-(3',5')-[N⁶-Bz-3'-fluoro-3'-deoxy-2'-O-(Allyl,CE)phosphotriester-xylo-Adenosine]

**Intermediate 36** (450 mg, 0.8 mmol) was subjected to a similar procedure to that described for **Intermediate 37** with the commercially available phosphoramidite of 2'-fluoro-2'-deoxyGuanosine, to give 660 mg (79% yield) of **Intermediate 85.** LC-MS: Rt = 4.71 min, m/z = 1017 [M+H]⁺, m/z = 1015 [M-H]⁻.

### Intermediate 86: [N²-iBu-2'-O-(CE)phosphotriester-2'-fluoro-2'-deoxy-Guanosine]-(3',5')-[N⁶-Bz-3'-fluoro-3'-deoxy-2'-O-(CE)phosphodiester-xylo-Adenosine]

**Intermediate 85** (660 mg, 0.65 mmol) was subjected to a similar procedure to that described for **Intermediate 38** to give 160 mg (25% yield) of **Intermediate 86.** LC-MS: Rt = 3.71 min, m/z = 977 [M+H]⁺, m/z = 975 [M-H]⁻.

### Intermediate 87: (3',2')Cyclic-[N²-iBu-2'-O-(CE)phosphotriester-2'-fluoro-2'-deoxy-Guanosine]-[N⁶-Bz-3'-fluoro-3'-deoxy-2'-O-(CE)phosphotriester-xylo-Adenosine]

**Intermediate 86** (160 mg, 0.16 mmol) was subjected to a similar procedure to that described for **Intermediate 39,** to give 55 mg (35% yield) of **Intermediate 87.** LC-MS: Rt = 4.36 min, m/z = 959 [M+H]⁺, m/z = 957 [M-H]⁻.

### General protocol

### General protocol for the deprotection of CDNs:

Method A: The protected CDN of was treated with a solution of methylamine in EtOH (33%), and the resulting mixture was stirred at 50 °C for 4 h. The reaction mixture was concentrated.

Method B: The protected CDN was dissolved in DCM (0.1 M) and was treated with tert-butylamine (10 equiv.). The resulting mixture was stirred at rt for 2h. The desired product was precipitated out by addition of ether.

Method C: The protected CDN was dissolved in dry ACN (0.2 M) and was treated with DBU (0.5 equiv.). The resulting mixture was stirred at rt for 30 min. Then, the reaction mixture was diluted with EtOAc and was washed with saturated NaHCO₃, water and brine. The organic layer was dried over MgSO₄, filtered and concentrated *in vacuo.* The crude compound was treated with a solution of HCl 4M in dioxane (30 equiv.) for 5h. The solvents were removed *in vacuo* and the residue was purified using a similar procedure to that described in general protocol method.

### General protocol for the purification of CDNs:

The crude was subjected to preparative HPLC using a C₁₈ Sunfire column (19 x 150 mm, 5µm) and ammonium formate/ACN as eluent. The fractions containing the desired compound were pooled and lyophilized. The structure of the compound was confirmed by LC-ES/MS analysis with ions at [M-H]⁻and/or [M+H]⁺.

### General protocol for the POM introduction on phosphorothioate moiety:

To a solution of CDN in ultrapure water (0.02 M) was added a solution of iodomethyl pivalate (9 equiv.) in acetone (0.2 M). The reaction mixture was covered in aluminum foil, and stirred overnight. Then the solvents were removed *in vacuo,* and the residue was purified by silica-gel column chromatography, using DCM/MeOH as eluent.

### General protocol for the POM introduction on Inosine base moiety:

To a solution of CDN in dry DMF (0.2 M) was added K₂CO₃ (15 equiv.) and POM-Cl (6 equiv.). The reaction mixture was stirred at rt overnight. Then the mixture was filtered (0.22 µ), then diluted with EtOAc and extracted with water. The aqueous layer was dried *in vacuo* and the desired product was precipitated out by addition of ether.

### Example 1.1:

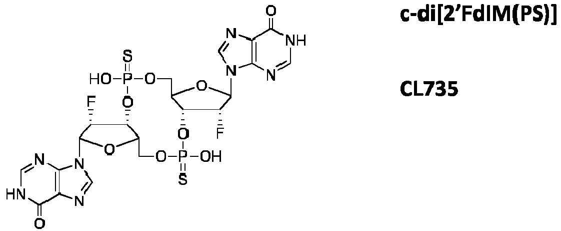

**Example 1.1** (240 mg, 39% yield) was prepared from **Intermediate 7** using a similar procedure to that described in general protocol method A. LC-MS: Rt = 2.71, 2.83, 3.11 min, m/z = 697 [M+H]⁺, m/z = 695 [M-H]⁻. ¹H NMR (D₂O, 300 MHz) δ (ppm) 8.52-7.97 (m, 4H), 6.56-6.32 (m, 2H), 6.05-5.58 (s, 2H), 5.49-5.16 (s, 2H), 4.62-4.46 (m, 4H), 4.18-4.11 (m, 2H).

### Example 1.2:

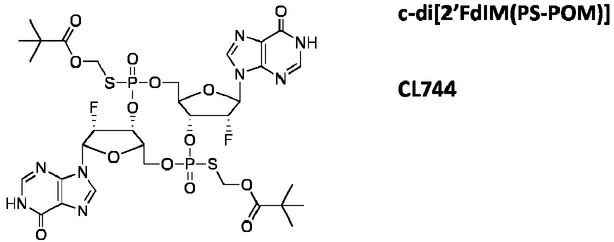

**Example 1.2** (15 mg, 62% yield) was prepared from **Exemple 1.1** using a similar procedure to that described in general protocol for the POM introduction on phosphorothioate moiety. LC-MS: Rt = 4.90 min, m/z = 925 [M+H]⁺, m/z = 923 [M-H]⁻. ¹H NMR (DMSO-*d₆*, 300 MHz) δ (ppm) 10.40 (s, 2H), 8.36 (s, 2H),8.11 (s, 2H), 6.50 (m, 2H), 6.26 (m, 4H), 5.65-5.49 (m, 2H), 5.20-5.02 (m, 2H), 4.55-4.26 (m, 6H), 1.10 (s, 9H), 1.05 (s, 9H)

### Example 1.3:

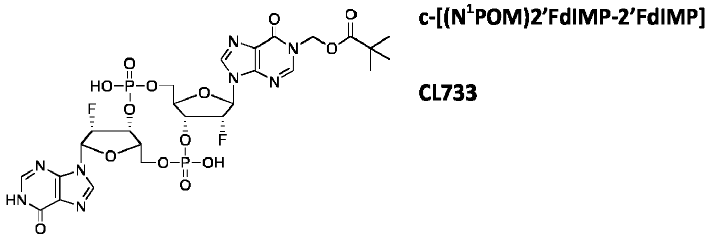

**Example 1.3** (34 mg, 22% yield) was prepared from **Intermediate 10** using a similar procedure to that described in general protocol method A. LC-MS: Rt = 2.69 min, m/z = 779 [M+H]⁺, m/z = 777 [M-H]⁻. ¹H NMR (D₂O, 300 MHz) δ (ppm) 8.48-8.11 (m, 4H), 6.54-6.42 (m, 2H), 6.17-5.81 (m, 2H), 5.64-5.33 (m, 2H), 5.66-5.03 (m, 2H), 4.56-4.40 (m, 4H), 4.20-4.16 (m, 2H), 1.15 (s, 9H).

### Example 1.4:

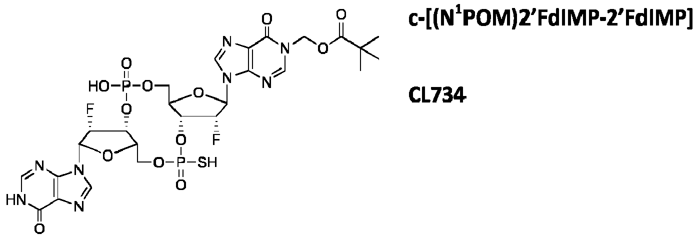

**Example 1.4** (23 mg, 14% yield) was prepared from **Intermediate 11** using a similar procedure to that described in general protocol method A. LC-MS: Rt = 2.89 min, m/z = 795 [M+H]⁺, m/z = 793 [M-H]⁻. ¹H NMR (D₂O, 300 MHz) δ (ppm) 8.70-8.16 (m,4), 6.55-6.43 (m, 2H), 5.97-5.46 (m, 4H), 5.07 (m, 2H), 4.58 (m, 4H), 4.15 (m, 2H), 1.14 (s, 9H).

### Example 1.5:

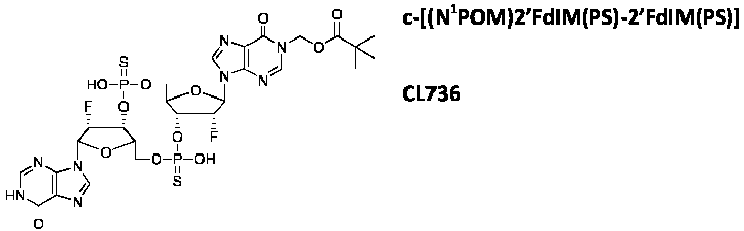

**Example 1.5** (59 mg, 19% yield) was prepared from **Intermediate 7** by treatment with *t*BuNH₂ for 30 min using a similar procedure to that described in general protocol method A. LC-MS: Rt = 3.22, 3.29, 3.67, 3.86 min, m/z = 811 [M+H]⁺, m/z = 809 [M-H]⁻. ¹H NMR (D₂O, 300 MHz) δ (ppm) 8.70-7.94 (m, 4H), 6.71-6.51 (m, 2H), 6.46-6.03 (s, 2H), 5.99-5.46 (s, 2H), 4.63-4.50 (m, 4H), 4.19-4.02 (m, 2H), 1.03 (s, 9H).

### Example 1.6:

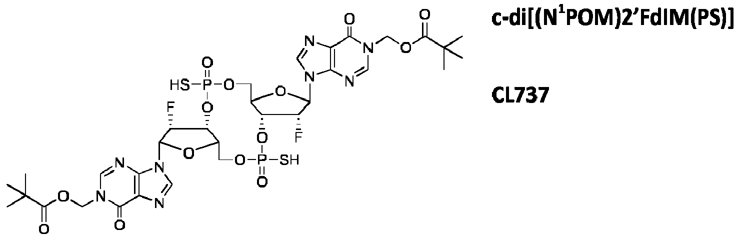

**Example 1.6** (10 mg, 43% yield) was prepared from **Example 1.1** using a similar procedure to that described in general protocol for the POM introduction on Inosine base moiety. LC-MS: Rt = 3.68, 3.82, 4.00 min, m/z = 925 [M+H]⁺, m/z = 923 [M-H]⁻. ¹H NMR (D₂O, 300 MHz) δ (ppm) 8.49 (s, 2H), 8.14 (s, 2H), 6.36 (s, 2H), 6.11 (m, 2H), 5.66 (m, 2H), 5.12 (m, 2H), 5.20 (m, 2H), 4.44-4.14 (m, 4H), 1.06 (s, 18H).

### Example 1.7:

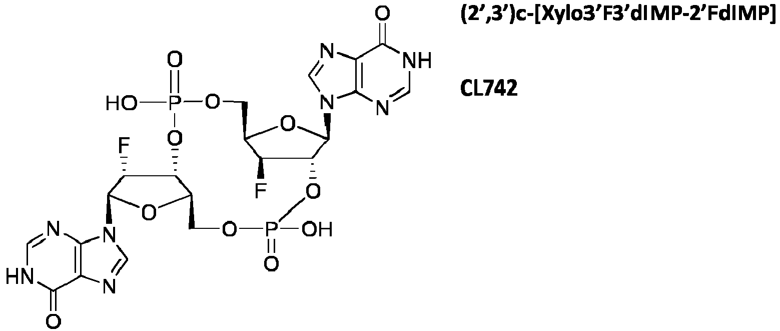

**Example 1.7** (64 mg, 20% yield) was prepared from **Intermediate 19** (340 mg, 0.41 mmol) which was co-evaporated three times with dry pyridine, and the resulting solid was dissolved in dry pyridine (53 mL). 2-Chloro-5,5-dimethyl-1,3,2-dioxaphosphorinane 2-oxide (DMOCP; 263 mg, 1.5 equiv.) was added. The resulting mixture was stirred for 40 min. Then, oxidation was realized by an addition of water (257 µL, 35 equiv.) and diiodine (135 mg, 1.3 equiv.). The resulting mixture was stirred for 1 h, and then sodium bicarbonate (1.6 g, 4.4 equiv.) was added. The crude solution was concentrated *in vacuo* and the residue was purified by preparative HPLC to provide after Sodium exchange and lyophilization. LC-MS: Rt = 2.57 min, m/z = 665 [M+H]⁺, m/z = 663 [M-H].]⁻. ¹H NMR (D₂O, 300 MHz) δ (ppm) 8.15 (m, 4H), 6.38 (m, 2H), 5.76 (m, 1H), 5.59 (m, 1H), 5.06 (m, 2H), 4.40 (m, 4H).

### Example 1.8

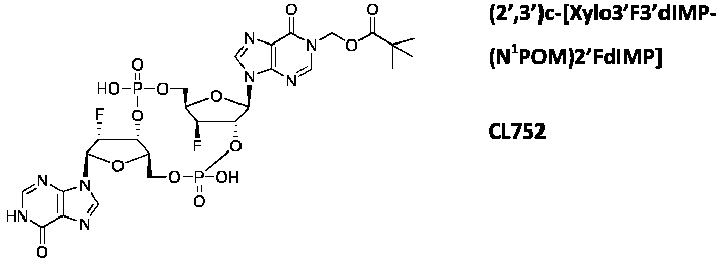

### Example 1.9

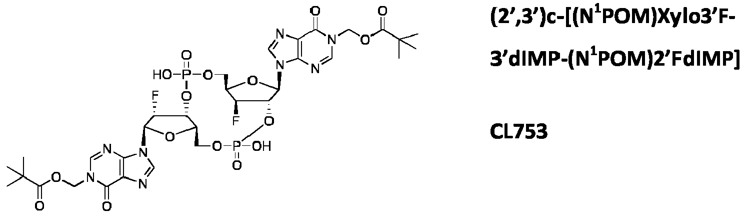

**Example 1.8** (0.5 mg, 0.5% yield) and **Example 1.9** (2 mg, 10% yield) were prepared from Example 1.7 using a similar procedure to that described in general protocol for the POM introduction on Inosine base moiety.

**Example 1.8:** LC-MS: Rt = 3.08 min, m/z = 779 [M+H]⁺, m/z = 777 [M-H], ¹H NMR (D₂O, 300 MHz) δ (ppm) 8.47, 8.25, 8.17 (4xs, 1H), 6.40 (m, 2H), 6.01 (s, 2H), 5.61 (m, 2H), 5.04 (m, 2H), 4.11-3.83 (m, 4H), 1.12, 1.11, 1.09 (3xs, 9H).

**Example 1.9:** LC-MS: Rt = 3.68 min, m/z = 893 [M+H]⁺, m/z = 891 [M-H]. ¹H NMR (D₂O, 300 MHz) δ (ppm) 8.49, 8.46, 8.25, 8.14 (4xs, 1H), 6.40 (m, 2H), 6.00 (s, 4H), 5.65 (m, 2H), 5.04 (m, 2H), 4.42-4.12 (m, 4H), 1.102, 1.06 (2xs, 18H).

### Example 1.10:

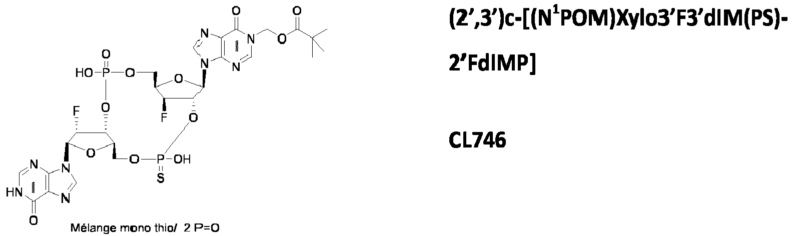

**Example 1.10** (170 mg, 48% yield) was prepared from **Intermediate 19** as starting material using a similar procedure to that described in general protocol method B. LC-MS: Rt = 3.49, 3.80 min, m/z = 795 [M+H]⁺, 793 [M-H]⁻.. ¹H NMR (D₂O, 300 MHz) δ (ppm) 8.65-7.98 (m, 4H), 6.30 (m, 2H), 5.95 (m, 4H), 5.71 (m, 2H), 5.45-5.15 (m, 4H), 4.37-4.32 (m, 2H), 1.10 (m, 9H).

### Example 1.11

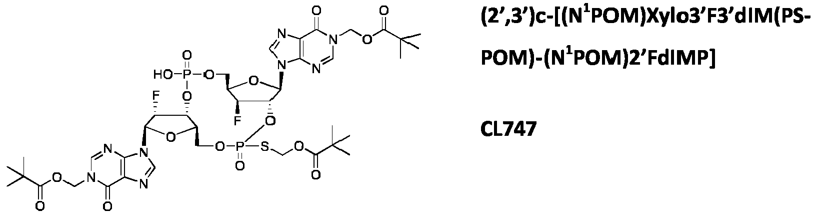

**Example 1.11** (34 mg, 18% yield) was prepared from **Intermediate 20** as starting material using a similar procedure to that described for **Exemple 1.2.** LC-MS: Rt = 5.06, 5.18 min, m/z = 1023 [M+H]⁺, 1021 [M-H. ¹H NMR (D₂O, 300 MHz) δ (ppm) 8.82-804 (m, 4H), 6.33 (m, 2H), 5.98 (m, 4H), 5.70 (m, 2H), 5.40-5.10 (m, 4H), 4.37-4.27 (m, 6H), 1.10, 0.74 (m, 27H).

### Example 1.12

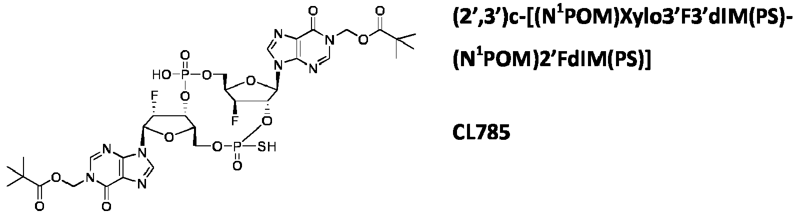

**Example 1.12** (157 mg, 35% yield) was prepared from Intermediate 25 using a similar procedure to that described in general protocol method C. LC-MS: Rt = 3.85, 3.79 min, m/z = 925 [M+H]⁺, m/z = 923 [M-H]⁻. ¹H NMR (D₂O, 300 MHz) δ (ppm) 8.38-8.48 (m, 3H), 7.95 (s, 1H), 6.21 (m, 2H), 5.86 (m, 4H), 5.60 (m, 1H), 4.18-4.59 (m, 6H), 3.65 (m, 3H), 1.04, 1.03 (2xs, 18H).

### Example 1.13

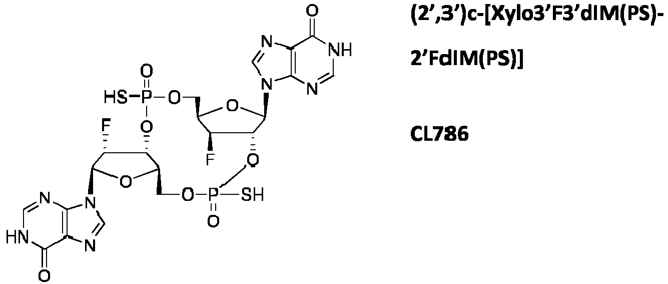

**Example 1.13** (280 mg, 73% yield) was prepared from **Example 1.12** using a similar procedure to that described in general protocol method A. LC-MS: Rt = 8.99, 15.4 min, m/z = 697 [M+H]⁺, m/z = 695 [M-H]⁻. ¹H NMR (D₂O, 300 MHz) δ (ppm) 8.05-8.22 (m, 4H), 6.12 (m, 2H), 5.60 (m, 1H), 5.52 (m, 1H), 5.00-5.011 (m, 2H), 4.13-4.58 (m, 4H).

### Example 1.14

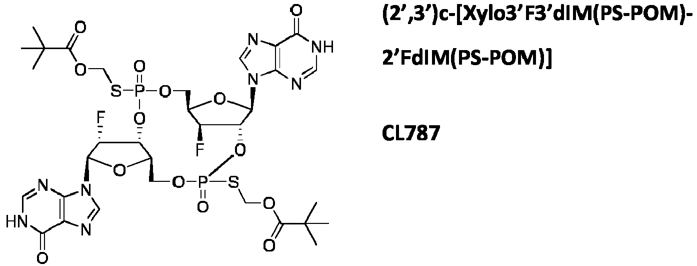

**Example 1.14** (70.5 mg, 26% yield) was prepared from **Example 1.13** using a similar procedure to that described in general protocol for the POM introduction on phosphorothioate moiety. LC-MS: Rt = 4.55, 4.74 min, m/z = 925 [M+H]⁺, m/z = 923 [M-H]⁻. ¹H NMR (D₂O, 300 MHz) δ (ppm) 8.00-8.27 (m, 4H), 5.03-6.44 (m, 10H), 4.39-4.75 (m, 6H), 1.12, 0.89 (2xs, 18H).

### Example 1.15

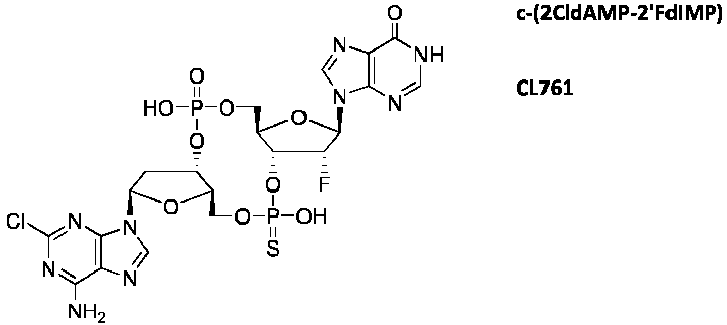

**Example 1.15** (50 mg, 75% yield) was prepared from Intermediate 31 (0.93 g, 0.97 mmol) using a similar procedure to that described in general protocol method A. LC-MS: Rt = 0.69 min, m/z = 680 [M+H]⁺, m/z = 678 [M-H]⁻. ¹H NMR (D₂O, 300 MHz) δ (ppm) 8.42 (s, 1H), 8.20 (s, 1H), 7.91 (s, 1H), 6.25 (m, 2H), 5.15 (m, 1H), 4.85 (m, 2H), 4.68-4.41 (m, 4H). 4.03 (m, 3H).

### Example 1.16:

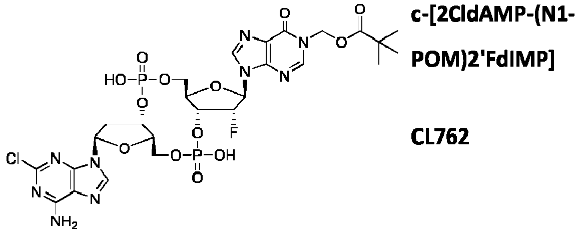

### Example 1.17:

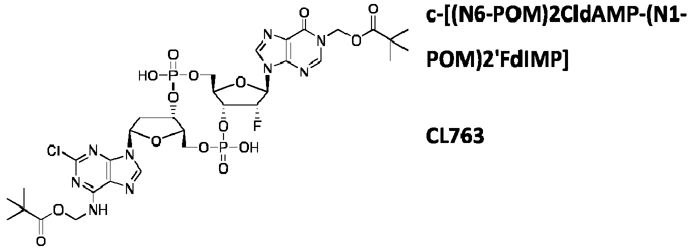

**Example 1.16** (5 mg, 37% yield) and **Example 1.17** (10 mg, 8%) were prepared from **Example 1.15** (0.09 g, 0.13 mmol) using a similar procedure to that described in general protocol for the POM introduction on base moiety.

**Example 1.16** LC-MS: Rt = 1.33 and 1.42 min, m/z = 794 [M+H]⁺, m/z = 792 [M-H]⁻. ¹H NMR (D₂O, 300 MHz) δ (ppm) 8.47 (s, 1H), 8.25 (s, 1H), 8.21 (s, 1H), 6.37 (m, 2H), 5.91 -5.68 (m, 3H), 5.22 (m, 2H), 4.91 (m, 1H), 4.40 (m, 2H), 4.27 (m, 2H), 4.06 (m, 1H), 2.86-2.63 (m, 2H), 1.23 (s, 9H).

**Example** 1.17 LC-MS: Rt = 3.88 min, m/z = 908 [M+H]⁺, m/z = 906 [M-H]⁻. ¹H NMR (D₂O, 300 MHz) δ (ppm) 8.64 (s, 1H), 8.24 (s, 1H), 8.15 (s, 1H), 6.34 (m, 2H), 5.82-5.52 (m, 5H), 4.40-4.10 (m, 6H), 3.03-2.76 (m, 2H). 1.09-1.07 (m, 18H).

### Example 1.18:

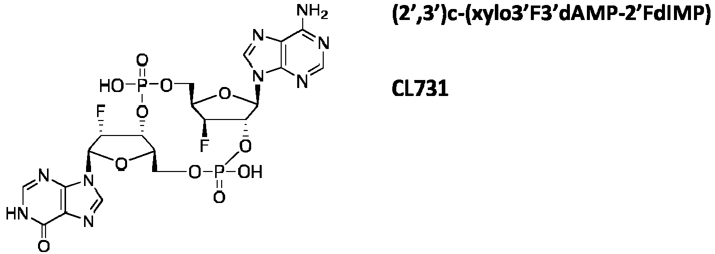

**Example 1.18** (18 mg, 16% yield) was prepared from of **Intermediate 38** (150 mg, 0.17 mmol) using a similar procedure to that described in general protocol method A to provide. LC-MS: Rt = 2.88 min, m/z = 664 [M+H]⁺, m/z = 662 [M-H]⁻. ¹H NMR (D₂O, 300 MHz) 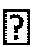 (ppm) 8.19 (m, 4H), 6.40 (m, 2H), 5.70 (m, 1H), 5.32 (m, 1H), 5.00 (m, 2H), 4.42 (m, 4H).

### Example 1.19:

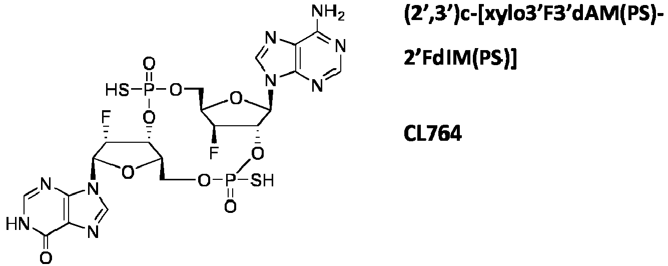

**Example 1.19** (110 mg, 84% yield) of was prepared from **Intermediate 46** (180 mg, 0.18 mmol) using a similar procedure to that described in general protocol method A. LC-MS: Rt = 2.53, 2.63 min, m/z = 696 [M+H]⁺, m/z = 694 [M-H]⁻. ¹H NMR (D₂O, 300 MHz) δ (ppm) 8.00-8.35 (m, 4H), 5.10-6.51 (m, 4H), 3.96-4.74 (m, 8H).

### Example 1.20:

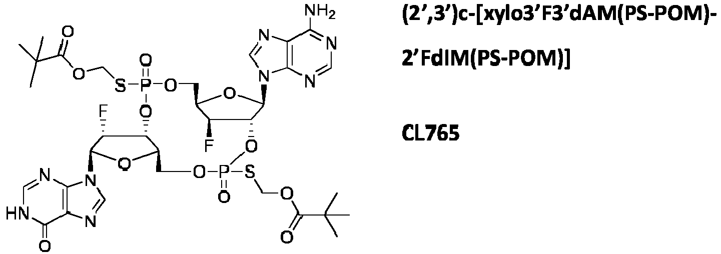

**Example 1.20** (30 mg, 2% yield) of was prepared from Example 1.19 (10 mg, 0.16 mmol) using a similar procedure to that described in general protocol for the POM introduction on phosphorothioate moiety. LC-MS: Rt = 4.45, 4.65 min, m/z = 924 [M+H]⁺, m/z = 922 [M-H]⁻. ¹H NMR (DMSO, 300 MHz) 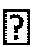 (ppm) 8.84 (s, 1H), 8.55 (s, 1H), 6.07 (d, 1H), 5.00 (dd, 1H), 4.80 (d, 1H), 4.35 (m, 1H), 3.73 (m, 2H), 1.44 (s, 18H).

### Example 1.21:

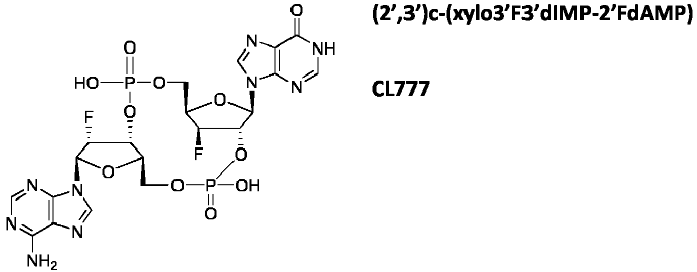

**Example 1.21** (77.5 mg, 26% yield) was prepared from **Intermediate 50** using a similar procedure to that described in general protocol method A. LC-MS: Rt = 2.75 min, m/z = 664 [M+H]⁺, m/z = 662 [M-H]⁻. ¹H NMR (D₂O, 300 MHz) δ (ppm) 8.12-8.26 (m, 4H), 6.37 (m, 2H), 5.49-5.75 (m, 2H), 4.21-5.09 (m, 8H).

### Example 1.22:

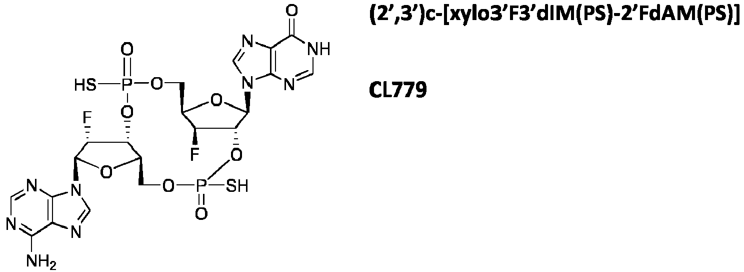

**Example 1.22** (30 mg, 10% yield) was prepared from **Intermediate 54** using a similar procedure to that described in general protocol method A. LC-MS: Rt = 2.53 min, m/z = 696 [M+H]⁺, m/z = 694 [M-H]⁻. ¹H NMR (D₂O, 300 MHz) δ (ppm) 8.7.82-8.47 (m, 4H), 4.82-6.48 (m, 4H), 4.05-4.66 (m, 8H).

### Example 1.23:

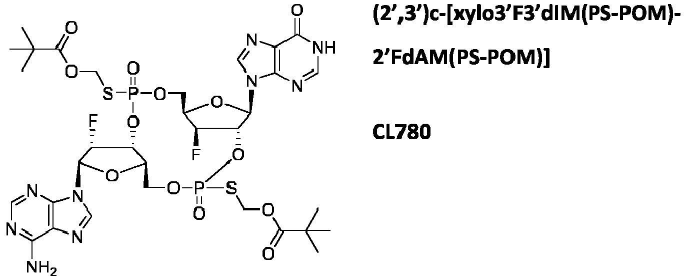

**Example 1.23** (24 mg, 11% yield) was prepared from **Example 1.22** using a similar procedure to that described in general protocol for the POM introduction on phosphorothioate moiety. LC-MS: Rt = 4.74, 4.88 min, m/z = 924 [M+H]⁺, m/z = 922 [M-H]⁻. ¹H NMR (D₂O, 300 MHz) δ (ppm) 8.01-8.19 (m, 4H), 4.37-6.45 (m, 14H), 1.06,0.82 (2xs, 18H).

### Example 1.24:

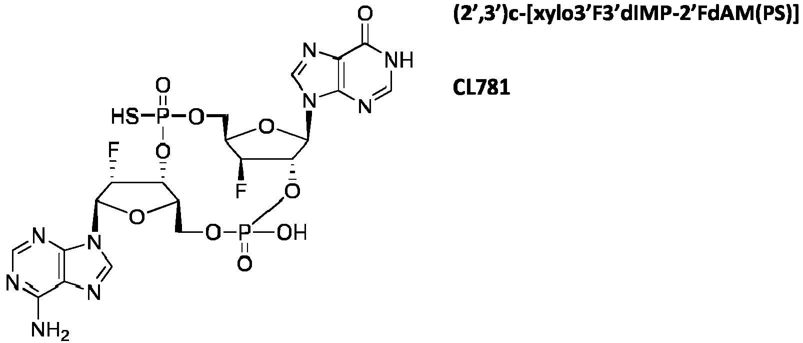

**Example 1.24** (24 mg, 11% yield) was prepared from **Intermediate 55** using a similar procedure to that described in general protocol method A. LC-MS: Rt = 2.38, 2.52 min, m/z = 680 [M+H]⁺, m/z = 679 [M-H]⁻. ¹H NMR (D₂O, 300 MHz) δ (ppm) 7.92-8.35 (m, 4H), 6.48 (m, 2H), 5.50-6.28 (m, 2H), 5.05-6.21 (m, 3H), 4.00-4.62 (m, 5H).

### Example 1.25:

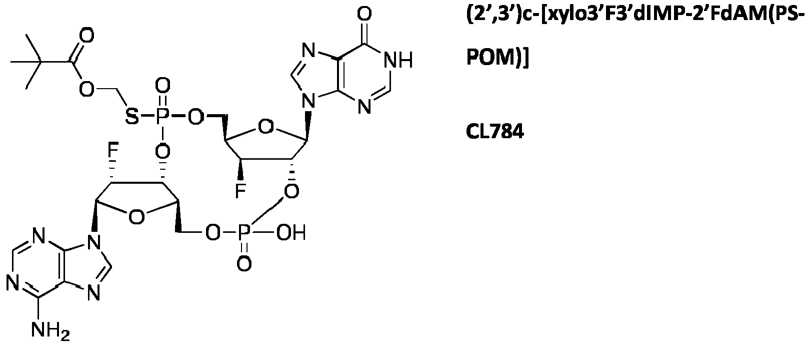

**Example 1.25** (24 mg, 11% yield) was prepared from Example 1.24 using a similar procedure to that described in general protocol for the POM-introduction on phosphorothioate moiety. LC-MS: Rt = 3.86, 3.93 min, m/z = 794 [M+H]⁺, m/z = 792 [M-H]⁻. ¹H NMR (D₂O, 300 MHz) δ (ppm) 8.01-8.52 (m, 4H), 6.44 (m, 2H), 6.15 (m, 2H), 5.43-5.91 (m, 4H), 3.84-4.93 (m, 6H), 1.14 (s, 9H).

### Example 1.26:

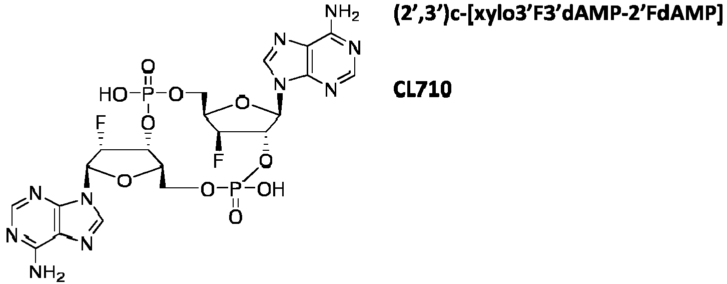

**Example 1.26** (9 mg, 7% yield) was prepared from **Intermediate 58** (175 mg, 0.18 mmol) using a similar procedure to that described in general protocol method A. Rt = 2.74 min, m/z = 664 [M+H]⁺, m/z = 662 [M-H]⁻. ¹H NMR (D₂O, 300 MHz) δ (ppm) 8.20 (m, 4H), 6.38 (m, 2H), 5.69 (m, 2H), 5.05 (m, 4H), 4.43 (m, 4H).

### Example 1.27.

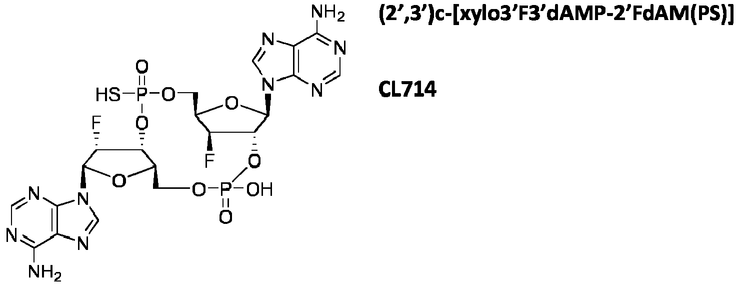

**Example 1.27** (12 mg, 3% yield) of was prepared from **Intermediate 61** (500 mg, 0.50 mmol) using a similar procedure to that described in general protocol method A. Rt = 2.73 min, m/z = 679 [M+H]⁺, m/z = 6772 [M-H]⁻. ¹H NMR (D₂O, 300 MHz) δ (ppm) 8.18 (m, 4H), 6.37 (m, 2H), 5.71 (m, 2H), 4.90 (m, 4H), 4.28 (m, 4H).

### Example 1.28:

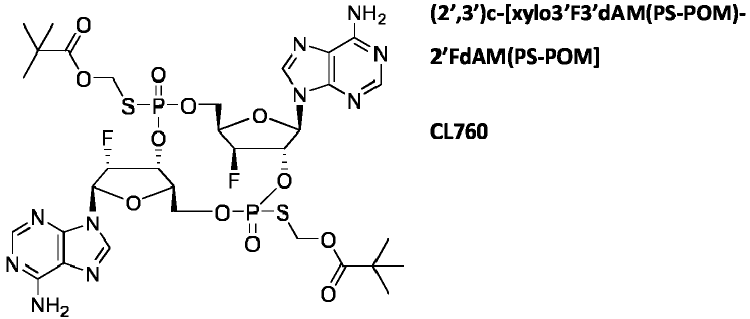

**Example 1.28** (7 mg, 5% yield) was prepared from **Intermediate 69** (264 mg, 0.25 mmol) which was treated with a solution of HCl 4M in dioxane (1.2 mL, 5.02 mmol) for 18h. The solvents were removed *in vacuo* and the residue was precipitated with acetone. Then the precipitate was treated with a solution of methylamine in EtOH (33%), and the resulting mixture was stirred at 50 °C for 4 h. The reaction mixture was concentrated, the crude was subjected to a similar procedure to that described for the **Example 1.2.** LC-MS: Rt = 4.38 and 4.41 min, m/z = 923 [M+H]⁺, m/z = 921 [M-H]⁻. ¹H NMR (D₂O, 300 MHz) δ (ppm) 7.89-8.36 (m, 4H), 5.4-6.48 (m, 6H), 5.36-5.20 (m, 4H), 3.89-4.60 (m, 6H), 1.11, 1.05 (2xs, 18H).

### Example 1.29:

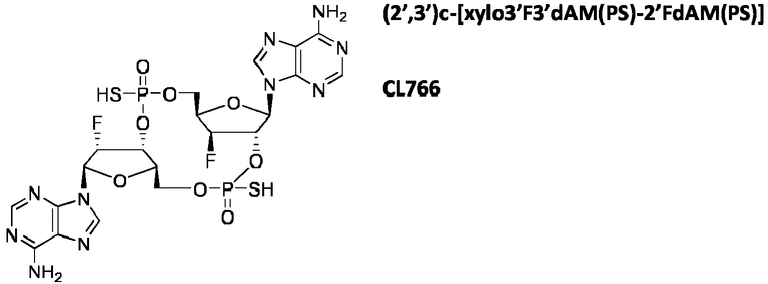

**Example 1.29** (24 mg, 11% yield) was prepared from **Intermediate 73** using a similar procedure to that described in general protocol method A. LC-MS: Rt = 2.80 min, m/z = 695 [M+H]⁺, m/z = 693 [M-H]⁻. ¹H NMR (D₂O, 300 MHz) δ (ppm) 8.13-8.34 (m, 4H), 6.37 (m, 2H), 5.59-5.88 (m, 2H), 4.16-5.19 (m, 8H).

### Example 1.30:

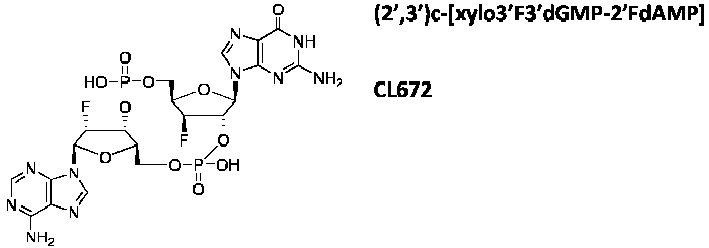

**Example 1.30** (36 mg, 27% yield) was prepared from **Intermediate 78** (0.19 g, 0.21 mmol) using a similar procedure to that described in general protocol method A. LC-MS: Rt = 3.06 min, m/z = 679 [M+H]⁺, m/z = 677 [M-H]⁻. ¹H NMR (D₂O, 300 MHz) δ (ppm) 8.26-7.99 (m, 4H), 6.22 (m, 2H), 5.49-5.00 (m, 2H), 4.13-3.73 (m, 5H), 3.54 (m, 2H).

### Example 1.31:

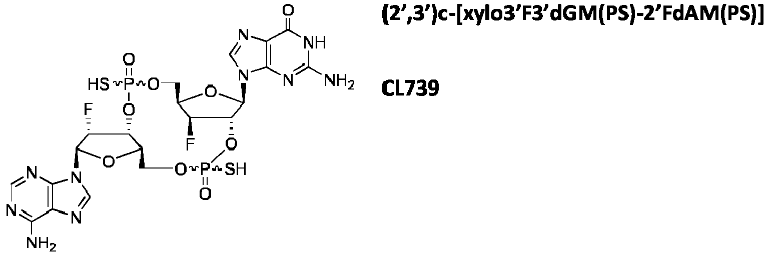

**Example 1.31** (175 mg, 77% yield) was prepared from **Intermediate 84** (0.30 g, 0.32 mmol) using a similar procedure to that described in general protocol method A. LC-MS: Rt = 2.57, 2.73 and 2.93 min, m/z = 679 [M+H]⁺, m/z = 677 [M-H]⁻. ¹H NMR (D₂O, 300 MHz) δ (ppm) 8.55-8.21 (m, 4H), 6.47-6.12 (m, 2H), 5.80-5.68 (m, 2H), 4.50-4.02 (m, 7H).

### Example 1.32:

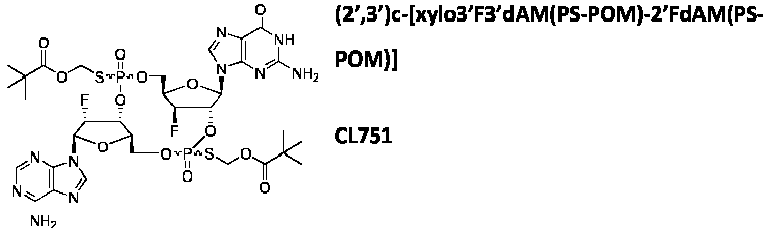

Example 1.32 (175 mg, 77% yield) was prepared from Example 1.31 (0.032 g, 0.073 mmol) using a similar procedure to that described in general protocol for the POM introduction on phosphorothioate moiety. LC-MS: Rt = 4.57 and 4.76 min, m/z = 939 [M+H]⁺, m/z = 937 [M-H]⁻. ¹H NMR (D₂O, 300 MHz) δ (ppm) 8.70-7.94 (m, 4H), 6.71-6.51 (m, 2H), 6.46-6.03 (s, 2H), 5.99-5.46 (s, 2H), 4.63-4.50 (m, 4H), 4.19-4.02 (m, 2H), 1.03 (s, 9H).

### Example 1.33:

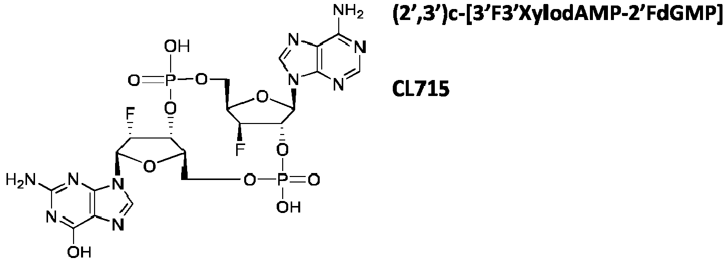

**Example 1.33** (20 mg, 50% yield) was prepared from **intermediate 87** (55 mg, 0.06 mmol) using a similar procedure to that described in general protocol method A protocol. LC-MS: Rt = 2.77 and 2.92 min, m/z = 979 [M+H]⁺, m/z = 977 [M-H]⁻. ¹H NMR (D₂O, 300 MHz) δ (ppm) 8.26-7.81 (m, 3H), 6.34 (m, 1H), 5.72 (m, 1H), 5.55 (m, 1H), 5.11-4.79 (m, 2H), 4.53-4.05 (m, 4H).

### EXAMPLE 2: Biological assays for c-diIMP analogs for their ability to activate STING-dependent cytokine induction in vitro, ex vivo on whole blood assay and to activate STING-dependent autophagy induction

In the present invention, the immunomodulatory activity of the c-diIMP analogs was ascertained in *in vitro*-cell based assay and in live human cells. These compounds induced the production of multiple cytokines specifically the production of Type I interferons and/or pro-inflammatory cytokines, as indirectly determined by an ISG54 (interferon-stimulated gene) reporter assay (Fensterl *et al.,* 2008).

The *in vitro* cytokine-induction activity of a representative set of these cyclic dinucleotides is reported here to require the presence of the eukaryotic cellular receptor known as "stimulator of interferon genes" (STING).

These experiments were performed as described below.

### Example 2.1: Evaluation of c-diIMP analogs for their ability to activate STING-dependent cytokine induction in vitro in a human reporter cell line

- **Cytokine reporter cell line used:** THP-1-Dual™ and THPl-Dual™ KO-STING
- **Compounds tested:** CL735, CL744, CL733, CL734, CL736, CL737, CL742, CL752 CL746, CL747, CL752 and CL753.
- **Reference compounds:** 2'3'-cGAMP (InvivoGen catalog code: tlrl-nacga23-5), CL626 and c-diIMP (InvivoGen catalog code: tlrl-nacdi)
- **Cytokines evaluated:** IFN-α/β

The *in vitro* STING agonist activity disclosed in the present invention has been measured by monitoring of the IRF pathway. The IRF pathway has been investigated by using the two following ISG reporter cell lines obtained directly from InvivoGen. They are described here and provided with their corresponding InvivoGen catalog code.

**THP-1-Dual™** (InvivoGen catalog code: thpd-nfis): These cells were derived from the human monocytic cell line THP-1 by stable integration of two inducible reporter constructs. They enable simultaneous study of two signaling pathways: the NF-κB pathway, by monitoring the activity of secreted embryonic alkaline phosphatase (SEAP); and the IRF pathway, by assessing the activity of a secreted luciferase (Lucia).

**THP-1-Dual™-KO-STING (InvivoGen catalog code: thpd-kostg):** These cells were generated from the human monocyte THP-1-Dual™, through stable homozygote knockout of the STING gene. Bialielic STING knockout was verified by functional assays, PCR and DNA sequencing.

Both reporter proteins can be readily measured in the cell culture supernatant by using QUANTI-Blue™ (InvivoGen catalog code: rep-qb1), a SEAP detection reagent that turns purple/blue in the presence of SEAP (quantified by measuring the optical density from 620 nm to 655 nm), and QUANTI-Luc™ (InvivoGen; catalog code: rep-qlc1), a luminometric enzyme assay that measures luciferase expression to report on ISG54 expression (as an indicator of IFN-α/β production).

To each well of a flat-bottom 96-well plate were added 20 µL of a solution of the tested CDNs (30 µg/ml in saline solution), followed by 180 µL of a suspension of a single cell line (THP-1-Dual™: ca. 100,000 cells per well). The plate was incubated for 18 h to 24 h at 37 °C in 5% CO2. The level of IFN-α/β in each well was indirectly quantified using QUANTI-Luc™ (as an indicator of IFN-β production), which was prepared and used according to the manufacturer's instructions (InvivoGen).

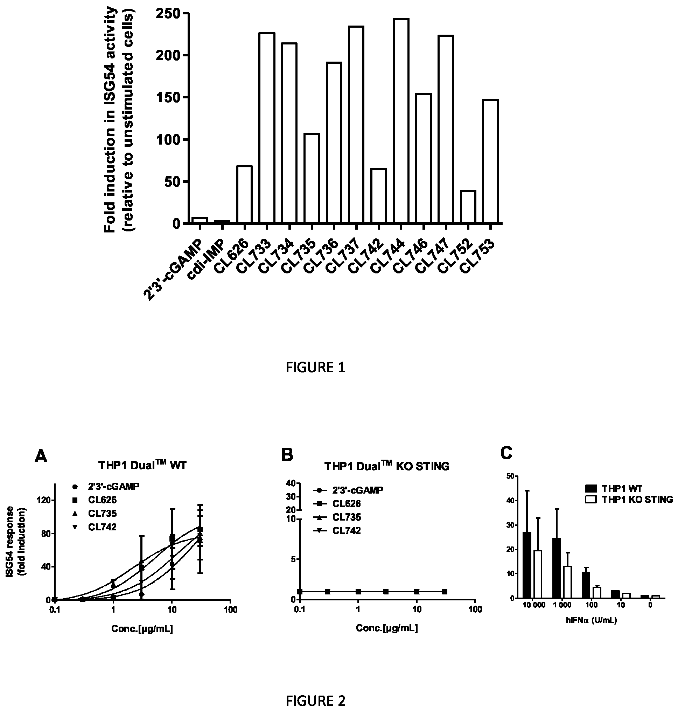

The results from this experiment are shown below in **Figure 1 and Figure 2.** Figure 1 illustrates the fold induction for each compound tested at 3 µg/mL relative to untreated cells and compared to the reference compound (2'3'-cGAMP and non-fluorinated c-diIMP) when used at the same concentration. The histogram shows that each one of the tested CDNs induces type I IFNs in THP-1-Dual cells to a greater extent than does 2'3'-cGAMP, the endogenous STING agonist in mammals. At the opposite, as shown in Figures 2A and 2B, these c-diIMP derivatives illustrated by the compounds CL735 and CL742 did not induced production of Type I interferons in the THP1-Dual KO-STING cells. Note that both THP1 WT and THP1 KO-STING cells are able to respond to human IFNα in a dose dependent manner as shown in Figure 2C. This finding demonstrated that STING is required for the cytokine-induction activity of these c-diIMP derivative compounds *in vitro* in cells.

### Example 2.2: Evaluation of c-diIMP analogs for their ability to induce cytokines ex vivo in whole blood from healthy human donors

- **Compounds tested:** CL735; CL744; CL733; CL734; CL736; CL737; CL742; CL752; CL746; CL747; CL752; CL753; CL785; CL786 and CL787
- **Reference compound:** 2'3'-cGAMP (InvivoGen catalog code: tlrl-nacga23-5) and CL626
- **Cytokines evaluated:** Type I IFNs (using HEK-Blue™ IFN-α/β cells KO STING) and Type III IFNs (using HEK-Blue™ IL-28 cells)

### Reporter cell lines:

**Type I IFNs:** HEK-Blue™ IFN-α/β-KO-STING: These cells, in which the STING gene has been inactivated, are derived from HEK293 cell line known as HEK-Blue™ IFN-α/β (InvivoGen catalog code: hkb-ifnab). HEK-Blue™ IFN-α/β cells enable detection of bioactive human type I IFNs through monitoring of activation of the ISG3 pathway. These cells were generated by stable transfection of HEK293 cells with the human STAT2 and IRF9 genes to obtain a fully active Type-I IFN signaling pathway. The other genes of the pathway (IFNAR1, IFNAR2, JAK1, TyK2 and STAT1) are naturally expressed in sufficient amounts. The cells were further transfected with a SEAP reporter gene under control of an IFN-α/β-inducible ISG54 promoter. This promoter comprises five IFN-stimulated response elements (ISREs) fused to a minimal promoter of the human ISG54 gene, which is unresponsive to activators of the NF-κB or AP-1 pathways. Stimulation of HEK-Blue™ IFN-α/β cells with human IFN-α or IFN-β activates the JAK/STAT/ISGF3 pathway and subsequently induces production of SEAP. Production of type I IFNs in these cells is measured using QUANTI-Blue™.

**Type III IFNs:** HEK-Blue™ IL-28: This HEK293 cell line is derived from HEK-Blue™ ISG cells (Invivogen catalog code: hkb-isg). It enables detection of bioactive Type III IFNs (IL-28A [IFN-λ2], IL-28B [IFN-λ3] and IL-29 [IFN-λ1]) through monitoring of activation of the ISG54 pathway. HEK-Blue™ IL-28 cells were generated by inactivation of the IFNAR2 and IFNGR1 genes, to abolish (i.e. reduce to undetectable levels) the Type I and Type II IFN response, followed by stable transfection with the human IFNLR1 and IL10R genes, to obtain a strong Type III IFN response. The other genes of the (shared Type I/Type III) IFN pathway (IFNAR1, JAK1, TyK2 and STAT1) are naturally expressed in sufficient amounts. The resultant cells were then transfected with a SEAP reporter gene under control of a promoter that comprises five IFN-stimulated response elements (ISREs) fused to a minimal promoter of the human ISG54 gene, which is unresponsive to activators of the NF-κB or AP-1 pathways. Stimulation of HEK-Blue™ IL-28 cells with Type III IFNs activates the JAK/STAT/ISGF3 pathway and subsequently induces production of SEAP, which is measured using QUANTI-Blue™.

### Acquisition and handling of human blood samples

Human blood samples were acquired from healthy donors at the Etablissement Français du Sang (EFS Pyrénées Méditerranée, Toulouse, France; per agreement #21/PLER/TOU/2016--0082). Briefly, the samples were collected by venipuncture into sodium heparin tubes at the time of donation. The samples were analyzed for rhesus (Rh), blood group, hematocrit and serological status (AgHBS, HIV, HCV, HTLV, HBC, CMV, Syph). The tubes were picked up on the day of collection and subsequently tested (blood analysis, and treatment with test items) on the same day.

### Treatment of human blood samples

Each blood sample was diluted (1:2 [v/v]) in RPMI medium and aliquoted into 96-well plates (180-µL wells) containing a CDN. The plates were incubated at 37 °C in a CO₂ incubator for 18 hours. Then, the supernatants were collected, transferred into the corresponding wells of round-bottom 96-well plates, and either stored at -80 °C, or immediately tested in the appropriate reporter cell line.

### Testing of human blood samples

A new 96-well plate was prepared for each reporter cell lines tested, as follows: 10 µL of supernatant from the previous plate (containing the incubated CDNs and plasma) were added to the corresponding well in the new reporter cell plate. Then, a 190-µL aliquot of cells of the desired reporter cell line, previously harvested in medium containing heat-inactivated serum and counted, was added to each well (approximately 50,000 cells/well), and the plate was incubated for approximately 20 hours. The desired cytokine induction activity was determined using the QUANTI-Blue™ assay, as previously described. Briefly, 20 µL of supernatant from the previously incubated plate was transferred to the corresponding well of a new 96-well plate in which 180 µL of QUANTI-Blue™ reagent had previously been added.

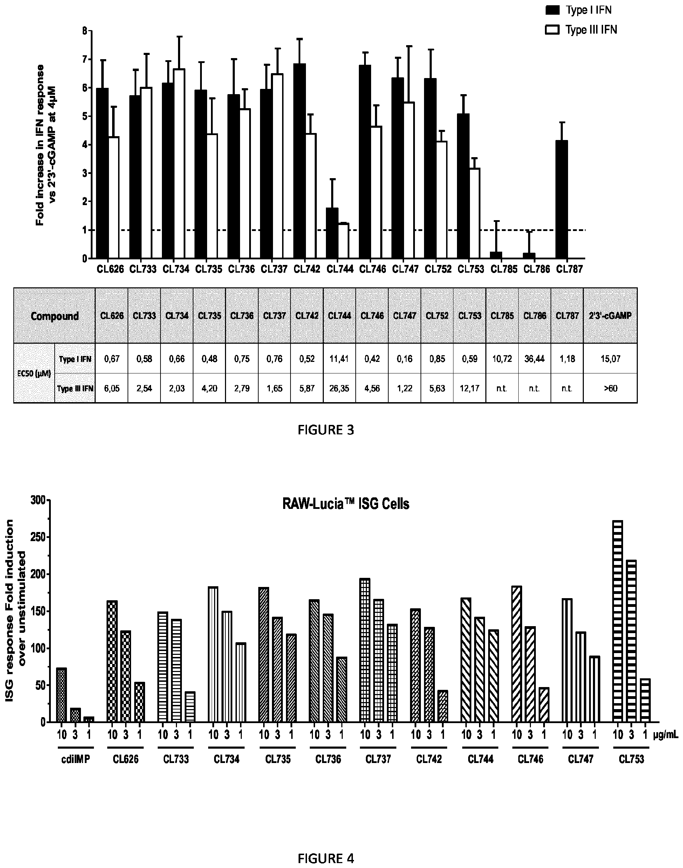

Figure 3 summarizes the fold induction of type I IFNs (black) or type III interferons (white) for each compound relative to the response for the reference compound, 2'3'-cGAMP (tested at the same concentration; ≈ 4 µM) in the whole-blood assay, as well as the corresponding EC50 values (expressed in µM) for each activity and each compound in this assay. The CDNs of the present invention provide equivalent (CL744) or superior induction of type I and type III interferons compared to the reference compound 2'3'-cGAMP. Those CDNs with at least one phosphorothioate linkage (CL734, CL735, CL736 CL737, CL746 and CL747) tend to induce type III IFN response more strongly than do those with only phosphodiester linkages (CL733, CL742, CL752 and CL753). Interestingly, CL747 (which has three POM groups) had lower EC50 values (i.e. higher potency) for both activities than did CL746 (which has only one POM group), suggesting that conversion of a given CDN into its corresponding POM prodrug improves both its type I and its type III interferon induction activities.

### Example 2.3: Evaluation of c-diIMP analogs for their ability to activate STING-dependent cytokine induction in vitro in a murine reporter cell line

- **Cytokine reporter cell line used:** RAW-lucia™ ISG cells (InvivoGen catalog code: rawl-isg)
- **Compounds tested:** CL735; CL744; CL733; CL734; CL736; CL737; CL742; CL746; CL747 and CL753.
- **Reference compound:** c-diIMP (InvivoGen catalog code: tlrl-nacdi), CL626

The ability of the CDNs used to activate STING-dependent induction of the IRF pathway was measured using the murine ISG reporter cell line obtained directly from InvivoGen.

**RAW-lucia™ ISG cells (InvivoGen catalog code: rawl-isg):** These cells were generated from the murine RAW 264.7 macrophage cell line by stable integration of an interferon regulatory factor (IRF)-inducible Lucia luciferase reporter construct. RAW-Lucia™ ISG cells express the Lucia luciferase gene under the control of an ISG54 minimal promoter in conjunction with five IFN-stimulated response elements. Thus, RAW-Lucia™ ISG cells allow the monitoring of IRF activation by determining the activity of secreted luciferase. The levels of IRF-induced luciferase in the cell culture supernatant can be easily monitored using QUANTI-Luc™ (InvivoGen; catalog code: rep-qlc1).

To each well of a flat-bottom 96-well plate were added 20 µL of a solution of each tested CDN (10 µg/mL, 3µg/mL or 1 µg/mL in saline solution), followed by 180 µL of a suspension of a single cell line (RAW-lucia™ ISG : ca. 100,000 cells per well). The plate was incubated for 18 h to 24 h at 37 °C in 5% CO2. The activation of ISG pathway in each well was indirectly quantified using QUANTI-Luc™ which was prepared and used according to the manufacturer's instructions (InvivoGen).

The results from this experiment are shown above in Figure 4, which illustrates the fold induction for each compound relative to untreated cells and compared to the reference compound (non-fluorinated c-diIMP) when used at the same concentration. The histogram shows that murine cell line can mount an ISG response and that each one of the tested CDNs induces type I IFNs in RAW cells to a greater extent than does c-diIMP.

### Example 2.4: Evaluation of c-diIMP analogs for their ability to induce autophagy in vitro in a murine reporter cell line

- Compound tested: CL735; CL744; CL733; CL734; CL736; CL737; CL742; CL746; CL747 and CL753
- Reference compounds: Rapamycin (InvivoGen catalog code: tlrl-rap) c-diIMP (InvivoGen catalog code: tlrl-nacdi) and CL626
- Reporter cell line used: RAW-Difluo™ mLC3 Cells (InvivoGen catalog code: rawdf-mlc3)

The ability of the CDNs of the present invention, to activate STING-dependent autophagy induction was measured using the murine autophagy reporter cell line obtained directly from InvivoGen.

### Reporter cell line

RAW-Difluo™ mLC3 Cells are autophagy reporter cells derived from the murine RAW 264.7 macrophage cell line, a physiologically relevant cell line. They express an RFP::GFP::LC3 fusion protein, RFP-GFP-LC3, in which the N-terminus of murine LC3 is fused to two fluorescent reporter proteins: a RFP (acid-stable) and a GFP (acid-sensitive). In these cells, the RFP-GFP pair enables monitoring of autophagic flux in real time by detecting the appearance of green (GFP-LC3) and/or red (RFP-LC3) puncta by fluorescence microscopy.

### Treatment of the reporter cells

Approximately 50,000 cells (500 µL) per well of 24-well plates were seeded 24 h before treatment. The next day, the cells were treated either with a solution containing a CDN at one of three different concentrations (1 µg/mL, 3 µg/mL or 10 µg/mL) or with Rapamycin at 25 µM, or were left untreated. The plates were incubated at 37 °C in a CO2 incubator for 8 hours. Fluorescence intensity and puncta formation was monitored at λ_{exc} = 480 nm and λₑₘ = 505 nm. Autophagy induction was quantified according to the percentage of cells exhibiting puncta formation.

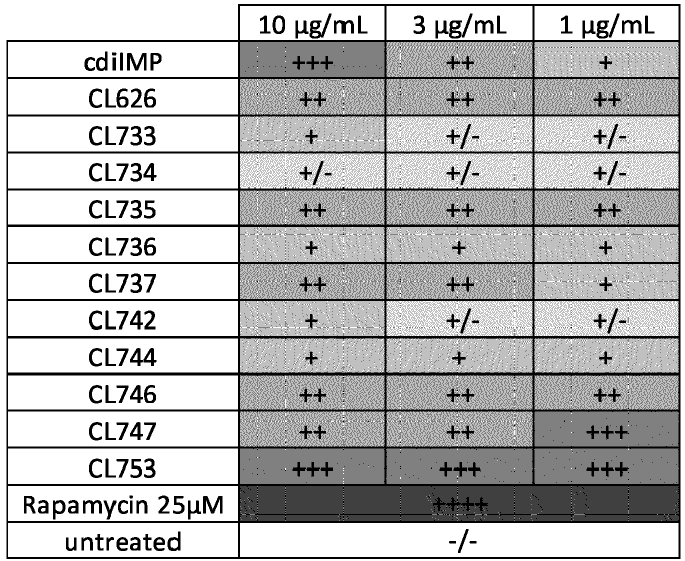

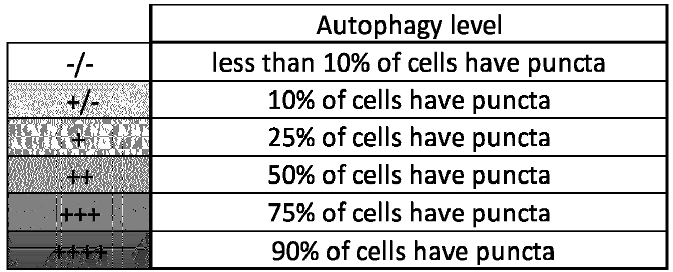

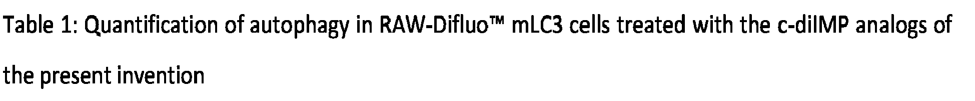

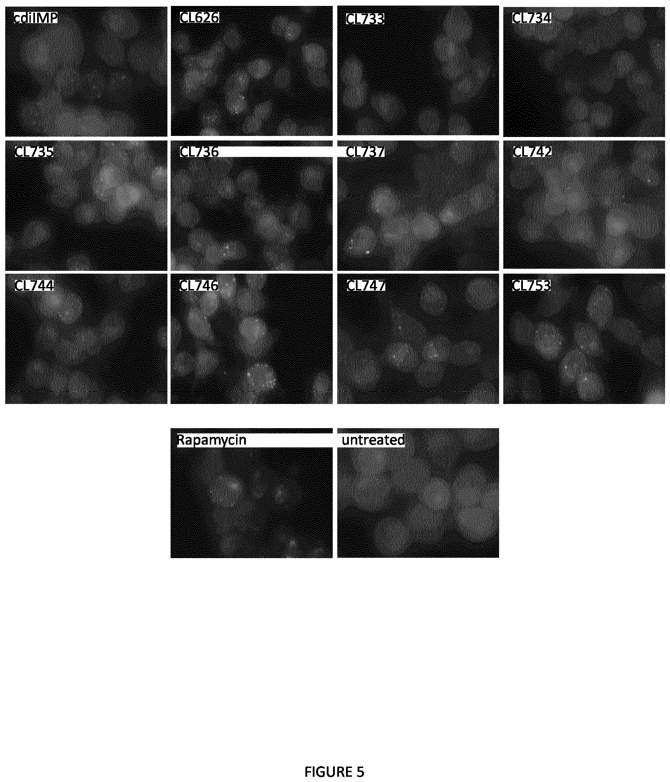

The results from this experiment are presented in Table 1 and representative images of cells separately treated with each compound at 1 µg/mL for 8 h are shown in Figure 5. Each one of the tested CDNs can induce formation of autophagic vesicles in RAW-Difluo reporter cells (Table 1 and Fig. 5). The ISG54 activity induction shown in Example 2.3. does not correlate with the level of autophagy found in this example. For example, CL734 and CL753 both induce ISG54 activity more strongly than does c-diIMP; however, CL734 induces autophagy to a lesser extent than c-diIMP whereas CL753 does so to a greater extent. Indeed, structure-activity relationship (SAR) analysis did not reveal any correlation between the linkage position (2'3' vs. 3'3'), the presence of fluorine atoms on the sugar, internucleotide linkage type (PS vs. PO: compare CL626 and CL735, respectively), sugar type (ribose, deoxyribose or xylose) or prodrug modification (without POM, mono-POM or bis-POM: compare CL735, CL736 and CL737, respectively) on one hand, and autophagy induction activity, on the other hand.

### Example 2.5: Evaluation of c-diIMP analogs for their ability to induce PD-L1 expression in vitro in a human cell line

- **Compound tested:** CL735; CL735; CL736; CL737; CL742; CL746 and CL747
- **Reference compounds:** 2'3'-cGAMP (InvivoGen catalog code: tlrl-nacga23-5) and CL626
- **Cell line used:** THP-1 WT Cells

### Protocol

Approximately 100,000 THP-1 WT cells (180 µL) per well of 96-well plates were either treated with a solution containing a CDN at one of four different concentrations (30 µg/mL; 10 µg/mL, 3 µg/mL or 1 µg/mL), or left untreated. The cells were incubated at 37 °C in a CO2 incubator for 18 hours. **PD-L1** expression was quantified on a FACSCanto™ (BD Biosciences) flow cytometer. Briefly, washed cells were incubated with human FcBlock reagent (Miltenyi Biotec) for 10 min at 4 °C, centrifuged and resuspended in PBS-BSA containing either PD-L1-APC (Biolegend, clone 29E.2A3, dilution 1/200) or its isotype control (APC Mouse IgG2b, κ Isotype Ctrl, Biolegend, clone MPC-11, dilution 1/200) for 30 min at 4 °C.

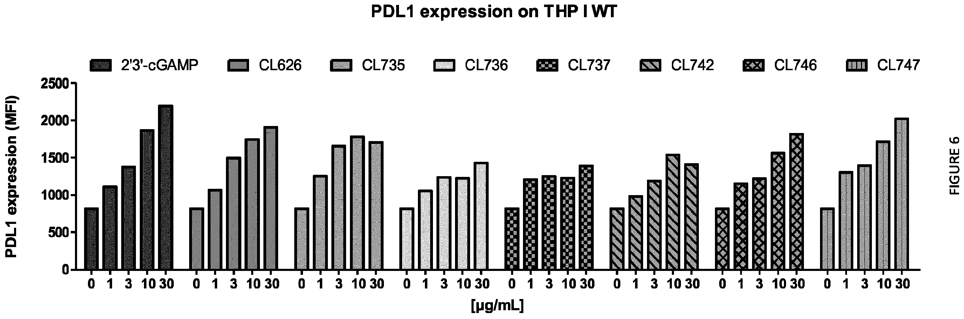

The results presented in Figure 6 reveal several findings. Firstly, each one of the tested CDNs can induce PD-L1 expression on THP-1 cells in a dose-dependent manner. Secondly, there does not seem to be any correlation between CDN structure and PD-L1 expression, as SAR analysis did not find any correlation between the linkage position (2'3' vs. 3'3'), fluorinated CDNs, internucleotide linkage type (PS vs. PO), sugar type (ribose, deoxyribose or xylose) or prodrug modification (without POM, mono-POM or bis-POM) on one hand, and PD-L1 expression induction activity, on the other hand. Lastly, compounds CL736, CL737 or CL742 might be especially interesting for use in immuno-oncology applications. Two of those CDNs are bis-fluorinated, contain two phosphorothioate linkages and at least one POM group (CL736: mono-POM; CL737: bis-POM), induce less PD-L1 expression than does the reference compound 2'3'-cGAMP.

### EXAMPLE 3: Biological assays for c-AIMP analogs for their ability to activate STING-dependent cytokine induction in vitro in a human reporter cell line and ex vivo on whole blood assay

In the present invention, the immunomodulatory activity of these c-AIMP analogs was ascertained in *in vitro*-cell based assay and in live human cells. The *in vitro* cytokine-induction activity of a representative set of these cyclic dinucleotides is reported here to require the presence of the eukaryotic cellular receptor known as "stimulator of interferon genes" (STING) using the methodology described in Example 2.1. The ex vivo (whole blood assay) cytokine-induction activity of a representative set these cyclic dinucleotides is reported here with the same protocol described in Example 2.2.

These experiments were performed as described below.
- **Compounds tested:** CL674, CL702, CL767, CL791, CL782, CL758, CL761, CL762, CL763, CL731, CL764, CL765, CL777, CL779, CL780, CL781 and CL784
- **Reference compounds:** 2'3'-cGAMP (InvivoGen catalog code: tlrl-nacga23-5)
- ***in vitro* read-out:** ISG and NFκB pathways (using THP1 Dual™ cells InvivoGen catalog code: thpd-nfis)
- ***ex vivo* read-out:** Type I IFNs (using HEK-Blue™ IFN-α/β KO STING cells), IL1 (using HEK- Blue™ IL1 cells InvivoGen catalog code: hkb-il1r)

### Reporter cell lines:

Type I **IFNs:** HEK-Blue™ IFN-α/β-KO-STING: These cells, in which the STING gene has been inactivated, are derived from HEK293 cell line known as HEK-Blue™ IFN-α/β (InvivoGen catalog code: hkb-ifnab). HEK-Blue™ IFN-α/β cells enable detection of bioactive human type I IFNs through monitoring of activation of the ISG3 pathway. These cells were generated by stable transfection of HEK293 cells with the human STAT2 and IRF9 genes to obtain a fully active Type-I IFN signaling pathway. The other genes of the pathway (IFNAR1, IFNAR2, JAK1, TyK2 and STAT1) are naturally expressed in sufficient amounts. The cells were further transfected with a SEAP reporter gene under control of an IFN-α/β-inducible ISG54 promoter. This promoter comprises five IFN-stimulated response elements (ISREs) fused to a minimal promoter of the human ISG54 gene, which is unresponsive to activators of the NF- 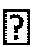 B or AP-1 pathways. Stimulation of HEK-Blue™ IFN-α/β cells with human IFN-α or IFN-β activates the JAK/STAT/ISGF3 pathway and subsequently induces production of SEAP. Production of type I IFNs in these cells is measured using QUANTI-Blue™.

IL1: HEK-Blue™ IL-1R (InvivoGen catalog code: hkb-il1r): The HEK293 cell line known as HEK-Blue™ IL-1R was designed to detect bioactive human and murine IL-1 through monitoring of activation of the NF-κB and AP-1 pathways. Additionally, these cells detect bioactive IL-1 from cynomolgus monkeys, dogs, hamsters and rats. In fact, HEK-Blue™ IL-1R cells can detect IL-1α and IL-1β, as these cytokines bind to the same receptor, IL-1R. These cells derive from HEK-Blue™ IL-1β cells (InvivoGen catalog code: hkb-il1b), in which the TNF-α response is blocked. Therefore, HEK-Blue™ IL-1R cells respond specifically to IL-1. These cells endogenously express the human IL-1 receptor and were stably transfected with the murine IL-1 receptor, rendering them sensitive to both human and murine IL-1β. HEK-Blue™ IL-1R cells express a SEAP reporter gene under control of an IFN-β minimal promoter fused to five NF-κB and five AP-1 binding sites. Binding of IL-1β to IL-1R on the surface of HEK-Blue™ IL-1R cells triggers a signaling cascade that leads to the activation of NF-κB and subsequent production of SEAP. Production of IL-1β in these cells is measured using QUANTI Blue™.

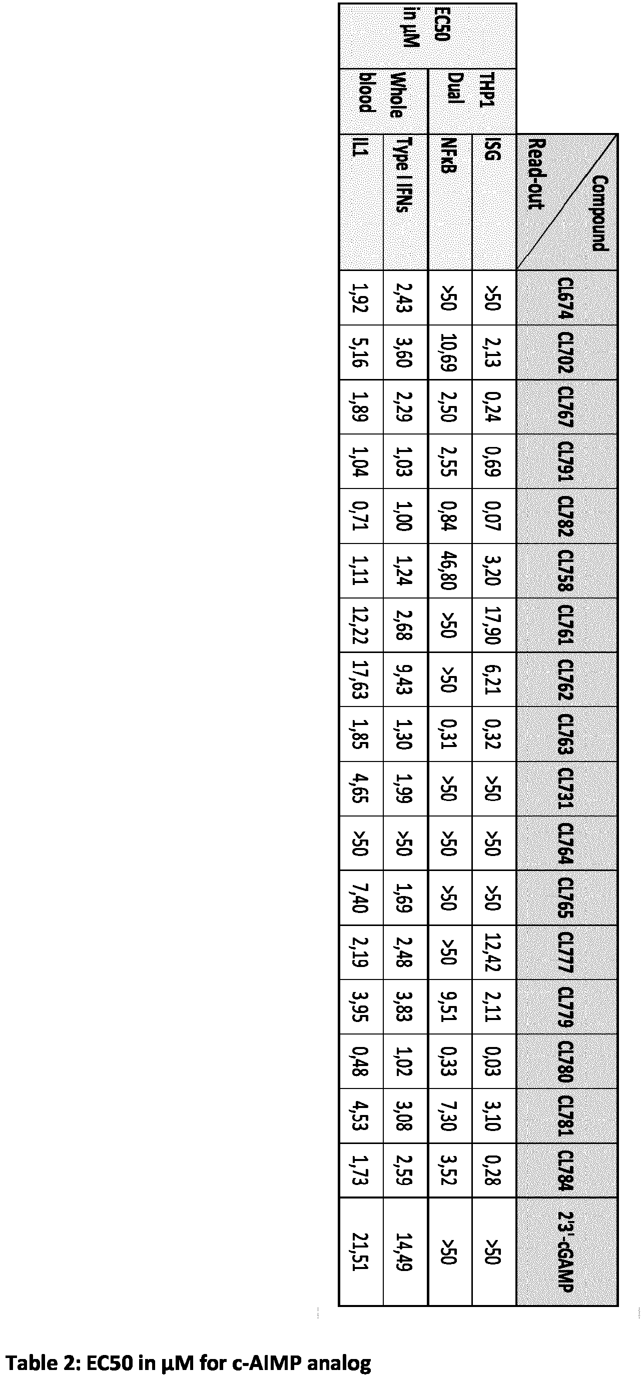

The results presented in Table 2 reveal several findings. Firstly, monophosphorothioate compounds displayed a better activity than diphosphorothioate (e.g. CL781 >> CL764). Then while enzyme-labile group modifications improved activity/stability when it is on the phospho-linkage (e.g. CL767>CL702 or CL791>CL702 or CL784 > CL781), they don't ameliorate compound potency when modifications are on the base (e.g. CL761 < CL760 or CL762 < CL760. Lastly, Xylose modification (e.g. CL781 or CL779) chemistry allows to keep a 2'5'-3'5' linkage close to the natural and endogenous STING ligand (2'3' c-GAMP) and maintain a strong activity.

### EXAMPLE 4: Biological assays for c-diAMP analogs for their ability to activate STING-dependent cytokine induction in vitro in a human reporter cell line and ex vivo on whole blood assay

In the present invention, the immunomodulatory activity of these c-AIMP analogs was ascertained in *in vitro*-cell based assay and in live human cells. The *in vitro* cytokine-induction activity of a representative set of these cyclic dinucleotides is reported here to require the presence of the eukaryotic cellular receptor known as "stimulator of interferon genes" (STING) using the methodology described in Example 2.1. The ex vivo (whole blood assay) cytokine-induction activity of a representative set these cyclic dinucleotides is reported here with the same protocol described in Example 2.2. These experiments were performed as described below.
- **Compounds tested:** CL766, CL714, CL713, CL710, CL709, CL685 and CL760
- **Reference compounds:** 2'3'-cGAMP (InvivoGen catalog code: tlrl-nacga23-5)
- ***in vitro* read-out:** ISG and NFκB pathways (using THP1 Dual™ cells InvivoGen catalog code: thpd-nfis)
- ***ex vivo* read-out:** Type I IFNs (using HEK-Blue™ IFN-α/β KO STING cells), IL1 (using HEK- Blue™ IL1 cells InvivoGen catalog code: hkb-il1r)

### Reporter cell lines:

Type I IFNs: HEK-Blue™ IFN-α/β-KO-STING: These cells, in which the STING gene has been inactivated, are derived from HEK293 cell line known as HEK-Blue™ IFN-α/β (InvivoGen catalog code: hkb-ifnab). HEK-Blue™ IFN-α/β cells enable detection of bioactive human type I IFNs through monitoring of activation of the ISG3 pathway. These cells were generated by stable transfection of HEK293 cells with the human STAT2 and IRF9 genes to obtain a fully active Type-I IFN signaling pathway. The other genes of the pathway (IFNAR1, IFNAR2, JAK1, TyK2 and STAT1) are naturally expressed in sufficient amounts. The cells were further transfected with a SEAP reporter gene under control of an IFN-α/β-inducible ISG54 promoter. This promoter comprises five IFN-stimulated response elements (ISREs) fused to a minimal promoter of the human ISG54 gene, which is unresponsive to activators of the NF- 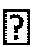 B or AP-1 pathways. Stimulation of HEK-Blue™ IFN-α/β cells with human IFN-α or IFN-β activates the JAK/STAT/ISGF3 pathway and subsequently induces production of SEAP. Production of type I IFNs in these cells is measured using QUANTI-Blue™.

IL1: HEK-Blue™ IL-1R (InvivoGen catalog code: hkb-il1r): The HEK293 cell line known as HEK-Blue™ IL-1R was designed to detect bioactive human and murine IL-1 through monitoring of activation of the NF-κB and AP-1 pathways. Additionally, these cells detect bioactive IL-1 from cynomolgus monkeys, dogs, hamsters and rats. In fact, HEK-Blue™ IL-1R cells can detect IL-1α and IL-1β, as these cytokines bind to the same receptor, IL-1R. These cells derive from HEK-Blue™ IL-1β cells (InvivoGen catalog code: hkb-il1b), in which the TNF-α response is blocked. Therefore, HEK-Blue™ IL-1R cells respond specifically to IL-1. These cells endogenously express the human IL-1 receptor and were stably transfected with the murine IL-1 receptor, rendering them sensitive to both human and murine IL-1β. HEK-Blue™ IL-1R cells express a SEAP reporter gene under control of an IFN-β minimal promoter fused to five NF-κB and five AP-1 binding sites. Binding of IL-1β to IL-1R on the surface of HEK-Blue™ IL-1R cells triggers a signaling cascade that leads to the activation of NF-κB and subsequent production of SEAP. Production of IL-1β in these cells is measured using QUANTI Blue™.

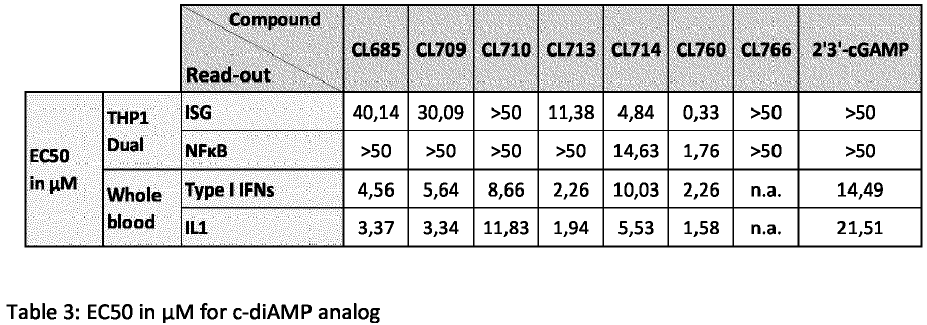

The results presented in Table 3 confirms some findings observed in Example 2 and 3. Firstly, monophosphorothioate compounds displayed a better activity than diphosphorothioate (e.g. CL714 > CL766). Lastly, Xylose modification (e.g. CL710, CL714, CL760 or CL766) chemistry allow to keep a 2'5'-3'5' linkage close to the natural and endogenous STING ligand (2'3' c-GAMP) and maintain a strong activity.

### EXAMPLE 5: Biological assays for c-GAMP analogs for their ability to activate STING-dependent cytokine induction in vitro in a human reporter cell line and ex vivo on whole blood assay

In the present invention, the immunomodulatory activity of these c-GAMP analogs was ascertained in *in vitro*-cell based assay and in live human cells. The *in vitro* cytokine-induction activity of a representative set of these cyclic dinucleotides is reported here to require the presence of the eukaryotic cellular receptor known as "stimulator of interferon genes" (STING) using the methodology described in Example 2.1. The *ex vivo* (whole blood assay) cytokine-induction activity of a representative set these cyclic dinucleotides is reported here with the same protocol described in Example 2.2. These experiments were performed as described below.
- **Compounds tested:** CL715, CL756, CL751, CL739, CL706 and CL672
- **Reference compounds:** 2'3'-cGAMP (InvivoGen catalog code: tlrl-nacga23-5)
- ***in vitro* read-out:** ISG and NFκB pathways (using THP1 Dual™ cells InvivoGen catalog code: thpd-nfis)
- ***ex vivo* read-out:** Type I IFNs (using HEK-Blue™ IFN-α/β KO STING), IL1 (using HEK-Blue™ IL1 cells InvivoGen catalog code: hkb-il1r)

### Reporter cell lines:

**Type I IFNs:** HEK-Blue™ IFN-α/β-KO-STING: These cells, in which the STING gene has been inactivated, are derived from HEK293 cell line known as HEK-Blue™ IFN-α/β (InvivoGen catalog code: hkb-ifnab). HEK-Blue™ IFN-α/β cells enable detection of bioactive human type I IFNs through monitoring of activation of the ISG3 pathway. These cells were generated by stable transfection of HEK293 cells with the human STAT2 and IRF9 genes to obtain a fully active Type-I IFN signaling pathway. The other genes of the pathway (IFNAR1, IFNAR2, JAK1, TyK2 and STAT1) are naturally expressed in sufficient amounts. The cells were further transfected with a SEAP reporter gene under control of an IFN-α/β-inducible ISG54 promoter. This promoter comprises five IFN-stimulated response elements (ISREs) fused to a minimal promoter of the human ISG54 gene, which is unresponsive to activators of the NF-κB or AP-1 pathways. Stimulation of HEK-Blue™ IFN-α/β cells with human IFN-α or IFN-β activates the JAK/STAT/ISGF3 pathway and subsequently induces production of SEAP. Production of type I IFNs in these cells is measured using QUANTI-Blue™.

IL1: HEK-Blue™ IL-1R (InvivoGen catalog code: hkb-il1r): The HEK293 cell line known as HEK-Blue™ IL-1R was designed to detect bioactive human and murine IL-1 through monitoring of activation of the NF-κB and AP-1 pathways. Additionally, these cells detect bioactive IL-1 from cynomolgus monkeys, dogs, hamsters and rats. In fact, HEK-Blue™ IL-1R cells can detect IL-1α and IL-1β, as these cytokines bind to the same receptor, IL-1R. These cells derive from HEK-Blue™ IL-1β cells (InvivoGen catalog code: hkb-il1b), in which the TNF-α response is blocked. Therefore, HEK-Blue™ IL-1R cells respond specifically to IL-1. These cells endogenously express the human IL-1 receptor and were stably transfected with the murine IL-1 receptor, rendering them sensitive to both human and murine IL-1β. HEK-Blue™ IL-1R cells express a SEAP reporter gene under control of an IFN-β minimal promoter fused to five NF-κB and five AP-1 binding sites. Binding of IL-1β to IL-1R on the surface of HEK-Blue™ IL-1R cells triggers a signaling cascade that leads to the activation of NF-κB and subsequent production of SEAP. Production of IL-1β in these cells is measured using QUANTI Blue™.

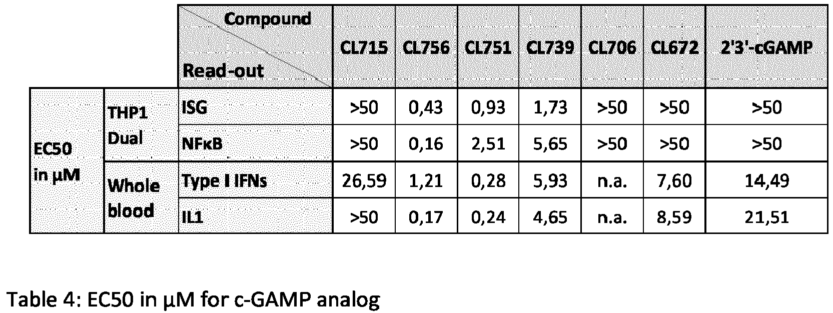

The results presented in Table 4 confirm some findings observed in Examples 2, 3 and 4. Particularly, Xylose modification (e.g. CL739, CL751, CL756) chemistry allow to keep a 2'5'-3'5' linkage close to the natural and endogenous STING ligand (2'3' c-GAMP) and maintain a strong activity.

### EXAMPLE 6: in vitro Penetration assay for CDNs prodrug analogs

In the present invention, the immunomodulatory activity of the CDNs analogs was ascertained in *in vitro-*cell based assay. These compounds induced the production of multiple cytokines specifically the production of Type I interferons and/or pro-inflammatory cytokines, as indirectly determined by an ISG54 (interferon-stimulated gene) reporter assay (Fensterl et al., 2008). In order to compare their ability to penetrate inside the cytosolic space, we redesigned the protocol described in examples 2.1, 3, 4 and 5 and measure the ability of CDNs from the present invention to activate STING pathway with a shorter induction period (18h instead of 48h). Thus compounds which penetrated better will activate stronger.

These experiments were performed as described below.
- **Cytokine reporter cell line used:** THP-1-Dual™
- **Prodrug Compounds tested:** CL733, CL760, CL780
- **Control compounds tested :** CL766, CL779
- **Reference compounds:** CL626, 2'3'-cGAMP (InvivoGen catalog code: tlrl-nacga23-5)
- **Cytokines evaluated:** IFN-α/β

The *in vitro* STING agonist activity disclosed in the present invention has been measured by monitoring of the IRF pathway. The IRF pathway has been investigated by using the two following ISG reporter cell lines obtained directly from InvivoGen. They are described here and provided with their corresponding InvivoGen catalog code.

THP-1-Dual™ (InvivoGen catalog code: thpd-nfis): These cells were derived from the human monocytic cell line THP-1 by stable integration of two inducible reporter constructs. They enable simultaneous study of two signaling pathways: the NF-κB pathway, by monitoring the activity of secreted embryonic alkaline phosphatase (SEAP); and the IRF pathway, by assessing the activity of a secreted luciferase (Lucia).

Both reporter proteins can be readily measured in the cell culture supernatant by using QUANTI-Blue™ (InvivoGen catalog code: rep-qb1), a SEAP detection reagent that turns purple/blue in the presence of SEAP (quantified by measuring the optical density from 620 nm to 655 nm), and QUANTI-Luc™ (InvivoGen; catalog code: rep-qlc1), a luminometric enzyme assay that measures luciferase expression to report on ISG54 expression (as an indicator of IFN-α/β production). To each well of a flat-bottom 96-well plate were added 20 µL of a solution of the tested CDNs (30 µg/mL in saline solution), followed by 180 µL of a suspension of a single cell line (THP-1-Dual™: ca. 100,000 cells per well). The plate was incubated for 18 h to 24 h at 37 °C in 5% CO2. The level of IFN-α/β in each well was indirectly quantified using QUANTI-Luc™ (as an indicator of IFN-β production), which was prepared and used according to the manufacturer's instructions (InvivoGen).

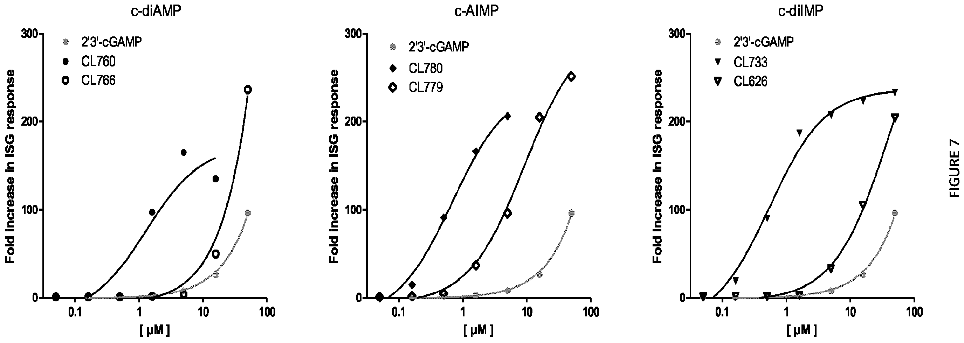

The results from this experiment are shown above in **Figure 7** which illustrates the fold induction for a prodrug derived compound from the c-diAMP, c-AIMP or c-diIMP families, relative to untreated cells and compared to the reference compound (2'3'-cGAMP) or the compound without the prodrug modification. Results show that each one of the tested CDNs induces type I IFNs in THP-1-Dual cells to a greater extent than does 2'3'-cGAMP, the endogenous STING agonist in mammals. Interestingly, POM-derivative compounds (CL760, CL780 and CL733) have a better ability to penetrate cells measured by a greater activation of ISG response than their unmodified counterpart (CL766, CL779 and CL626 respectively).

### EXAMPLE 7: Biological assays in vivo

### Example 7.1: Evaluation of representative c-diIMP analogs intraperitoneally administered as immunotherapies in a murine model of pancreatic cancer

- **Species evaluated:** mouse
- **Tumor model:** Panc02 (murine pancreatic tumor cell line)
- **Treatments tested:** CL735 and CL742 (monotherapy)
- **Clinical parameters evaluated:** tumor volume, tumor weight and incidence of metastasis
- **Administration routes evaluated:** intraperitoneal (i.p.) injection

On Day 1, 25 mice (C57BL/6; male) received an orthotopic injection of Panc02 tumor cells (1 x 106) in the pancreas. On Day 8, the mice were then divided into two groups of eight each (treated groups) and one group of five (control group). Each group received a different treatment, as outlined below:
**Group 1 (control):** saline (by i.p. injection) once daily, 4 days a week for 3 weeks;
**Group 2:** CL735 at 1 mg/Kg (by i.p. injection) once daily, 4 days a week for 3 weeks;
**Group 3:** CL742 at 1 mg/Kg (by i.p. injection) once daily, 4 days a week for 3 weeks

Animals were euthanized at Day 28 and the tumor volume, tumor weight and incidence of metastasis in each mouse were measured.

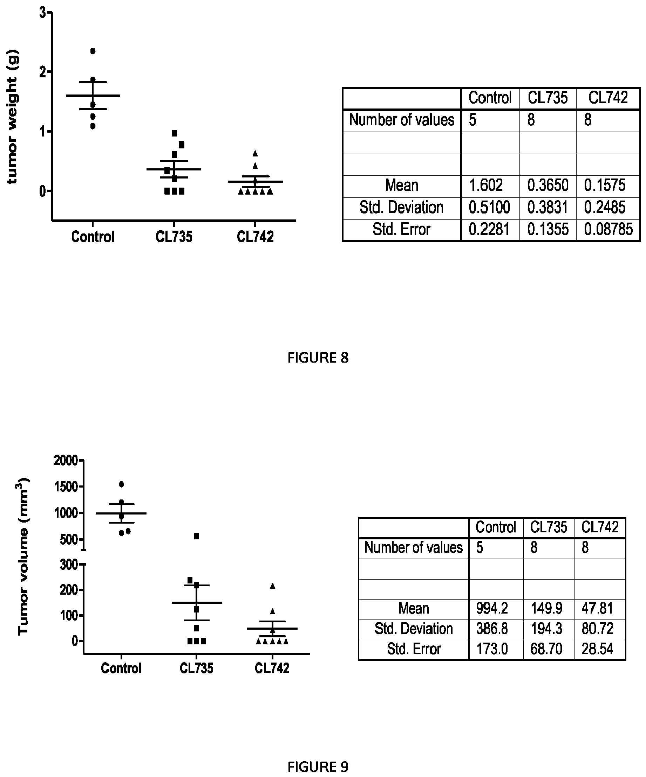

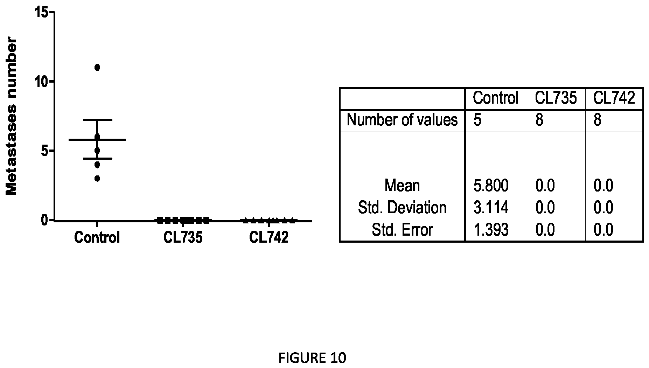

**Figure 8** represents the individual tumor volumes for all mice at the end of the experiment, **Figure 9** represents the corresponding tumor weights and **Figure 10** shows the incidence of metastasis.
**Figure 8****. Left:** Weight of individual Panc02 tumors at day 28 by treatment group. **Right:** Mean tumor weight at day 28, sd and sem for each group.
**Figure 9****. Left:** Volume of individual Panc02 tumors at day 28 by treatment group. **Right:** Mean tumor volume at day 28, sd and sem for each group.
**Figure 10****.** Incidence of metastasis in a murine model of Panc02 tumors. The mice were treated with saline (control), CL735 or CL742 monotherapies and metastases number was recorded at day 28. On the right, table indicated the mean of metastases number on animals that have survived by day 28, sd and sem for each group.

The results from this experiment are shown in **Figures 8 to 10****.** **Figure 8** reveals that in comparison to the control group, each treatment group (CL735 or CL742 monotherapy) underwent a significant reduction in tumor growth. At a dose of 1 mg/kg, the c-diIMP derivatives CL735 and CL742 each caused the tumor to progress much more slowly than in the control group. Thus, by the end of the experiment (day 28), the final mean tumor volumes for the CL735 and CL742 treatment groups were 150 +/- 69 mm3 and 47 +/- 29 mm3, respectively, compared to a value of 994 +/- 173 mm3 for the untreated group (Figure 9, right). Among the two treatments tested, CL742 appears to be the most effective. Moreover, Figure 10 shows that no metastases were found in any of the animals that had received CL735 or CL742, whereas all five animals in the control group developed metastases in their bowels and liver.

### Example 7.2: Evaluation of representative c-diIMP analogs intratumorally administered as immunotherapies in a murine model of melanoma cancer

- **Species evaluated:** mouse
- **Tumor model:** B16 (murine melanoma tumor cell line)
- **Treatments tested:** CL742 and CL747 (monotherapy)
- **Clinical parameters evaluated:** tumor volume
- **Administration routes evaluated:** intratumoral (i.t.) injection

On Day 1, 15 mice (C57BL/6; male) received a subcutaneous injection of B16 tumor cells (1 x 105) on the right flank. On Day 13, the mice were then divided into two groups of five each (treated groups) and one group of five (control group). Each group received a different treatment, as outlined below:
**Group 1 (control):** saline (i.t. injection) at days 13 16 and 20;
**Group 2:** CL742 at 1.25 mg/Kg (i.t. injection) at days 13 16 and 20;
**Group 3:** CL747 at 1.25 mg/Kg (i.t. injection) at days 13 16 and 20

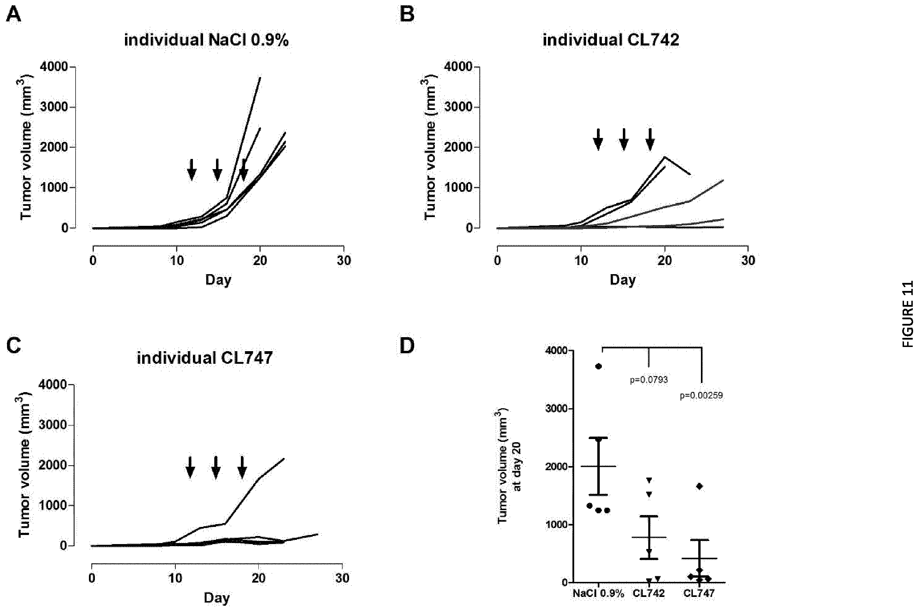

The tumor volume and animal weight were monitored every 2 days and animals were euthanized at Day 28 (Figure 11).

The results from this experiment shown in **Figure 11** reveal that in comparison to the control group, each treatment group (CL742 or CL747 monotherapy) underwent a significant reduction in tumor growth. At a dose of 1.25 mg/kg, the c-diIMP derivatives CL742 and CL747 each caused the tumor to progress much more slowly than in the control group. Thus, by day 20 the mean tumor volumes for the CL742 and CL747 treatment groups were 779 +/- 816 mm3 and 422 +/- 698 mm3, respectively, compared to a value of 2006 +/- 1002 mm3 for the untreated group (Figure 10D). Among the two treatments tested, CL747 appears to be the most effective.

## Claims

1. A cyclic dinucleotide compound of Formula (I) or Formula (II): 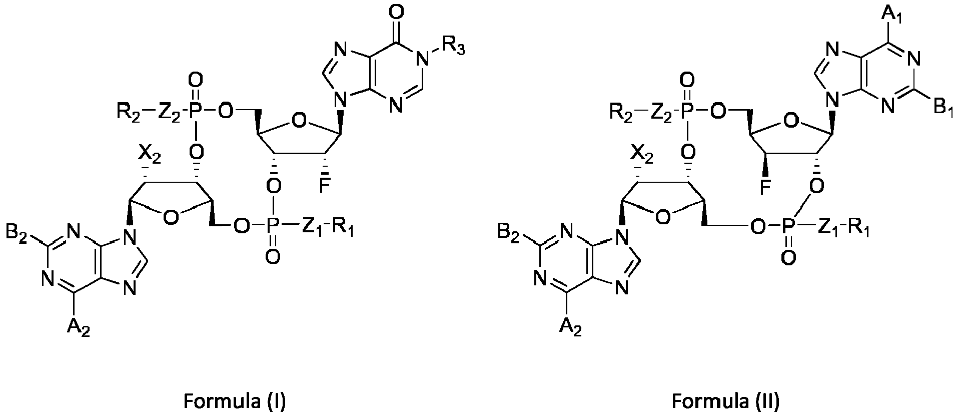 wherein:
• X₂ is H or F;
• Z₁ and Z₂ are independently O or S;
• R₁ and R₂ are independently H or an enzyme-labile group which, together with the oxygen atom or sulfur atom to which it is attached, provides OH or SH *in vivo* such as pivaloyloxymethyl or acyloxybenzyl group;
• A₁ is O-R₃ or NH-R₃;
• A₂ is O-R_{3'} or NH-R_{3'} ;
• B₁ is H, NH-R₄ or an halogen such as F, Cl, Br, I;
• B₂ is H, NH-R_{4'} or an halogen such as F, Cl, Br, I;
• R₃, R_{3'}, R₄ and R_{4'} are independently H or an enzyme-labile group which, together with the oxygen atom or sulfur atom to which it is attached, provides OH or NH₂ *in vivo* such as pivaloyloxymethyl,
or a pharmaceutically acceptable salt, stereoisomer, tautomer or solvate thereof,
except a compound of Formula (I) wherein X₂ = F, Z₁ = Z₂ = O, R₁ = R₂ = R₃ = H, A₂ = OH and B₂ = H.

2. The cyclic dinucleotide compound according to claim 1, wherein said compound is of Formula (III): 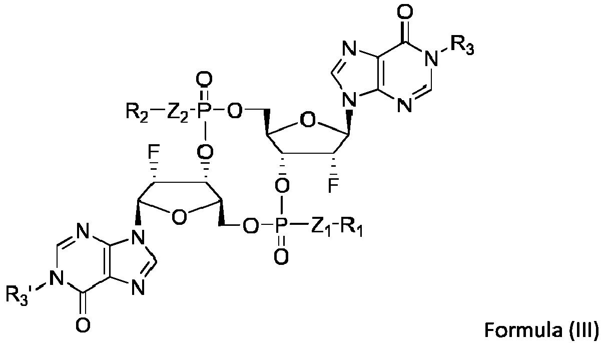 wherein R₁, R₂, Z₁, Z₂, R₃ and R'₃ are as defined in claim 1.

3. The cyclic dinucleotide compound according to claim 1, wherein said compound is of Formula (IV): 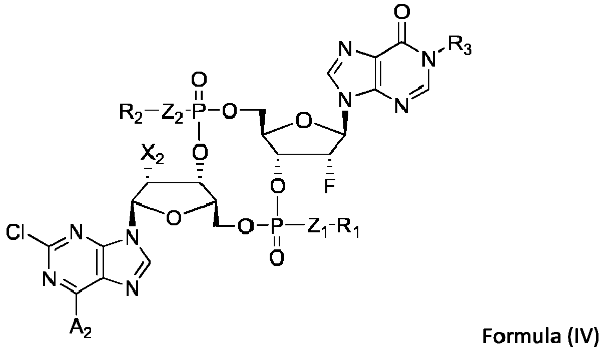 wherein X₂, R₁, R₂, Z₁, Z₂, R₃ and A₂ are as defined in claim 1.

4. The cyclic dinucleotide compound according to claim 1 or claim 2, wherein said compound is of Formula (I) and A₂ is O-R₃ and B₂ is H.

5. The cyclic dinucleotide compound according to claim 1, wherein said compound is of Formula (V): 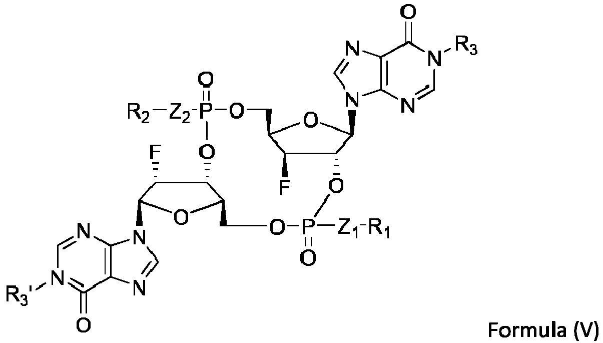 wherein R₁, R₂, Z₁, Z₂, R₃ and R_{3'} are as defined in claim 1.

6. The cyclic dinucleotide compound according to claim 1, wherein said compound is of Formula (VI): 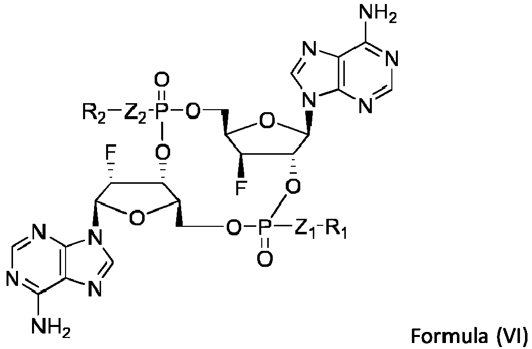 wherein R₁, R₂, Z₁ and Z₂ are as defined in claim 1.

7. The cyclic dinucleotide compound according to claim 1, wherein said compound is of Formula (VII): 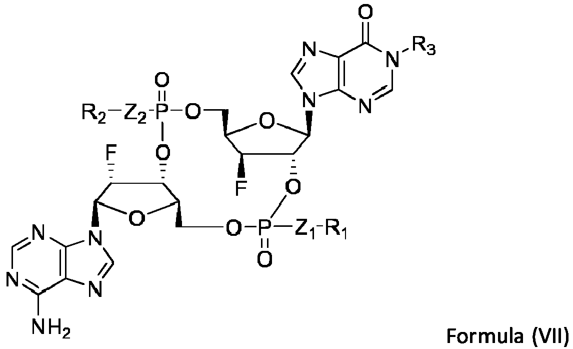 wherein R₁, R₂, Z₁, Z₂ and R₃ are as defined above.

8. The cyclic dinucleotide compound according to claim 1, wherein said compound is of Formula (VIII): 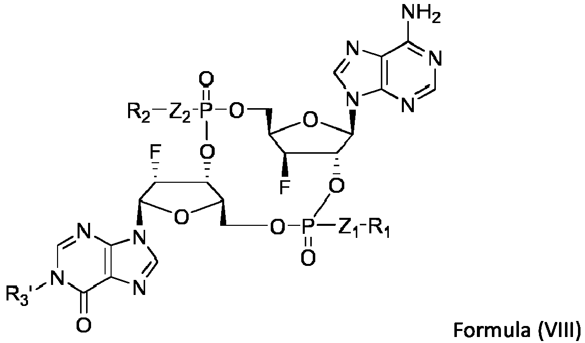 wherein R₁, R₂, Z₁, Z₂ and R_{3'} are as defined above.

9. The cyclic dinucleotide compound according to claim 1, wherein said compound is of Formula (IX): 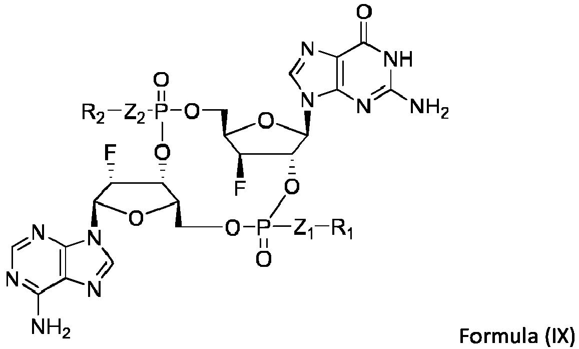 wherein R₁, R₂, Z₁ and Z₂ are as defined above.

10. The cyclic dinucleotide compound according to claim 1, wherein said compound is of Formula (X): 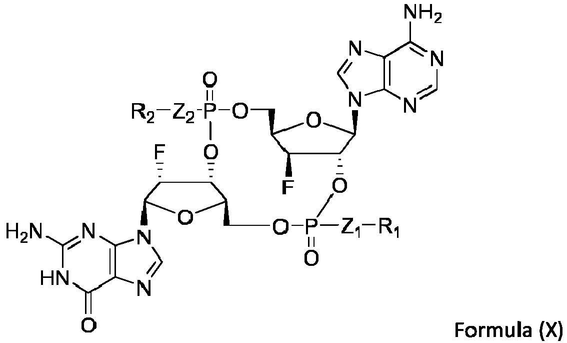 wherein R₁, R₂, Z₁ and Z₂ are as defined above.

11. The cyclic dinucleotide compound according to any one of claims 1 to 10, wherein Z₁ and/or Z₂ are S.

12. The cyclic dinucleotide compound according to any one of claims 1 to 11, wherein said cyclic dinucleotide compound is chosen from the following compounds:

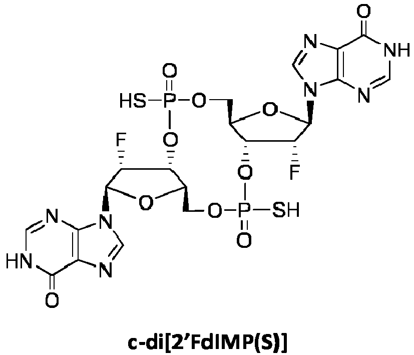

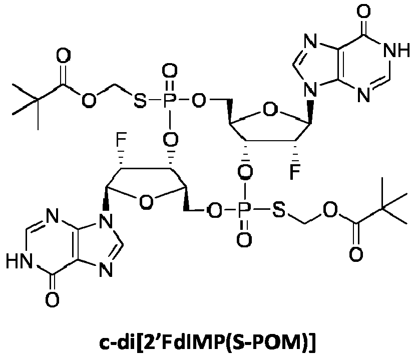

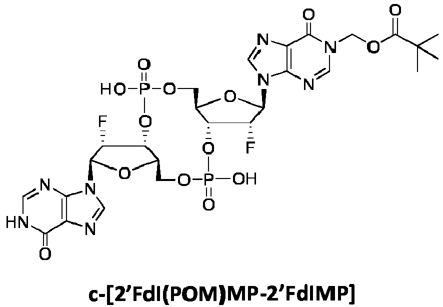

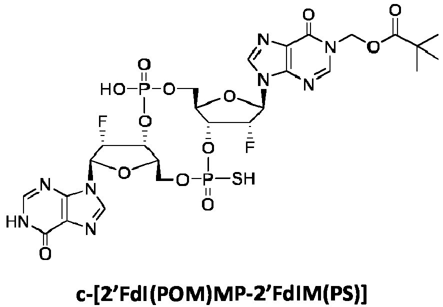

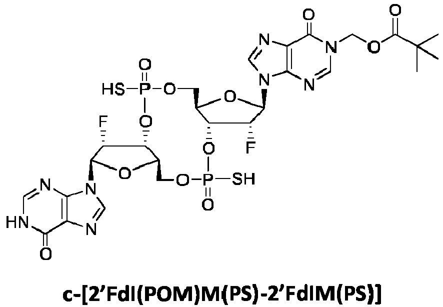

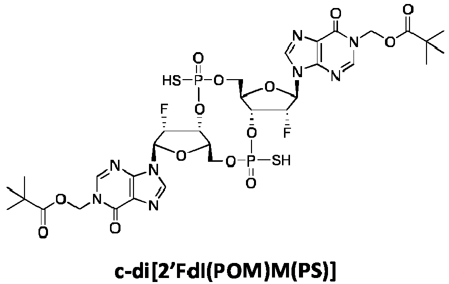

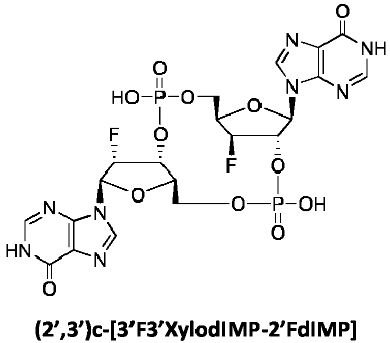

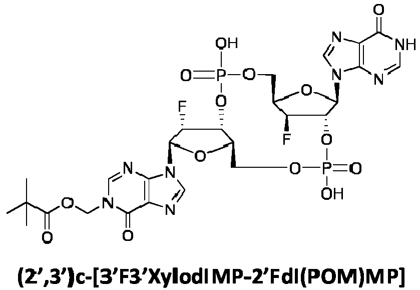

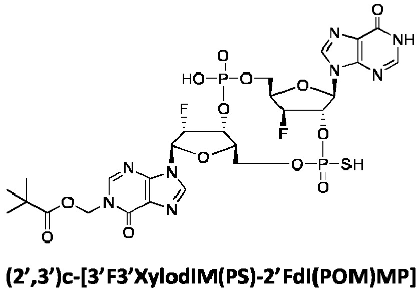

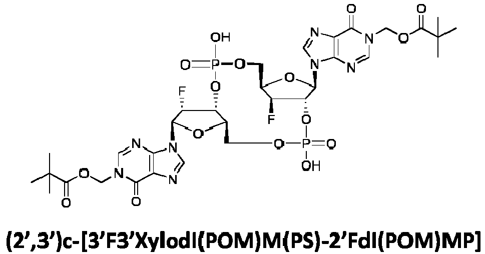

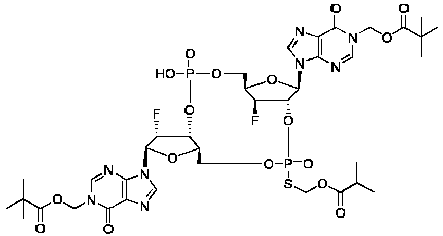

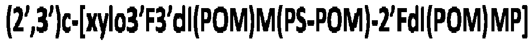

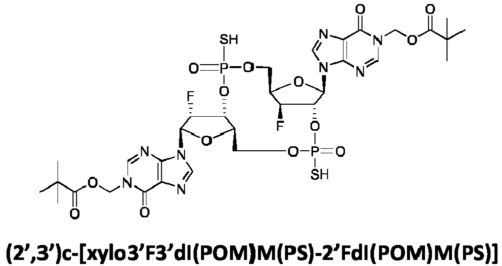

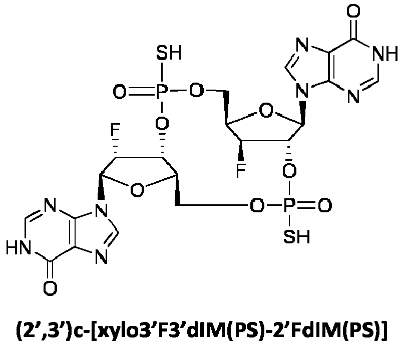

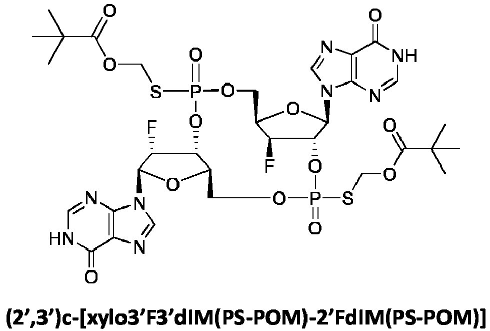

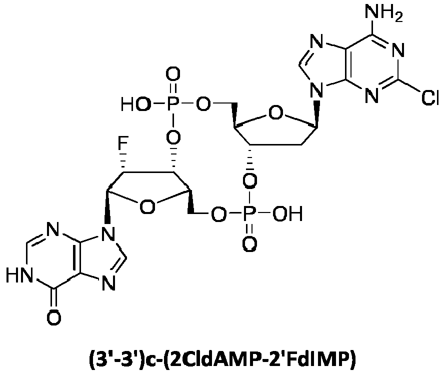

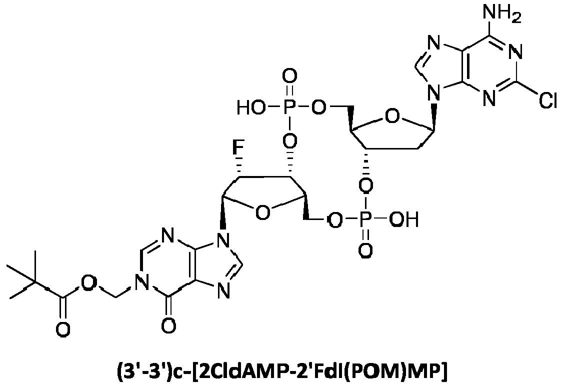

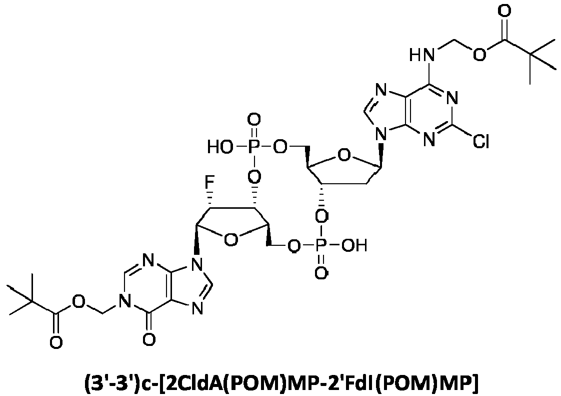

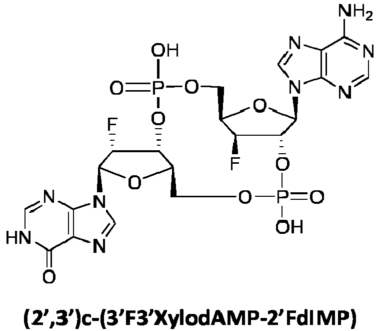

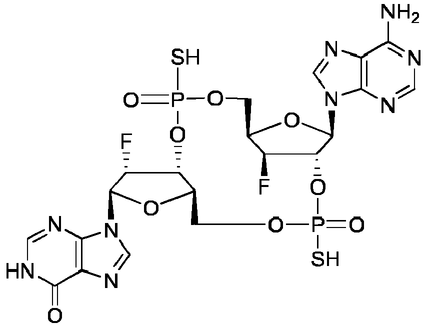

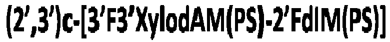

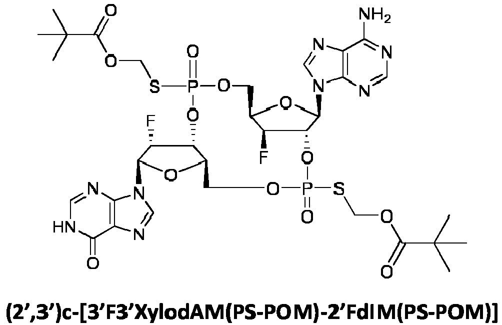

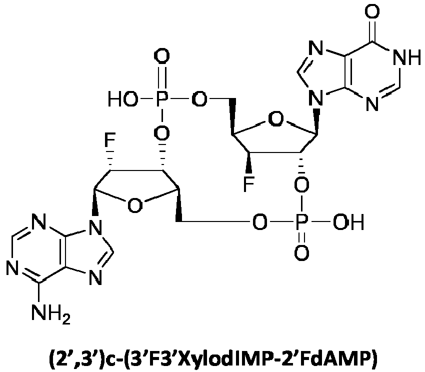

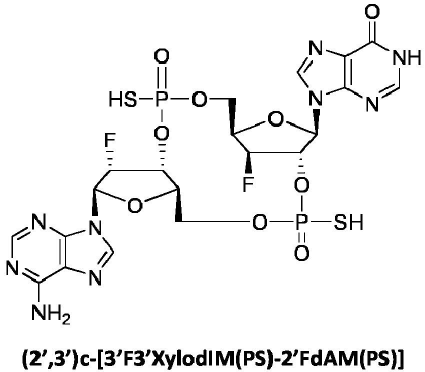

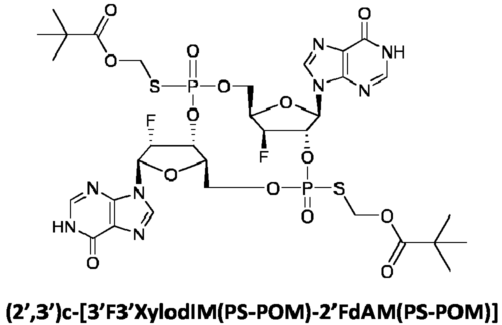

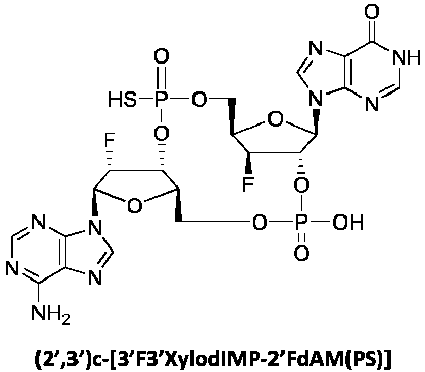

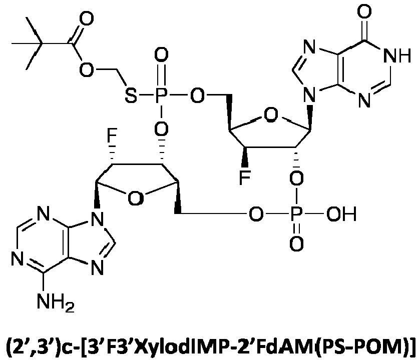

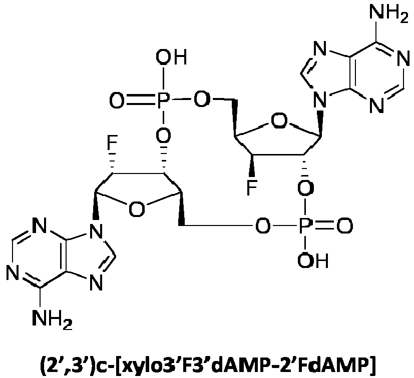

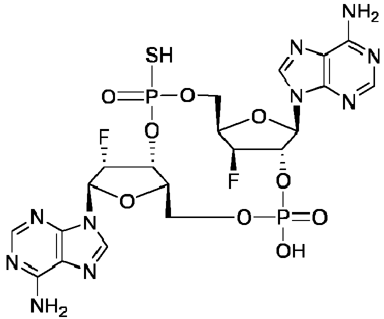

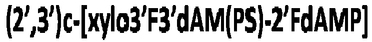

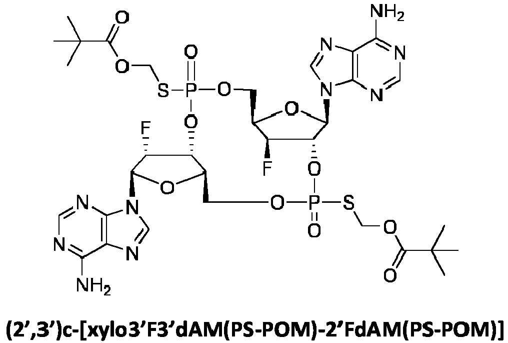

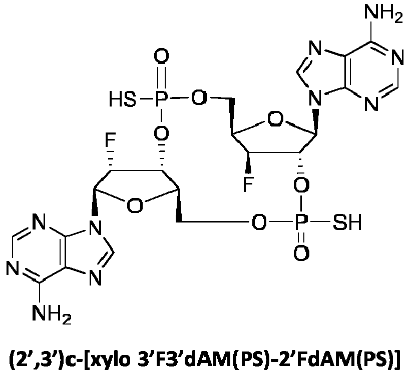

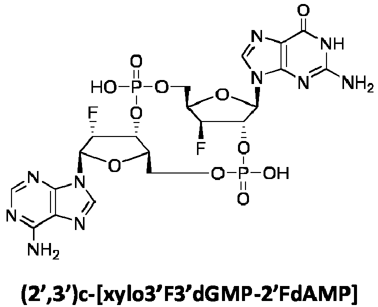

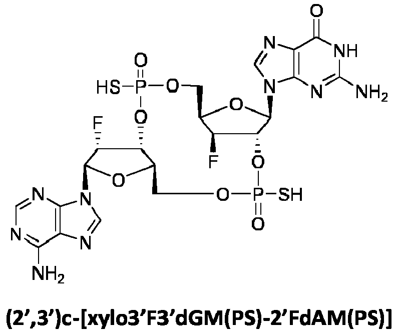

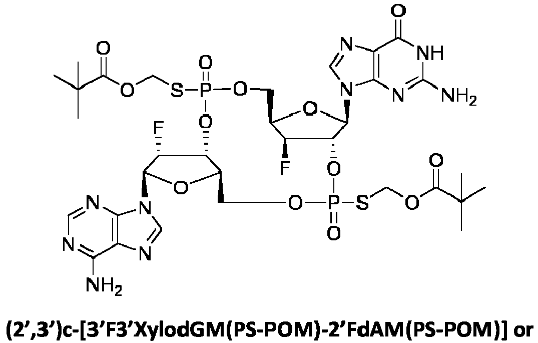

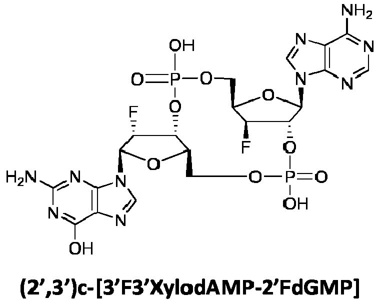

13. A pharmaceutical composition comprising a cyclic dinucleotide compound according to any one of claims 1 to 12 and a pharmaceutically acceptable excipient.

14. A cyclic dinucleotide compound according to any one of claims 1 to 12, for use in a therapeutic treatment in humans or animals.

15. A cyclic dinucleotide compound according to any one of claims 1 to 12, for use in the treatment of a disease that may be alleviated by the induction of an immune response via the STING pathway.

16. A cyclic dinucleotide compound according to any one of claims 1 to 12, for use in an immunomodulatory agent.

17. A cyclic dinucleotide compound according to any one of claims 1 to 12, for use in the treatment of cancer or pre-cancerous syndromes or infectious diseases, such as viral infection, in particular an AIDS infection or an HIV infection.

18. A cyclic dinucleotide compound according to any one of claims 1 to 12, for use as immunoadjuvant.

19. A therapeutic combination comprising a cyclic dinucleotide compound according to any one of claims 1 to 12 and a therapeutic agent.

20. A kit-of-parts comprising a cyclic dinucleotide compound according to any one of claims 1 to 12 and a chemotherapeutic agent, for use in the treatment of cancer.
